# EUROPEAN PATENT SPECIFICATION

(11) **EP 0 944 648 B1**
(45) Date of publication and mention of the grant of the patent: **14.03.2007**
(21) Application number: 97935509.6
(22) Date of filing: 22.08.1997
(51) Int. Cl.: C07K 14/605, A61K 38/26

(54) **GLP-1 DERIVATIVES**
GLP-1 DERIVATE
DERIVES DE GLP-1

(30) Priority: 30.08.1996 DK 93196; 08.11.1996 DK 125996; 20.12.1996 DK 147096
(43) Date of publication of application: 29.09.1999
(73) Proprietor: NOVO NORDISK A/S, 2880 Bagsvaerd (DK)
(72) Inventor: KNUDSEN, Liselotte, Bjerre, DK-2500 Valby (DK); SØRENSEN, Per, Olaf, DK-1411 Copenhagen K (DK); NIELSEN, Per, Franklin, DK-3500 Varlose (DK)
(86) International application number: PCT/DK1997/000340
(87) International publication number: WO 1998/008871

(56) References cited:
- WO-A-91/11457
- WO-A-95/31214
- NN-A-
- US-A- 5 512 549

## Description

### FIELD OF THE INVENTION

The present invention relates to novel derivatives of human glucagon-like peptide-1 (GLP-1) and fragments thereof and analogues of such fragments which have a protracted profile of action and to methods of making and using them.

### BACKGROUND OF THE INVENTION

Peptides are widely used in medical practice, and since they can be produced by recombinant DNA technology it can be expected that their importance will increase also in the years to come. When native peptides or analogues thereof are used in therapy it is generally found that they have a high clearance. A high clearance of a therapeutic agent is inconvenient in cases where it is desired to maintain a high blood level thereof over a prolonged period of time since repeated administrations will then be necessary. Examples of peptides which have a high clearance are: ACTH, corticotropin-releasing factor, angiotensin, calcitonin, insulin, glucagon, glucagon-like peptide-1, glucagon-like peptide-2, insulin-like growth factor-1, insulin-like growth factor-2, gastric inhibitory peptide, growth hormone-releasing factor, pituitary adenylate cyclase activating peptide, secretin, enterogastrin, somatostatin, somatotropin, somatomedin, parathyroid hormone, thrombopoietin, erythropoietin, hypothalamic releasing factors, prolactin, thyroid stimulating hormones, endorphins, enkephalins, vasopressin, oxytocin, opiods and analogues thereof, superoxide dismutase, interferon, asparaginase, arginase, arginine deaminase, adenosine deaminase and ribonuclease. In some cases it is possible to influence the release profile of peptides by applying suitable pharmaceutical compositions, but this approach has various shortcomings and is not generally applicable.

The hormones regulating insulin secretion belong to the so-called enteroinsular axis, designating a group of hormones, released from the gastrointestinal mucosa in response to the presence and absorption of nutrients in the gut, which promote an early and potentiated release of insulin. The enhancing effect on insulin secretion, the so-called incretin effect, is probably essential for a normal glucose tolerance. Many of the gastrointestinal hormones, including gastrin and secretin (cholecystokinin is not insulinotropic in man), are insulinotropic, but the only physiologically important ones, those that are responsible for the incretin effect, are the glucose-dependent insulinotropic polypeptide, GIP, and glucagon-like peptide-1 (GLP-1). Because of its insulinotropic effect, GIP, isolated in 1973 (1) immediately attracted considerable interest among diabetologists. However, numerous investigations carried out during the following years clearly indicated that a defective secretion of GIP was not involved in the pathogenesis of insulin dependent diabetes mellitus (IDDM) or non insulin-dependent diabetes mellitus (NIDDM) (2). Furthermore, as an insulinotropic hormone, GIP was found to be almost ineffective in NIDDM (2). The other incretin hormone, GLP-1 is the most potent insulinotropic substance known (3). Unlike GIP, it is surprisingly effective in stimulating insulin secretion in NIDDM patients. In addition, and in contrast to the other insulinotropic hormones (perhaps with the exception of secretin) it also potently inhibits glucagon secretion. Because of these actions it has pronounced blood glucose lowering effects particularly in patients with NIDDM.

GLP-1, a product of the proglucagon (4), is one of the youngest members of the secretin-VIP family of peptides, but is already established as an important gut hormone with regulatory function in glucose metabolism and gastrointestinal secretion and metabolism (5). The glucagon gene is processed differently in the pancreas and in the intestine. In the pancreas (9), the processing leads to the formation and parallel secretion of 1) glucagon itself, occupying positions 33-61 of proglucagon (PG); 2) an N-terminal peptide of 30 amino acids (PG (1-30)) often called glicentin-related pancreatic peptide, GRPP (10, 11); 3) a hexapeptide corresponding to PG (64-69); 4) and, finally, the so-called major proglucagon fragment (PG (72-158)), in which the two glucagon-like sequences are buried (9). Glucagon seems to be the only biologically active product. In contrast, in the intestinal mucosa, it is glucagon that is buried in a larger molecule, while the two glucagon-like peptides are formed separately (8). The following products are formed and secreted in parallel: 1) glicentin, corresponding to PG (1-69), with the glucagon sequence occupying residues Nos. 33-61 (12); 2) GLP-1 (7-36)amide (PG (78-107))amide (13), not as originally believed PG (72-107)amide or 108, which is inactive). Small amounts of C-terminally glycine-extended but equally bioactive GLP-1 (7-37), (PG (78-108)) are also formed (14); 3) intervening peptide-2 (PG (111-122)amide) (15); and 4) GLP-2 (PG (126-158)) (15, 16). A fraction of glicentin is cleaved further into GRPP (PG (1-30)) and oxyntomodulin (PG (33-69)) (17, 18). Of these peptides, GLP-1, has the most conspicuous biological activities.

Being secreted in parallel with glicentin/enteroglucagon, it follows that the many studies of enteroglucagon secretion (6, 7) to some extent also apply to GLP-1 secretion, but GLP-1 is metabolised more quickly with a plasma half-life in humans of 2 min (19). Carbohydrate or fat-rich meals stimulate secretion (20), presumably as a result of direct interaction of yet unabsorbed nutrients with the microvilli of the open-type L-cells of the gut mucosa. Endocrine or neural mechanisms promoting GLP-1 secretion may exist but have not yet been demonstrated in humans.

The incretin function of GLP-1(29-31) has been clearly illustrated in experiments with the GLP-1 receptor antagonist, exendin 9-39, which dramatically reduces the incretin effect elicited by oral glucose in rats (21, 22). The hormone interacts directly with the β-cells via the GLP-1 receptor (23) which belongs to the glucagon/VIP/calcitonin family of G-protein-coupled 7-transmembrane spanning receptors. The importance of the GLP-1 receptor in regulating insulin secretion was illustrated in recent experiments in which a targeted disruption of the GLP-1 receptor gene was carried out in mice. Animals homozygous for the disruption had greatly deteriorated glucose tolerance and fasting hyperglycaemia, and even heterozygous animals were glucose intolerant (24). The signal transduction mechanism (25) primarily involves activation of adenylate cyclase, but elevations of intracellular Ca²⁺ are also essential (25, 26). The action of the hormone is best described as a potentiation of glucose stimulated insulin release (25), but the mechanism that couples glucose and GLP-1 stimulation is not known. It may involve a calcium-induced calcium release (26, 27). As already mentioned, the insulinotropic action of GLP-1 is preserved in diabetic β-cells. The relation of the latter to its ability to convey "glucose competence" to isolated insulin-secreting cells (26, 28), which respond poorly to glucose or GLP-1 alone, but fully to a combination of the two, is also not known. Equally importantly, however, the hormone also potently inhibits glucagon secretion (29). The mechanism is not known, but seems to be paracrine, via neighbouring insulin or somatostatin cells (25). Also the glucagonostatic action is glucose-dependent, so that the inhibitory effect decreases as blood glucose decreases. Because of this dual effect, if the plasma GLP-1 concentrations increase either by increased secretion or by exogenous infusion the molar ratio of insulin to glucagon in the blood that reaches the liver via the portal circulation is greatly increased, whereby hepatic glucose production decreases (30). As a result blood glucose concentrations decrease. Because of the glucose dependency of the insulinotropic and glucagonostatic actions, the glucose lowering effect is self-limiting, and the hormone, therefore, does not cause hypoglycaemia regardless of dose (31). The effects are preserved in patients with diabetes mellitus (32), in whom infusions of slightly supraphysiological doses of GLP-1 may completely normalise blood glucose values in spite of poor metabolic control and secondary failure to sulphonylurea (33). The importance of the glucagonostatic effect is illustrated by the finding that GLP-1 also lowers blood glucose in type-1 diabetic patients without residual β-cell secretory capacity (34).

In addition to its effects on the pancreatic islets, GLP-1 has powerful actions on the gastrointestinal tract. Infused in physiological amounts, GLP-1 potently inhibits pentagastrin-induced as well as meal-induced gastric acid secretion (35, 36). It also inhibits gastric emptying rate and pancreatic enzyme secretion (36). Similar inhibitory effects on gastric and pancreatic secretion and motility may be elicited in humans upon perfusion of the ileum with carbohydrate- or lipid-containing solutions (37, 38). Concomitantly, GLP-1 secretion is greatly stimulated, and it has been speculated that GLP-1 may be at least partly responsible for this so-called "ileal-brake" effect (38). In fact, recent studies suggest that, physiologically, the ileal-brake effects of GLP-1 may be more important than its effects on the pancreatic islets. Thus, in dose response studies GLP-1 influences gastric emptying rate at infusion rates at least as low as those required to influence islet secretion (39).

GLP-1 seems to have an effect on food intake. Intraventricular administration of GLP-1 profoundly inhibits food intake in rats (40, 42). This effect seems to be highly specific. Thus, N-terminally extended GLP-1 (PG 72-107)amide is inactive and appropriate doses of the GLP-1 antagonist, exendin 9-39, abolish the effects of GLP-1 (41). Acute, peripheral administration of GLP-1 does not inhibit food intake acutely in rats (41, 42). However, it remains possible that GLP-1 secreted from the intestinal L-cells may also act as a satiety signal.

Not only the insulinotropic effects but also the effects of GLP-1 on the gastrointestinal tract are preserved in diabetic patients (43), and may help curtailing meal-induced glucose excursions, but, more importantly, may also influence food intake. Administered intravenously, continuously for one week, GLP-1 at 4 ng/kg/min has been demonstrated to dramatically improve glycaemic control in NIDDM patients without significant side effects (44). The peptide is fully active after subcutaneous administration (45), but is rapidly degraded mainly due to degradation by dipeptidyl peptidase IV-like enzymes (46, 47).

The amino acid sequence of GLP-1 is given *i.a.* by Schmidt *et al.* (*Diabetologia* **28** 704-707 (1985). Although the interesting pharmacological properties of GLP-1 (7-37) and analogues thereof have attracted much attention in recent years only little is known about the structure of these molecules. The secondary structure of GLP-1 in micelles has been described by Thorton *et al.* (*Biochemistry* **33** 3532-3539 (1994)), but in normal solution, GLP-1 is considered a very flexible molecule. Surprisingly, we found that derivatisation of this relatively small and very flexible molecule resulted in compounds whose plasma profile were highly protracted and still had retained activity.

GLP-1 and analogues of GLP-1 and fragments thereof are potentially useful *i.a.* in the treatment of type 1 and type 2 diabetes. However, the high clearance limits the usefulness of these compounds, and thus there still is a need for improvements in this field. Accordingly, it is one object of the present invention to provide derivatives of GLP-1 and analogues thereof which have a protracted profile of action relative to GLP-1 (7-37). It is a further object of the invention to provide derivatives of GLP-1 and analogues thereof which have a lower clearance than GLP-1 (7-37). It is a further object of the invention to provide a pharmaceutical composition comprising a compound according to the invention and to use a compound of the invention to provide such a composition. Also, it is an object of the present invention to provide a method of treating insulin dependent and non-insulin dependent diabetes mellitus.

### References.

1. Pederson RA. Gastric Inhibitory Polypeptide. In Walsh JH, Dockray GJ (eds) Gut peptides: Biochemistry and Physiology. Raven Press, New York 1994, pp. 217259.
2. Krarup T. Immunoreactive gastric inhibitory polypeptide. Endocr Rev 1988;9:122-134.
3. Ørskov C. Glucagon-like peptide-1, a new hormone of the enteroinsular axis. Diabetologia 1992; 35:701-711.
4. Bell Gl, Sanchez-Pescador R, Laybourn PJ, Najarian RC. Exon duplication and divergence in the human preproglucagon gene. Nature 1983; 304: 368-371.
5. Holst JJ. Glucagon-like peptide-1 (GLP-1) - a newly discovered Gl hormone. Gastroenterology 1994; 107: 1848-1855.
6. Holst JJ. Gut glucagon, enteroglucagon, gut GLl, glicentin - current status. Gastroenterology 1983;84:1602-1613.
7. Holst JJ, Ørskov C. Glucagon and other proglucagon-derived peptides. In Walsh JH, Dockray GJ, eds. Gut peptides: Biochemistry and Physiology. Raven Press, New York, pp. 305-340, 1993.
8. Ørskov C, Holst JJ, Knuhtsen S, Baldissera FGA, Poulsen SS, Nielsen OV. Glucagon-like peptides GLP-1 and GLP-2, predicted products of the glucagon gene, are secreted separately from the pig small intestine, but not pancreas. Endocrinology 1986;119:1467-1475.
9. Holst JJ, Bersani M, Johnsen AH, Kofod H, Hartmann B, Ørskov C. Proglucagon processing in porcine and human pancreas. J Biol Chem, 1994; 269: 18827-1883.
10. Moody AJ, Holst JJ, Thim L, Jensen SL. Relationship of glicentin to proglucagon and glucagon in the porcine pancreas. Nature 1981; 289: 514-516.
11. Thim L, Moody AJ, Purification and chemical characterisation of a glicentin-related pancreatic peptide (proglucagon fragment) from porcine pancreas. Biochim Biophys Acta 1982;703:134-141.
12. Thim L, Moody AJ. The primary structure of glicentin (proglucagon). Regul Pept 1981;2:139-151.
13. Ørskov C, Bersani M, Johnsen AH, Højrup P, Holst JJ. Complete sequences of glucagon-like peptide-1 (GLP-1) from human and pig small intestine. J. Biol. Chem. 1989;264:12826-12829.
14. Ørskov C, Rabenhøj L, Kofod H, Wettergren A, Holst JJ. Production and secretion of amidated and glycine-extended glucagon-like peptide-1 (GLP-1) in man. Diabetes 1991; 43: 535-539.
15. Buhl T, Thim L, Kofod H, Ørskov C, Harling H, & Holst JJ: Naturally occurring products of proglucagon 111-160 in the porcine and human small intestine. J. Biol. Chem. 1988;263:8621-8624.
16. Ørskov C, Buhl T, Rabenhøj L, Kofod H, Holst JJ: Carboxypeptidase-B-like processing of the C-terminus of glucagon-like peptide-2 in pig and human small intestine. FEBS letters, 1989;247:193-106.
17. Holst JJ. Evidence that enteroglucagon (II) is identical with the C-terminal sequence (residues 33-69) of glicentin. Biochem J. 1980;187:337-343.
18. Bataille D, Tatemoto K, Gespach C, Jörnvall H, Rosselin G, Mutt V. Isolation of glucagon-37 (bioactive enteroglucagon/oxyntomodulin) from porcine jejuno-ileum. Characterisation of the peptide. FEBS Lett 1982;146:79-86.
19. Ørskov C, Wettergren A, Holst JJ. The metabolic rate and the biological effects of GLP-1 7-36amide and GLP-1 7-37 in healthy volunteers are identical. Diabetes 1993;42:658-661.
20. Elliott RM, Morgan LM, Tredger JA, Deacon S, Wright J, Marks V. Glucagon-like peptide-1 (7-36)amide and glucose-dependent insulinotropic polypeptide secretion in response to nutrient ingestion in man: acute post-prandial and 24-h secretion patterns. J Endocrinol 1993; 138: 159-166.
21. Kolligs F, Fehmann HC, Göke R, Göke B. Reduction of the incretin effect in rats by the glucagon-like peptide-1 receptor antagonist exendin (9-39)amide. Diabetes 1995; 44: 16-19.
22. Wang Z, Wang RM, Owji AA, Smith DM, Ghatei M, Bloom SR. Glucagon-like peptide-1 is a physiological incretin in rat. J. Clin. Invest. 1995; 95: 417-421.
23. Thorens B. Expression cloning of the pancreatic b cell receptor for the gluco-incretin hormone glucagon-like peptide 1. Proc Natl Acad Sci 1992;89:8641-4645.
24. Scrocchi L, Auerbach AB, Joyner AL, Drucker DJ. Diabetes in mice with targeted disruption of the GLP-1 receptor gene. Diabetes 1996; 45: 21A.
25. Fehmann HC, Göke R, Göke B. Cell and molecular biology of the incretin hormones glucagon-like peptide-I (GLP-1) and glucose-dependent insulin releasing polypeptide (GIP). Endocrine Reviews, 1995; 16: 390-410.
26. Gromada J, Dissing S, Bokvist K, Renström E, Frokjaer-Jensen J, Wulff BS, Rorsman P. Glucagon-like peptide I increases cytoplasmic calcium in insulin-secreting bTC3-cells by enhancement of intracellular calcium mobilisation. Diabetes 1995; 44: 767-774.
27. Holz GG, Leech CA, Habener JF. Activation of a cAMP-regulated Ca²⁺-signaling pathway in pancreatic β-cells by the insulinotropic hormone glucagon-like peptide-1. J Biol Chem, 1996; 270: 17749-17759.
28. Holz GG, Kühltreiber WM, Habener JF. Pancreatic beta-cells are rendered glucose competent by the insulinotropic hormone glucagon-like peptide-1(7-37). Nature 1993;361:362-365.
29. Ørskov C, Holst JJ, Nielsen OV: Effect of truncated glucagon-like peptide-1 (proglucagon 78-107 amide) on endocrine secretion from pig pancreas, antrum and stomach. Endocrinology 1988;123:2009-2013.
30. Hvidberg A, Toft Nielsen M, Hilsted J, Ørskov C, Holst JJ. Effect of glucagon-like peptide-1 (proglucagon 78-107amide) on hepatic glucose production in healthy man. Metabolism 1994;43:104-108.
31. Qualmann C, Nauck M, Holst JJ, Ørskov C, Creutzfeldt W. Insulinotropic actions of intravenous glucagon-like peptide-1 [7-36 amide] in the fasting state in healthy subjects. Acta Diabetologica, 1995; 32: 13-16.
32. Nauck MA, Heimesaat MM, Ørskov C, Holst JJ, Ebert R, Creutzfeldt W. Preserved incretin activity of GLP-1 (7-36amide) but not of synthetic human GIP in patients with type 2-diabetes mellitus. J Clin Invest 1993;91:301-307.
33. Nauck MA, Kleine N, ∅rskov C, Holst JJ, Willms B, Creutzfeldt W. Normalisation of fasting hyperglycaemia by exogenous GLP-1(7-36amide) in type 2-diabetic patients. Diabetologia 1993;36:741-744.
34. Creutzfeldt W, Kleine N, Willms B, ∅rskov C, Holst JJ, Nauck MA. Glucagonostatic actions and reduction of fasting hyperglycaemia by exogenous glucagon-liem, peptide-1 (7-36amide) in type I diabetic patients. Diabetes Care 1996; 19: 580-586.
35. Schjoldager BTG, Mortensen PE, Christiansen J, Ørskov C, Hoist JJ. GLP-1 (glucagon-like peptide-1) and truncated GLP-1, fragments of human proglucagon, inhibit gastric acid secretion in man. Dig. Dis. Sci. 1989; 35:703-708.
36. Wettergren A, Schjoldager B, Mortensen PE, Myhre J, Christiansen J, Holst JJ. Truncated GLP-1 (proglucagon 72-107amide) inhibits gastric and pancreatic functions in man. Dig Dis Sci 1993;38:665-673.
37. Layer P, Holst JJ, Grandt D, Goebell H: Ileal release of glucagon-like peptide-1 (GLP-1): association with inhibition of gastric acid in humans. Dig Dis Sci 1995; 40: 1074-1082.
38. Layer P, Holst JJ. GLP-1: A humoral mediator of the ileal brake in humans? Digestion 1993; 54: 385-386.
39. Nauck M, Ettler R, Niedereichholz U, Ørskov C, Holst JJ, Schmiegel W. Inhibition of gastric emptying by GLP-1 (7-36 amide) or (7-37): effects on postprandial glycaemia and insulin secretion. Abstract. Gut 1995; 37 (suppl. 2): A124.
40. Schick RR, vorm Walde T, Zimmermann JP, Schusdziarra V, Classen M. Glucagon-like peptide 1 - a novel brain peptide involved in feeding regulation. in Ditschuneit H, Gries FA, Hauner H, Schusdziarra V, Wechsler JG (eds.) Obesity in Europe. John Libbey & Company ltd, 1994; pp. 363-367.
41. Tang-Christensen M, Larsen PJ, Göke R, Fink-Jensen A, Jessop DS, Møller M, Sheikh S. Brain GLP-1(7-36) amide receptors play a major role in regulation of food and water intake. Am. J. Physiol., 1996, in press.
42. Turton MD, O'Shea D, Gunn I, Beak SA, Edwards CMB, Meeran K, et al. A role for glucagon-like peptide-1 in the regulation of feeding. Nature 1996; 379: 69-72.
43. Willms B, Werner J, Creutzfeldt W, Ørskov C, Holst JJ, Nauck M. Inhibition of gastric emptying by glucagon-like peptide-1 (7-36 amide) in patients with type-2-diabetes mellitus. Diabetologia 1994; 37, suppl.1: A118.
44. Larsen J, Jallad N, Damsbo P. One-week continuous infusion of GLP-1 (7-37) improves glycaemic control in NIDDM. Diabetes 1996; 45, suppl. 2: 233A.
45. Ritzel R, Ørskov C, Holst JJ, Nauck MA. Pharmacokinetic, insulinotropic, and glucagonostatic properties of GLP-1 [7-36 amide] after subcutaneous injection in healthy volunteers. Dose-response relationships. Diabetologia 1995; 38: 720-725.
46. Deacon CF, Johnsen AH, Holst JJ. Degradation of glucagon-like peptide-1 by human plasma in vitro yields an N-terminally truncated peptide that is a major endogenous metabolite in vivo. J Clin Endocrinol Metab 1995; 80: 952-957.
47. Deacon CF, Nauck MA, Toft-Nielsen M, Pridal L, Willms B, Hoist JJ. 1995. Both subcutaneous and intravenously administered glucagon-like peptide-1 are rapidly degraded from the amino terminus in type II diabetic patients and in healthy subjects. Diabetes 44: 1126-1131.

### SUMMARY OF THE INVENTION

Human GLP-1 is a 37 amino acid residue peptide originating from preproglucagon which is synthesised *i.a.* in the L-cells in the distal ileum, in the pancreas and in the brain. Processing of preproglucagon to give GLP-1(7-36)amide, GLP-1(7-37) and GLP-2 occurs mainly in the L-cells. A simple system is used to describe fragments and analogues of this peptide. Thus, for example, Gly⁸-GLP-1(7-37) designates a fragment of GLP-1 formally derived from GLP-1 by deleting the amino acid residues Nos. 1 to 6 and substituting the naturally occurring amino acid residue in position 8 (Ala) by Gly. Similarly. Lys³⁴(N^{ε}-tetradecanoyl)-GLP-1(7-37) designates GLP-1 (7-37) wherein the ε-amino group of the Lys residue in position 34 has been tetradecanoylated. Where reference in this text is made to C-terminally extended GLP-1 analogues, the amino acid residue in position 38 is Arg unless otherwise indicated, the optional amino acid residue in position 39 is also Arg unless otherwise indicated and the optional amino acid residue in position 40 is Asp unless otherwise indicated. Also, if a C-terminally extended analogue extends to position 41, 42, 43, 44 or 45, the amino acid sequence of this extension is as in the corresponding sequence in human preproglucagon unless otherwise indicated.

In its broadest aspect, the present invention relates to derivatives of GLP-1 and analogues thereof. The derivatives according to the invention have interesting pharmacological properties, in particular they have a more protracted profile of action than the parent peptides.

In the present text, the designation "an analogue" is used to designate a peptide wherein one or more amino acid residues of the parent peptide have been substituted by another amino acid residue and/or wherein one or more amino acid residues of the parent peptide have been deleted and/or wherein one or more amino acid residues have been added to the parent peptide. Such addition can take place either at the N-terminal end or at the C-terminal end of the parent peptide or both.

The term "derivative" is used in the present text to designate a peptide in which one or more of the amino acid residues of the parent peptide have been chemically modified, e.g. by alkylation, acylation, ester formation or amide formation.

The term "a GLP-1 derivative" is used in the present text to designate a derivative of GLP-1 or an analogue thereof. In the present text, the parent peptide from which such a derivative is formally derived is in some places referred to as the "GLP-1 moiety" of the derivative.

Described herein, but not explicitly claimed, is a GLP-1 derivative wherein at least one amino acid residue of the parent peptide has a lipophilic substituent attached with the proviso that if only one lipophilic substituent is present and this substituent is attached to the N-terminal or to the C-terminal amino acid residue of the parent peptide then this substituent is an alkyl group or a group which has an ω-carboxylic acid group.

Described herein, but no explicitly claimed, is a GLP-1 derivative having only one lipophilic substituent.

Described herein, but not explicitly claimed, is a GLP-1 derivative having only one lipophilic substituent which substituent is an alkyl group or a group which has an ω-carboxylic acid group and is attached to the N-terminal amino acid residue of the parent peptide.

Described herein, but not explicitly claimed, is a GLP-1 derivative having only one lipophilic substituent which substituent is an alkyl group or a group which has an ω-carboxylic acid group and is attached to the C-terminal amino acid residue of the parent peptide.

In a preferred embodiment, as described in Claim 1, the present invention relates to a derivative of GLP-1(7-37) or an analogue of GLP-1(7-37) which derivative is an agonist of the human GLP-1 receptor, characterised in that the derivative has only one lipophilic substituent which is attached to an amino acid residue which is not the N-terminal or C-terminal amino acid residue.

Described herein, but not explicitly claimed, is a GLP-1 derivative wherein two lipophilic substituents are present.

Described herein, but not explicitly claimed, is a GLP-1 derivative wherein two lipophilic substituents are present, one being attached to the N-terminal amino acid residue while the other is attached to the C-terminal amino acid residue.

In another preferred embodiment, as described in Claim 3, the present invention relates to a derivative of GLP-1(7-37) or an analogue of GLP-1(7-37) which derivative is an agonist of the human GLP-1 receptor, characterised in that the derivative has only two lipophilic substituents which are attached to amino acid residues which are not the N-terminal or the C-terminal amino acid residue.

In another preferred embodiment, as described in Claim 2, the present invention relates to a derivative of GLP-1 (7-37) or an analogue of GLP-1(7-37) which derivative is an agonist of the human GLP-1 receptor, characterised in that the derivative has only two lipophilic substituents, one of which is attached to the C-termina1 amino acid residue while the other is attached to an amino acid residue which is not the N-terminal or the C-terminal amino acid residue.

In a further preferred embodiment, as described in Claim 4, the present invention relates to a derivative of an analogue of GLP-1 (7-37) which derivative is an agonist of the human GLP-1 receptor and wherein the analogue is GLP-1(7-C), wherein C is from 38, 39, 40, 41, 42, 43, 44 and 45 characterised in that the derivative has only one lipophilic substituent which is attached to the C-terminal amino acid residue.

In a further preferred embodiment, the present invention relates to a GLP-1 derivative wherein the lipophilic substituent comprises from 4 to 40 carbon atoms, more preferred from 8 to 25 carbon atoms.

In a further preferred embodiment, the present invention relates to a GLP-1 derivative wherein a lipophilic substituent is attached to an amino acid residue in such a way that a carboxyl group of the lipophilic substituent forms an amide bond with an amino group of the amino acid residue.

In a further preferred embodiment, the present invention relates to a GLP-1 derivative wherein a lipophilic substituent is attached to an amino acid residue in such a way that an amino group of the lipophilic substituent forms an amide bond with a carboxyl group of the amino acid residue.

In a further preferred embodiment, the present invention relates to a GLP-1 derivative wherein a lipophilic substituent is attached to the parent peptide by means of a spacer.

Described herein, but not explicitly claimed, is a GLP-1 derivative wherein a lipophilic substituent - optionally via a spacer - is attached to the ε-amino group of a Lys residue contained in the parent peptide.

In a further preferred embodiment, the present invention relates to a GLP-1 derivative wherein a lipophilic substituent is attached to the parent peptide by means of a spacer which is an unbranched alkane α,ω-dicarboxylic acid group having from 1 to 7 methylene groups, preferably two methylene groups which spacer forms a bridge between an amino group of the parent peptide and an amino group of the lipophilic substituent.

In a further preferred embodiment, the present invention relates to a GLP-1 derivative wherein a lipophilic substituent is attached to the parent peptide by means of a spacer which is an amino acid residue except Cys, or a dipeptide such as Gly-Lys. In the present text, the expression "a dipeptide such as Gly-Lys" is used to designate a dipeptide wherein the C-terminal amino acid residue is Lys, His or Trp, preferably Lys, and wherein the N-terminal amino acid residue is selected from the group comprising Ala, Arg, Asp, Asn, Gly, Glu, Gln, Ile, Leu, Val, Phe and Pro.

In a further preferred embodiment, the present invention relates to a GLP-1 derivative wherein a lipophilic substituent is attached to the parent peptide by means of a spacer which is an amino acid residue except Cys, or is a dipeptide such as Gly-Lys and wherein a carboxyl group of the parent peptide forms an amide bond with an amino group of a Lys residue or a dipeptide containing a Lys residue, and the other amino group of the Lys residue or a dipeptide containing a Lys residue forms an amide bond with a carboxyl group of the lipophilic substituent.

In a further preferred embodiment, the present invention relates to a GLP-1 derivative wherein a lipophilic substituent is attached to the parent peptide by means of a spacer which is an amino acid residue except Cys, or is a dipeptide such as Gly-Lys and wherein an amino group of the parent peptide forms an amide bond with a carboxylic group of the amino acid residue or dipeptide spacer, and an amino group of the amino acid residue or dipeptide spacer forms an amide bond with a carboxyl group of the lipophilic substituent.

In a further preferred embodiment, the present invention relates to a GLP-1 derivative wherein a lipophilic substituent is attached to the parent peptide by means of a spacer which is an amino acid residue except Cys, or is a dipeptide such as Gly-Lys and wherein a carboxyl group of the parent peptide forms an amide bond with an amino group of the amino acid residue spacer or dipeptide spacer, and the carboxyl group of the amino acid residue spacer or dipeptide spacer forms an amide bond with an amino group of the lipophilic substituent.

In a further preferred embodiment, the present invention relates to a GLP-1 derivative wherein a lipophilic substituent is attached to the parent peptide by means of a spacer which is an amino acid residue except Cys, or is a dipeptide such as Gly-Lys, and wherein a carboxyl group of the parent peptide forms an amide bond with an amino group of a spacer which is Asp or Glu, or a dipeptide spacer containing an Asp or Glu residue, and a carboxyl group of the spacer forms an amide bond with an amino group of the lipophilic substituent.

In a further preferred embodiment, the present invention relates to a GLP-1 derivative having a lipophilic substituent which comprises a partially or completely hydrogenated cyclopentanophenathrene skeleton.

In a further preferred embodiment, the present invention relates to a GLP-1 derivative having a lipophilic substituent which is a straight-chain or branched alkyl group.

In a further preferred embodiment, the present invention relates to a GLP-1 derivative having a lipophilic substituent which is the acyl group of a straight-chain or branched fatty acid.

In a further preferred embodiment, the present invention relates to a GLP-1 derivative having a lipophilic substituent which is an acyl group selected from the group comprising CH₃(CH₂)ₙCO-, wherein n is an integer from 4 to 38, preferably an integer from 4 to 24, more preferred selected from the group comprising CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-.

In a further preferred embodiment, the present invention relates to a GLP-1 derivative having a lipophilic substituent which is an acyl group of a straight-chain or branched alkane α,ω-dicarboxylic acid.

In a further preferred embodiment, the present invention relates to a GLP-1 derivative having a lipophilic substituent which is an acyl group selected from the group comprising HOOC(CH₂)ₘCO-, wherein m is an integer from 4 to 38, preferably an integer from 4 to 24, more preferred selected from the group comprising HOOC(CH₂)₁₄CO-, HOOC(CH₂)₁₆CO-, HOOC(CH₂)₁₈CO-, HOOC(CH₂)₂₀CO- and HOOC(CH₂)₂₂CO-.

In a further preferred embodiment, the present invention relates to a GLP-1 derivative having a lipophilic substituent which is a group of the formula CH₃(CH₂)ₚ((CH₂)_{q}COOH)CHNH-CO(CH₂)₂CO-, wherein p and q are integers and p+q is an integer of from 8 to 33, preferably from 12 to 28.

In a further preferred embodiment, the present invention relates to a GLP-1 derivative having a lipophilic substituent which is a group of the formula CH₃(CH₂)ᵣCO-NHCH(COOH)(CH₂)₂CO-, wherein r is an integer of from 10 to 24.

In a further preferred embodiment, the present invention relates to a GLP-1 derivative having a lipophilic substituent which is a group of the formula CH₃(CH₂)ₛCO-NHCH((CH₂)₂COOH)CO-, wherein s is an integer of from 8 to 24.

In a further preferred embodiment, the present invention relates to a GLP-1 derivative having a lipophilic substituent which is a group of the formula COOH(CH₂)ₜCO- wherein t is an integer of from 8 to 24.

In a further preferred embodiment, the present invention relates to a GLP-1 derivative having a lipophilic substituent which is a group of the formula-NHCH(COOH)(CH₂)₄NH-CO(CH₂)CH₃ wherein u is an integer of from 8 to 18.

In a further preferred embodiment, the present invention relates to a GLP-1 derivative having a lipophilic substituent which is a group of the formula - NHCH(COOH)(CH₂)₄NH-COCH((CH₂)₂COOH)NH-CO(CH₂)_{w}CH₃, wherein w is an integer of from 10 to 16.

In a further preferred embodiment, the present invention relates to a GLP-1 derivative having a lipophilic substituent which is a group of the formula - NHCH(COOH)(CH₂)₄NH-CO(CH₂)₂CH(COOH)NH-CO(CH₂)ₓCH₃, wherein x is an integer of from 10 to 16.

In a further preferred embodiment, the present invention relates to a GLP-1 derivative having a lipophilic substituent which is a group of the formula - NHCH(COOH)(CH₂)₄NH-CO(CH₂)₂CH(COOH)NHCO(CH₂)CH₃, wherein y is zero or an integer of from 1 to 22.

Described herein, but not explicitly claimed, is a GLP-1 derivative having a lipophilic substituent which can be negatively charged. Such a lipophilic substituent can for example be a substituent which has a carboxyl group.

Described herein, but not explicitly claimed, is a GLP-1 derivative the parent peptide of which is selected from the group comprising GLP-1(1-45) or an analogue thereof.

Described herein, but not explicitly claimed, is a GLP-1 derivative derived from a GLP-1 fragment selected from the group comprising GLP-1(7-35), GLP-1(7-36), GLP-1(7-36) amide, GLP-1(7-37), GLP-1(7-38), GLP-1(7-39), GLP-1(7-40) and GLP-1(7-41) or an analogue thereof.

Described herein, but not explicitly claimed, is a GLP-1 analogue derived from a GLP-1 analogue selected from the group comprising GLP-1(1-35), GLP-1(1-36), GLP-1(1-36) amide, GLP-1(1-37), GLP-1(1-38), GLP-1(1-39), GLP-1(1-40) and GLP-1(1-41) or an analogue thereof.

Described herein, but not explicitly claimed, is a GLP-1 derivative wherein the designation analogue comprises derivatives wherein a total of up to fifteen, preferably up to ten amino acid residues have been exchanged with any α-amino acid residue.

Described herein, but not explicitly claimed, is a GLP-1 derivative wherein the designation analogue comprises derivatives wherein a total of up to fifteen, preferably up to ten amino acid residues have been exchanged with any α-amino acid residue which can be coded for by the genetic code.

In a further preferred embodiment, the present invention relates to a GLP-1 derivative wherein the designation analogue comprises derivatives wherein a total of up to six amino acid residues have been exchanged with another α-amino acid residue which can be coded for by the genetic code.

Described herein, but not explicitly claimed, is a parent peptide for a derivative according to the invention is selection from the group comprising Arg²⁶-GLP-1(7-37); Arg³⁴-GLP-1(7-37); Lys³⁶-GLP-1 (7-37); Arg^{26.34}Lys³⁶-GLP-1 (7-37); Arg^{26.34}Lys³⁸GLP-1(7-38); Arg^{26.34}Lys³⁹-GLP-1(7-39); Arg^{26.34}Lys⁴⁰-GLP-1 (7-40); Arg²⁶Lys³⁶-GLP-1(7-37); Arg³⁴Lys³⁶-GLP-1(7-37); Arg²⁶Lys³⁹-GLP-1 (7-39); Arg³⁴Lys⁴⁰-GLP-1(7-40); Arg^{26.34}Lys^{36.39}-GLP-1(7-39); Arg^{26.34}Lys^{36.40}-GLP-1(7-40); Gly⁸Arg26-GLP-1(7-37); Gly⁸Arg³⁴-GLP-1(7-37); Gly⁸Lys³⁶-GLP-1(7-37); Gly⁸Arg^{26.34}Lys³⁶-GLP-1 (7-37); Gly⁸Arg^{26.34}Lys³⁹-GLP-1(7-39); Gly⁸Arg^{26.34}Lys⁴⁰-GLP-1(7-40); Gly⁸Arg²⁶Lys³⁶-GLP-1 (7-37); Gly⁸Arg³⁴Lys³⁶-GLP-1(7-37); Gly⁸Arg²⁶Lys³⁹-GLP-1 (7-39); Gly⁸Arg³⁴Lys⁴⁰-GLP-1 (7-40); Gly⁸Arg^{26.34}Lys^{36.39}-GLP-1 (7-39) and Gly⁸Arg^{26.34}Lys^{36.40}-GLP-1 (7-40).

In a further preferred embodiment, a parent peptide for a derivative according to the invention is selected from the group comprising Arg^{26.34}Lys³⁸GLP-1 (7-38); Arg^{26.34}Lys³⁹GLP-1(7-39); Arg^{26.34}Lys⁴⁰GLP-1 (7-40); Arg^{26.34}Lys⁴¹GLP-1 (7-41); Arg^{26.34}Lys⁴²GLP-1 (7-42); Arg^{26,34}Lys⁴³GLP-1 (7-43); Arg^{26,34}Lys⁴⁴GLP-1(7-44); Arg^{26,34}Lys⁴⁵GLP-1(7-45); Arg^{26.34}Lys³⁸GLP-1(1-38); Arg^{26,34}Lys³⁹GLP-1(1-39); Arg^{26,4}Lys⁴⁰GLP-1(1-40); Arg^{26,34}Lys⁴¹GLP-1(1-41); Arg^{26,34}Lys⁴²GLP-1(1-42); Arg^{26,34}Lys⁴³GLP-1(1-43); Arg^{26,34}Lys⁴⁴GLP-1 (1-44); Arg^{28,34}Lys⁴⁵GLP-1(1-45); Arg^{26,34}Lys³⁸GLP-1(2-38); Arg^{26,34}Lys³⁹GLP-1(2-39); Arg^{26,34}Lys⁴⁰GLP-1(2-40); Arg^{26,34}Lys⁴¹GLP-1(2-41); Arg^{26,34}Lys⁴²GLP-1(2-42); Arg^{26,34}Lys⁴³GLP-1(2-43); Arg^{26,34}Lys⁴⁴GLP-1(2-44); Arg^{26,34}Lys⁴⁵GLP-1(2-45); Arg^{26,34}Lys³⁸GLP-1(3-38); Arg^{26,34}Lys³⁹GLP-1 (3-39); Arg^{26.34}Lys⁴⁰GLP-1(3-40); Arg^{26,34}Lys⁴¹GLP-1(3-41); Arg^{26,34}Lys⁴²GLP-1(3-42); Arg^{26,34}Lys⁴³GLP-1(3-43); Arg^{26,34}Lys⁴⁴GLP-1(3-44); Arg^{26,34}Lys⁴⁵GLP-1(3-45); Arg^{26,34}Lys³⁸GLP-1(4-38); Arg^{26,34}Lys³⁹GLP-1(4-39); Arg^{26,34}Lys⁴⁰GLP-1(4-40); Arg^{26,34}Lys⁴¹GLP-1(4-41); Arg^{26,34}Lys⁴²GLP-1(4-42); Arg^{26,34}Lys⁴³GLP-1(4-43); Arg^{26,34}Lys⁴⁴GLP-1(4-44); Arg^{26,3}4Lys⁴⁵GLP-1(4-45); Arg^{26,34}Lys³⁸GLP-1(5-38); Arg^{26,34}Lys³⁹GLP-1(5-39); Arg^{26,34}Lys⁴⁰GLP-1(5-40); Arg^{26,34}Lys⁴¹GLP-1(5-41); Arg^{26,34}Lys⁴²GLP-1(5-42); Arg^{26,34}Lys⁴³GLP-1(5-43); Arg^{26,34}Lys⁴⁴GLP-1(5-44); Arg^{26,34}Lys⁴⁵GLP-1(5-45); Arg^{26,34}Lys³⁸GLP-1(6-38); Arg^{26,34}Lys³⁹GLP-1(6-39); Arg^{26,34}Lys⁴⁰GLP-1(6-40); Arg^{26,34}Lys⁴¹GLP-1(6-41); Arg^{26,34}Lys⁴²GLP-1(6-42); Arg^{26,34}Lys⁴³GLP-1(6-43); Arg^{26,34}Lys⁴⁴GLP-1(6-44); Arg^{26,34}Lys⁴⁵GLP-1(6-45); Arg²⁶Lys³⁸GLP-1(1-38); Arg³⁴Lys³⁸GLP-1(1-38); Arg^{26,34}Lys^{36,38}GLP-1(1-38); Arg²⁶Lys³⁸GLP-1(7-38); Arg³⁴Lys³⁸GLP-1(7-38); Arg^{26,34}Lys^{36,38}GLP-1(7-38); Arg^{26,34}Lys³⁸GLP-1(7-38); Arg²⁶Lys³⁹GLP-1(1-39); Arg³⁴Lys³⁹GLP-1(1-39); Arg^{26,34}Lys^{36,39}GLP-1(1-39); Arg²⁶Lys³⁹GLP-1(7-39); Arg³⁴Lys³⁹GLP-1(7-39) and Arg^{26,34}Lys^{36,39}GLP-1(7-39).

In a further preferred embodiment, the present invention relates to a GLP-1 derivative wherein the parent peptide is selected from the group comprising Arg²⁶-GLP-1(7-37), Arg³⁴-GLP-1 (7-37), Lys³⁶-GLP-1(7-37), Arg^{28,34}Lys³⁶-GLP-1(7-37), Arg²⁶Lys³⁶-GLP-1(7-37), Arg³⁴Lys³⁶-GLP-1(7-37), Gly⁸Arg²⁶-GLP-1(7-37), Gly⁸Arg³⁴-GLP-1(7-37), Gly⁸Lys³⁶-GLP-1(7-37), Gly⁸Arg^{26,34}Lys³⁶-GLP-1(7-37), Gly⁸Arg²⁶Lys³⁶-GLP-1(7-37) and Gly⁸Arg³⁴Lys³⁶-GLP-1(7-37).

In a further preferred embodiment, the present invention relates to a GLP-1 derivative wherein the parent peptide is selected from the group comprising Arg²⁶Lys³⁸-GLP-1(7-38), Arg^{26,34}Lys³⁸-GLP-1(7-38), Arg^{26,34}Lys^{36,38}-GLP-1(7-38), Gly⁸Arg²⁶Lys³⁸-GLP-1(7-38) and Gly⁸Arg^{26,34}Lys^{36,38}-GLP-1(7-38).

In a further preferred embodiment, the present invention relates to a GLP-1 derivative wherein the parent peptide is selected from the group comprising Arg²⁶Lys³⁹-GLP-1(7-39), Arg^{26,34}Lys^{36,39}-GLP-1(7-39), Gly⁸Arg²⁶Lys³⁹-GLP-1 (7-39) and Gly⁸Arg^{26,34}Lys^{36,39}-GLP-1 (7-39).

In a further preferred embodiment, the present invention relates to a GLP-1 derivative wherein the parent peptide is selected from the group comprising Arg³⁴Lys⁴⁰-GLP-1(7-40), Arg^{26.34}Lys^{36.40}-GLP-1 (7-40), Gly⁸Arg³⁴Lys⁴⁰-GLP-1 (7-40) and Gly⁸Arg^{26.34}Lys^{36.40}-GLP-1 (7-40).

In a further preferred embodiment, the present invention relates to a GLP-1 derivative which is selected from the group comprising:
Lys²⁶(N^{ε}-tetradecanoyl)-GLP-1(7-37);
Lys³⁴(N^{ε}-tetradecanoyl)-GLP-1(7-37);
Lys^{26,34}-bis(N^{ε}-tetradecanoyl)-GLP-1(7-37);
Gly⁸Lys²⁶(N^{ε}-tetradecanoyl)-GLP-1(7-37);
Gly⁸Lys³⁴(N^{ε}-tetradecanoyl)-GLP-1(7-37);
Gly⁸Lys^{26,34}-bis(N^{ε}-tetradecanoyl)-GLP-1(7-37);
Arg²⁶Lys³⁴(N^{ε}-tetradecanoyl)-GLP-1(7-37);
Lys²⁶(N^{ε}-tetradecanoyl)-GLP-1(7-38);
Lys³⁴(N^{ε}-tetradecanoyl)-GLP-1(7-38);
Lys^{26,34}-bis(N^{ε}-tetradecanoyl)-GLP-1(7-38);
Gly⁸Lys²⁶(N^{ε}-tetradecanoyl)-GLP-1(7-38);
Gly⁸Lys³⁴(N^{ε}-tetradecanoyl)-GLP-1(7-38);
Gly⁸Lys^{26.34}-bis(N^{ε}-tetradecanoyl)-GLP-1 (7-38);
Arg²⁶Lys³⁴(N^{ε}-tetradecanoyl)-GLP-1 (7-38);
Lys²⁶(N^{ε}-tetradecanoyl)-GLP-1 (7-39);
Lys³⁴(N^{ε}-tetradecanoyl)-GLP-1 (7-39);
Lys^{26.34}-bis(N^{ε}-tetradecanoyl)-GLP-1 (7-39);
Gly⁸Lys²⁶(N^{ε}-tetradecanoyl)-GLP-1 (7-39);
Gly⁸Lys³⁴(N^{ε}-tetradecanoyl)-GLP-1 (7-39);
Gly⁸Lys^{26.34}-bis(N^{ε}-tetradecanoyl)-GLP-1 (7-39);
Arg²⁶Lys³⁴(N^{ε}-tetradecanoyl)-GLP-1 (7-39);
Lys²⁶(N^{ε}-tetradecanoyl)-GLP-1 (7-40);
Lys³⁴(N^{ε}-tetradecanoyl)-GLP-1(7-40);
Lys²⁶⁻³⁴-bis(N^{ε}-tetradecanoyl)-GLP-1(7-40);
Gly⁸Lys²⁶(N^{ε}-tetradecanoyl)-GLP-1(7-40);
Gly⁸Lys³⁴(N^{ε}-tetradecanoyl)-GLP-1(7-40);
Gly⁸Lys^{26,34}-bis(N^{ε}-tetradecanoyl)-GLP-1(7-40);
Arg²⁶Lys³⁴(N^{ε}-tetradecanoyl)-GLP-1(7-40);
Lys²⁸(N^{ε}-tetradecanoyl)-GLP-1(7-36);
Lys³⁴(N^{ε}-tetradecanoyl)-GLP-1(7-36);
Lys^{26,34}-bis(N^{ε}-tetradecanoyl)-GLP-1(7-36);
Gly⁸Lys²⁶(N^{ε}-tetradecanoyl)-GLP-1(7-36);
Gly⁸Lys³⁴(N^{ε}-tetradecanoyl)-GLP-1(7-36);
Gly⁸Lys^{26,34}-bis(N^{ε}-tetradecanoyl)-GLP-1(7-36);
Arg²⁶Lys³⁴(N^{ε}-tetradecanoyl)-GLP-1(7-36);
Lys²⁶(N^{ε}-tetradecanoyl)-GLP-1(7-35);
Lys³⁴(N^{ε}-tetradecanoyl)-GLP-1(7-35);
Lys^{26,34}-bis(N^{ε}-tetradecanoyl)-GLP-1(7-35);
Gly⁸Lys²⁶(N^{ε}-tetradecanoyl)-GLP-1(7-35);
Gly⁸Lys³⁴(N^{ε}-tetradecanoyl)-GLP-1(7-35);
Gly⁸Lys^{26,34}-bis(N^{ε}-tetradecanoyl)-GLP-1(7-35);
Arg²⁶Lys³⁴(N^{ε}-tetradecanoyl)-GLP-1(7-35);
Lys²⁶(N^{ε}-tetradecanoyl)-GLP-1 (7-36)amide;
Lys³⁴(N^{ε}-tetradecanoyl)-GLP-1 (7-36)amide;
Lys^{26,34}-bis(N^{ε}-tetradecanoyl)-GLP-1 (7-36)amide;
Gly⁸Lys²⁶(N^{ε}-tetradecanoyl)-GLP-1 (7-36)amide;
Gly⁸Lys³⁴(N^{ε}-tetradecanoyl)-GLP-1 (7-36)amide;
Gly⁸Lys^{26.34}-bis(N^{ε}-tetradecanoyl)-GLP-1 (7-36)amide;
Arg²⁶Lys³⁴(N^{ε}-tetradecanoyl)-GLP-1 (7-36)amide;
Gly⁸Arg²⁶Lys³⁴(N^{ε}-tetradecanoyl)-GLP-1 (7-37);
Lys²⁶(N^{ε}-tetradecanoyl)Arg³⁴ -GLP-1 (7-37);
Gly⁸Lys²⁶(N^{ε}-tetradecanoyl)Arg³⁴ -GLP-1 (7-37);
Arg^{26.34}Lys³⁶(N^{ε}-tetradecanoyl)-GLP-1 (7-37);
Gly⁸Arg^{26.34}Lys³⁶(N^{ε}-tetradecanoyl)-GLP-1 (7-37);
Gly⁸Arg²⁶Lys³⁴(N^{ε}-tetradecanoyl)-GLP-1 (7-38);
Lys²⁶(N^{ε}-tetradecanoyl)Arg³⁴-GLP-1(7-38);
Gly⁸Lys²⁶(N^{ε}-tetradecanoyl)Arg³⁴-GLP-1(7-38);
Arg^{26,34}Lys³⁶(N^{ε}-tetradecanoyl)-GLP-1(7-38);
Arg^{26,34}Lys³⁸(N^{ε}-tetradecanoyl)-GLP-1(7-38);
Gly⁸Arg^{26,34}Lys³⁶(N^{ε}-tetradecanoyl)-GLP-1(7-38);
Gly⁸Arg²⁶Lys³⁴(N^{ε}-tetradecanoyl)-GLP-1(7-39);
Lys²⁶(N^{ε}-tetradecanoyl)Arg³⁴-GLP-1(7-39);
Gly⁸Lys²⁶(N^{ε}-tetradecanoyl)Arg³⁴-GLP-1 (7-39);
Arg^{26,34}Lys³⁶(N^{ε}-tetradecanoyl)-GLP-1 (7-39);
Gly⁸Arg^{26,34}Lys³⁶(N^{ε}-tetradecanoyl)-GLP-1 (7-39);
Gly⁸Arg²⁶Lys³⁴(N^{ε}-tetradecanoyl)-GLP-1(7-40);
Lys²⁶(N^{ε}-tetradecanoyl)Arg³⁴-GLP-1(7-40);
Gly⁸Lys²⁶(N^{ε}-tetradecanoyl)Arg³⁴-GLP-1(7-40);
Arg^{26,34}Lys³⁶(N^{ε}-tetradecanoyl)-GLP-1(7-40);
Gly⁸Arg^{26,34}Lys³⁶(N^{ε}-tetradecanoyl)-GLP-1(7-40);
Lys²⁶(N^{ε}-(ω-carboxynonadecanoyl))-GLP-1(7-37);
Lys³⁴(N^{ε}-(ω-carboxynonadecanoyl))-GLP-1(7-37);
Lys^{26,34}-bis(N^{ε}-(ω-carboxynonadecanoyl))-GLP-1 (7-37);
Gly⁸Lys²⁶(N^{ε}-(ω-carboxynonadecanoyl))-GLP-1 (7-37);
Gly⁶Lys³⁴(N^{ε}-(ω-carboxynonadecanoyl))-GLP-1(7-37);
Gly⁸Lys^{26,34}-bis(N^{ε}-(ω-carboxynonadecanoyl))-GLP-1(7-37);
Lys²⁶(N^{ε}-(ω-carboxynonadecanoyl))-GLP-1 (7-38);
Lys³⁴(N^{ε}-(ω-carboxynonadecanoyl))-GLP-1(7-38);
Lys^{26,34}-bis(N^{ε}-(ω-carboxynonadecanoyl))-GLP-1 (7-38);
Gly⁸Lys²⁶(N^{ε}-(ω-carboxynonadecanoyl))-GLP-1 (7-38);
Gly⁸Lys³⁴(N^{ε}-(ω-carboxynonadecanoyl))-GLP-1 (7-38);
Gly⁸Lys^{26,34}-bis(N^{ε}-(ω-carboxynonadecanoyl))-GLP-1 (7-38);
Lys²⁶(N^{ε}-(ω-carboxynonadecanoyl))-GLP-1 (7-39);
Lys³⁴(N^{ε}-(ω-carboxynonadecanoyl))-GLP-1 (7-39);
Lys^{26,34}-bis(N^{ε}-(ω-carboxynonadecanoyl))-GLP-1 (7-39);
Gly⁸Lys²⁶(N^{ε}-(ω-carboxynonadecanoyl))-GLP-1 (7-39);
Gly⁸Lys³⁴(N^{ε}-(ω-carboxynonadecanoyl))-GLP-1 (7-39);
Gly⁸Lys^{26,34}-bis(N^{ε}-(ω-carboxynonadecanoyl))-GLP-1 (7-39);
Lys²⁶(N^{ε}-(ω-carboxynonadecanoyl))-GLP-1 (7-40);
Lys³⁴(N^{ε}-(ω-carboxynonadecanoyl))-GLP-1 (7-40);
Lys^{26,34}-bis(N^{ε}-(ω-carboxynonadecanoyl))-GLP-1 (7-40);
Gly⁸Lys²⁶(N^{ε}-(ω-carboxynonadecanoyl))-GLP-1 (7-40);
Gly⁸Lys³⁴(N^{ε}-(ω-carboxynonadecanoyl))-GLP-1 (7-40);
Gly⁸Lys^{26.34}-bis(N^{ε}-(ω-carboxynonadecanoyl))-GLP-1 (7-40);
Lys²⁸(N^{ε}-(ω-carboxynonadecanoyl))-GLP-1 (7-36);
Lys³⁴(N^{ε}-(ω-carboxynonadecanoyl))-GLP-1 (7-36);
Lys^{26,34}-bis(N^{ε}-(ω-carboxynonadecanoyl))-GLP-1 (7-36);
Gly⁸Lys²⁶(N^{ε}-(ω-carboxynonadecanoyl))-GLP-1 (7-36);
Gly⁸Lys³⁴(N^{ε}-(ω-carboxynonadecanoyl))-GLP-1(7-36);
Gly⁸Lys^{26,34}-bis(N^{ε}-(ω-carboxynonadecanoyl)-GLP-1(7-36);
Lys²⁶(N^{ε}-(ω-carboxynonadecanoyl))-GLP-1(7-36)amide;
Lys³⁴(N^{ε}-(ω-carboxynonadecanoyl))-GLP-1(7-36)amide;
Lys^{26.34} -bis(N^{ε}-(ω-carboxynonadecanoyl))-GLP-1(7-36)amide;
Gly⁸Lys²⁶(N^{ε}-(ω-carboxynonadecanoyl))-GLP-1(7-36)amide;
Gly⁸Lys³⁴(N^{ε}-(ω-carboxynonadecanoyl))-GLP-1(7-36)amide;
Gly⁸Lys^{26,34}-bis(N^{ε}-(ω-carboxynonadecanoyl))-GLP-1(7-36)amide;
Lys²⁸(N^{ε}-(ω-carboxynonadecanoyl))-GLP-1(7-35);
Lys³⁴(N^{ε}-(ω-carboxynonadecanoyl))-GLP-1(7-35);
Lys^{26.34}-bis(N^{ε}-(ω-carboxynonadecanoyl))-GLP-1 (7-35);
Gly⁸Lys²⁶(N^{ε}-(ω-carboxynonadecanoyl))-GLP-1 (7-35);
Gly⁸Lys³⁴(Nε-(ω-carboxynonadecanoyl))-G LP-1 (7-35);
Gly⁸Lys^{26,34}-bis(N^{ε}-(ω-carboxynonadecanoyl))-GLP-1(7-35);
Arg²⁶Lys³⁴(N^{ε}-(ω-carboxynonadecanoyl))-GLP-1(7-37);
Gly⁸Arg²⁶Lys³⁴(N^{ε}-(ω-carboxynonadecanoyl))-GLP-1(7-37);
Lys²⁸(N^{ε}-(ω-carboxynonadecanoyl))Arg³⁴-GLP-1(7-37);
Gly⁸Lys²⁶(N^{ε}(ω-carboxynonadecanoyl))Arg³⁴-GLP-1(7-37) ;
Arg^{26,34}Lys³⁶(N^{ε}-(ω-carboxynonadecanoyl))-GLP-1(7-37);
Gly⁸Arg^{26,34}Lys³⁶(N^{ε}-(ω-carboxynonadecanoyl))-GLP-1(7-31);
Arg²⁶Lys³⁴(N^{ε}-(ω-carboxynonadacanoyl))-GLP-1(7-38);
Gly⁸Arg²⁶Lys³⁴(N^{ε}-(ω-carboxynonadecanoyl))-GLP-1(7-38);
Lys²⁶(N^{ε}-(ω-carboxynonadecanoyl))Arg³⁴-GLP-1(7-38);
Gly³Lys²⁶(N^{ε}-(ω-carboxynonadecanoyl))Arg³⁴-GLP-1(7-38);
Arg^{26,34}Lys²⁶(N^{ε}-(ω-carboxynonadecanoyl))-GLP-1(7-38);
Gly⁶Arg^{26,34}Lys³⁶(N^{ε}-(ω-carboxynonadecanoyl))-GLP-1(7-38);
Arg²⁶Lys³⁴(N^{ε}-(ω-carboxynonadecanoyl))-GLP-1(7-39);
Gly⁶Arg²⁶Lys³⁴(N^{ε}-(ω-carboxynonadecanoyl))-GLP-1(7-39);
Lys²⁶(N^{ε}-(ω-carboxynonadecanoyl))Arg³⁴-GLP-1(7-39);
Gly⁶Lys²⁶(N^{ε}-(ω-carboxynonadecanoyl))Arg³⁴-GLP-1(7-39);
Arg^{26,34}Lys³⁵(N^{ε}-(ω-carboxynonadecanoyl))-GLP-1(7-39);
Gly⁶Arg^{26,34}Lys³⁶(N^{ε}(ω-carboxynonadecanoyl))-GLP-1(7-39);
Arg²⁶Lys³⁴(N^{ε}-(ω-carboxynonadecanoyl))-GLP-1(7-40);
Gly⁸Arg²⁶Lys³⁴(N^{ε}-(ω-carboxynonadecanoyl))-GLP-1(7-40);
Lys²⁵(N^{ε}-(ω-carboxynonadecanoyl))Arg³⁴-GLP-1(7-40);
Gly⁸Lys²⁶(N^{ε}-(ω-carboxynonadecanoyl))Arg³⁴-GLP-1(7-40);
Arg^{26,34}Lys³⁶(N^{ε}-(ω-carboxynonadecanoyl))-GLP-1(7-40);
Gly⁸Arg^{26,34}Lys³⁸(N^{ε}-(ω-carboxynonadecanoyl))-GLP-1(7-40);
Lys²⁸(N^{ε}-(7-deoxycholoyl))-GLP-1(7-37);
Lys³⁴(N^{ε}-(7-deoxycholoyl))-GLP-1(7-37);
Lys^{26,34}-bis(N^{ε}-(7-deoxycholoyl))-GLP-1(7-37);
Gly⁶Lys²⁰(N^{ε}-(7-deoxycholoyl))-GLP-1(7-37);
Gly⁶Lys³⁴(N^{ε}-(7-deoxycholoyl))-GLP-1(7-37);
Gly⁸Lys^{26,34}-bis(N^{ε}-(7-deoxycholoyl))-GLP-1(7-37);
Arg²⁸Lys³⁴(N^{ε}-(7-deoxycholoyl))-GLP-1(7-37);
Lys²⁶(N^{ε}-(7-deoxycholoyl))-GLP-1 (7-38);
Lys³⁴(N^{ε}-(7-deoxycholoyl))-GLP-1 (7-38);
Lys^{26,34}-bis(N^{ε}-(7-deoxycholoyl))-GLP-1 (7-38);
Gly⁸Lys²⁶(N^{ε}-(7-deoxycholoyl))-GLP-1 (7-38);
Gly⁸Lys³⁴(N^{ε}-(7-deoxycholoyl))-GLP-1 (7-38);
Gly⁸Lys^{26,34}-bis(N^{ε}-(7-deoxycholoyl))-GLP-1 (7-38);
Arg²⁶Lys³⁴(N^{ε}-(7-deoxycholoyl))-GLP-1 (7-38);
Lys²⁶(N^{ε}-(7-deoxycholoyl))-GLP-1 (7-39);
Lys³⁴(N^{ε}-(7-deoxycholoyl))-GLP-1 (7-39);
Lys^{26,34}-bis(N^{ε}-(7-deoxycholoyl))-GLP-1 (7-39);
Gly⁸Lys²⁶(N^{ε}-(7-deoxycholoyl))-GLP-1 (7-39);
Gly⁸Lys³⁴(N^{ε}-(7-deoxycholoyl))-GLP-1(7-39);
Gly⁸Lys^{26,34}-bis(N^{ε}-(7-deoxycholoyl))-GLP-1(7-39);
Arg²⁶Lys³⁴(N^{ε}-(7-deoxycholoyl))-GLP-1(7-39);
Lys²⁶(N^{ε}-(7-deoxycholoyl))-GLP-1 (7-40);
Lys³⁴(N^{ε}-(7-deoxycholoyl))-GLP-1(7-40);
Lys^{26,34}-bis(N^{ε}-(7-deoxycholoyl))-GLP-1(7-40);
Gly⁸Lys²⁶(N^{ε}-(7-deoxycholoyl))-GLP-1 (7-40);
Gly⁸Lys³⁴(N^{ε}-(7-deoxycholoyl))-GLP-1 (7-40);
Gly⁸Lys^{26,34}-bis(N^{ε}-(7-deoxycholoyl))-GLP-1(7-40);
Arg²⁶Lys³⁴(N^{ε}-(7-deoxycholoyl))-GLP-1(7-40);
Lys²⁶(N^{ε}-(7-deoxycholoyl))-GLP-1(7-36);
Lys³⁴(N^{ε}-(7-deoxycholoyl))-GLP-1(7-36);
Lys^{26,34}-bis(N^{ε}-(7-deoxycholoyl))-GLP-1(7-36);
Gly⁸Lys²⁶(N^{ε}-(7-deoxycholoyl))-GLP-1(7-36);
Gly⁸Lys³⁴(N^{ε}-(7-deoxycholoyl))-GLP-1(7-36);
Gly⁸Lys^{26,34}-bis(N^{ε}-(7-deoxycholoyl))-GLP-1(7-36);
Arg²⁶Lys³⁴(N^{ε}-(7-deoxycholoyl))-GLP-1(7-36);
Lys²⁶(N^{ε}-(7-deoxycholoyl))-GLP-1(7-35);
Lys³⁴(N^{ε}-(7-deoxycholoyl))-GLP-1(7-35);
Lys^{26,34}-bis(N^{ε}-(7-deoxycholoyl))-GLP-1(7-35);
Gly⁸Lys²⁶(N^{ε}-(7-deoxycholoyl))-GLP-1(7-35);
Gly⁸Lys³⁴(N^{ε}-(7-deoxycholoyl))-GLP-1(7-35);
Gly⁸Lys^{26,34}-bis(N^{ε}-(7-deoxycholoyl))-GLP-1(7-35);
Arg²⁶Lys³⁴(N^{ε}-(7-deoxycholoyl))-GLP-1(7-35);
Lys²⁶(N^{ε}-(7-deoxycholoyl))-GLP-1(7-36)amide;
Lys³⁴(N^{ε}-(7-deoxycholoyl))-GLP-1(7-36)amide;
Lys^{26,34}-bis(N^{ε}-(7-deoxycholoyl))-GLP-1(7-36)amide;
Gly⁸Lys²⁶(N^{ε}-(7-deoxycholoyl))-GLP-1(7-36)amide;
Gly⁸Lys³⁴(N^{ε}-(7-deoxycholoyl))-GLP-1(7-36)amide;
Gly⁸Lys^{26,34}-bis(N^{ε}-(7-deoxycholoyl))-GLP-1(7-36)amide;
Arg²⁶Lys³⁴(N^{ε}-(7-deoxycholoyl))-GLP-1(7-36)amide;
Gly⁸Arg²⁶Lys³⁴(N^{ε}-(7-deoxycholoyl))-GLP-1(7-37);
Lys²⁶(N^{ε}-(7-deoxycholoyl))Arg³⁴-GLP-1 (7-37);
Gly⁸Lys²⁶(N^{ε}-(7-deoxycholoyl))Arg³⁴-GLP-1 (7-37);
Arg^{26,34}Lys³⁶(N^{ε}-(7-deoxycholoyl))-GLP-1 (7-37);
Gly⁸Arg^{26,34}Lys³⁶(N^{ε}-(7-deoxycholoyl))-GLP-1 (7-37);
Lys²⁶(N^{ε}-(choloyl))-GLP-1 (7-37);
Lys³⁴(N^{ε}-(choloyl))-GLP-1 (7-37);
Lys^{26,34}-bis(N^{ε}-(choloyl))-GLP-1 (7-37);
Gly⁸Lys²⁶(N^{ε}-(choloyl))-GLP-1 (7-37);
Gly⁸Lys³⁴(N^{ε}-(choloyl))-GLP-1 (7-37);
Gly⁸Lys^{26,34}-bis(N^{ε}-(choloyl))-GLP-1 (7-37);
Arg²⁶Lys³⁴(N^{ε}-(choloyl))-GLP-1 (7-37);
Gly⁸Arg²⁶Lys³⁴(N^{ε}-(7-deoxycholoyl))-GLP-1 (7-38);
Lys²⁶(N^{ε}-(7-deoxycholoyl))Arg³⁴-GLP-1 (7-38);
Gly⁸Lys²⁶(N^{ε}-(7-deoxycholoyl))Arg³⁴-GLP-1 (7-38);
Arg^{26,34}Lys³⁶(N^{ε}-(7-deoxycholoyl))-GLP-1 (7-38);
Arg^{26,34}Lys³⁸(N^{ε}-(7-deoxycholoyl))-GLP-1 (7-38);
Gly⁸Arg^{26,34}lys³⁶(N^{ε}-(7-deoxycholoyl))-GLP-1 (7-38);
Lys²⁶(N^{ε}-(choloyl))-GLP-1 (7-38);
Lys³⁴(N^{ε}-(choloyl))-GLP-1 (7-38);
Lys^{26,34}-bis(N^{ε}-(choloyl))-GLP-1 (7-38);
Gly⁸Lys²⁶(N^{ε}-(choloyl))-GLP-1 (7-38);
Gly⁸Lys³⁴(N^{ε}-(choloyl))-GLP-1 (7-38);
Gly⁸Lys^{26,34}-bis(N^{ε}-(choloyl))-GLP-1 (7-38);
Arg²⁶Lys³⁴(N^{ε}-(choloyl))-GLP-1 (7-38);
Gly⁸Arg²⁶Lys³⁴(N^{ε}-(7-deoxycholoyl))-GLP-1 (7-39);
Lys²⁶(N^{ε}-(7-deoxycholoyl))Arg³⁴-GLP-1(7-39);
Gly⁸Lys²⁶(N^{ε}-(7-deoxycholoyl))Arg³⁴-GLP-1(7-39);
Arg^{26,34}Lys³⁶(N^{ε}-(7-deoxycholoyl))-GLP-1(7-39);
Gly⁸Arg^{26,34}Lys³⁶(N^{ε}-(7-deoxycholoyl))-GLP-1(7-39);
Lys²⁶(N^{ε}-(choloyl))-GLP-1(7-39);
Lys³⁴(N^{ε}-(choloyl))-GLP-1(7-39);
Lys^{26,34}-bis(N^{ε}-(choloyl))-GLP-1(7-39);
Gly⁸Lys²⁶(N^{ε}-(choloyl))-GLP-1(7-39);
Gly⁸Lys³⁴(N^{ε}-(choloyl))-GLP-1(7-39);
Gly⁸Lys^{26,34}-bis(N^{ε}-(choloyl))-GLP-1(7-39);
Arg²⁶Lys³⁴(N^{ε}-(choloyl))-GLP-1(7-39);
Gly⁸Arg²⁶Lys³⁴(N^{ε}-(7-deoxycholoyl))-GLP-1(7-40);
Lys²⁶(N^{ε}-(7-deoxycholoyl))Arg³⁴-GLP-1(7-40);
Gly⁸Lys²⁶(N^{ε}-(7-deoxycholoyl))Arg³⁴-GLP-1 (7-40);
Arg^{26,34}Lys³⁶(N^{ε}-(7-deoxycholoyl))-GLP-1 (7-40);
Gly⁸Arg^{26,34}Lys³⁶(N^{ε}-(7-deoxycholoyl))-GLP-1(7-40);
Lys²⁶(N^{ε}-(choloyl))-GLP-1(7-40);
Lys³⁴(N^{ε}-(choloyl))-GLP-1(7-40);
Lys^{26,34}-bis(N^{ε}-(choloyl))-GLP-1(7-40);
Gly⁸Lys²⁶(N^{ε}-(choloyl))-GLP-1(7-40);
Gly⁸Lys³⁴(N^{ε}-(choloyl))-GLP-1(7-40);
Gly⁸Lys^{26,34}-bis(N^{ε}-(choloyl))-GLP-1(7-40);
Arg²⁶Lys³⁴(N^{ε}-(choloyl))-GLP-1(7-40);
Lys²⁶(N^{ε}-(choloyl))-GLP-1(7-36);
Lys³⁴(N^{ε}-(choloyl))-GLP-1(7-36);
Lys^{26,34}-bis(N^{ε}-(choloyl))-GLP-1(7-36);
Gly⁸Lys²⁶(N^{ε}-(choloyl))-GLP-1(7-36);
Gly⁸Lys³⁴(N^{ε}-(choloyl))-GLP-1(7-36);
Gly⁸Lys^{26,34}-bis(N^{ε}-(choloyl))-GLP-1 (7-36);
Arg²⁶Lys³⁴(N^{ε}-(choloyl))-GLP-1 (7-36);
Lys²⁶(N^{ε}-(choloyl))-GLP-1 (7-35);
Lys³⁴(N^{ε}-(choloyl))-GLP-1 (7-35);
Lys^{26,34}-bis(N^{ε}-(choloyl))-GLP-1(7-35);
Gly⁸Lys²⁸(N^{ε}-(choloyl))-GLP-1(7-35);
Gly⁸Lys³⁴(N^{ε}(choloyl))-GLP-1 (7-35);
Gly⁸Lys^{26,34}-bis(N^{ε}-(choloyl))-GLP-1 (7-35);
Arg²⁶Lys³⁴(N^{ε}-(choloyl))-GLP-1 (7-35);
Lys²⁶(N^{ε}-(choloyl))-GLP-1 (7-36)amide;
Lys³⁴(N^{ε}-(choloyl))-GLP-1 (7-36)amide;
Lys^{26,34}-bis(N^{ε}-(choloyl))-GLP-1 (7-36)amide;
Gly⁸Lys²⁶(N^{ε}-(choloyl))-GLP-1(7-36)amide;
Gly⁸Lys³⁴(N^{ε}-(choloyl))-GLP-1(7-36)amide;
Gly⁸Lys^{26,34}-bis(N^{ε}-(choloyl))-GLP-1(7-36)amide;
Arg²⁶Lys³⁴(N^{ε}-(choloyl))-GLP-1(7-36)amide;
Gly⁸Arg²⁶Lys³⁴(N^{ε}-(choloyl))-GLP-1(7-37);
Lys²⁶(N^{ε}-(choloyl))Arg³⁴-GLP-1 (7-37);
Gly⁸Lys²⁶(N^{ε}-(choloyl))Arg³⁴-GLP-1(7-37);
Arg^{26,34}Lys³⁶(N^{ε}-(choloyl))-GLP-1(7-37);
Gly⁸Arg^{26,34}LyS³⁶(N^{ε}-(choloyl))-GLP-1(7-37);
Lys²⁶(N^{ε}-(lithocholoyl))-GLP-1(7-37);
Lys³⁴(N^{ε}-(lithocholoyl))-GLP-1(7-37);
Lys^{26,34}-bis(N^{ε}-(lithocholoyl))-GLP-1(7-37);
Gly⁸Lys²⁶(N^{ε}-(lithocholoyl))-GLP-1(7-37);
Gly⁸Lys³⁴(N^{ε}-(lithocholoyl))-GLP-1(7-37);
Gly⁸Lys^{26,34}-bis(N^{ε}-(lithocholoyl))-GLP-1(7-37);
Arg²⁶Lys³⁴(N^{ε}-(lithocholoyl))-GLP-1(7-37);
Gly⁸Arg²⁶Lys³⁴(N^{ε}-(choloyl))-GLP-1(7-38);
Lys²⁶(N^{ε}-(choloyl))Arg³⁴-GLP-1(7-38);
Gly⁸Lys²⁶(N^{ε}-(choloyl))Arg³⁴-GLP-1(7-38);
Arg^{26,34}Lys³⁶(N^{ε}-(choloyl))-GLP-1(7-38);
Arg^{26,34}Lys³⁸(N^{ε}-(choloyl))-GLP-1(7-38);
Gly⁸Arg^{26,34}Lys³⁶(N^{ε}-(choloyl))-GLP-1 (7-38);
Lys²⁶(N^{ε}-(lithocholoyl))-GLP-1 (7-38);
Lys³⁴(N^{ε}-(lithocholoyl))-GLP-1 (7-38);
Lys^{26,34}-bis(N^{ε}-(lithocholoyl))-GLP-1 (7-38);
Gly⁸Lys²⁶(N^{ε}-(lithocholoyl))-GLP-1(7-38);
Gly⁸Lys³⁴(N^{ε}-(lithocholoyl))-GLP-1 (7-38);
Gly⁸Lys^{26,34}-bis(N^{ε}-(lithocholoyl))-GLP-1 (7-38);
Arg²⁶Lys³⁴(N^{ε}-(lithocholoyl))-GLP-1 (7-38);
Gly⁸Arg²⁶Lys³⁴(N^{ε}-(choloyl))-GLP-1 (7-39);
Lys²⁶(N^{ε}-(choloyl))Arg³⁴-GLP-1 (7-39);
Gly⁸Lys²⁶(N^{ε}-(choloyl))Arg³⁴-GLP-1 (7-39);
Arg^{26,34}Lys³⁶(N^{ε}-(choloyl))-GLP-1(7-39);
Gly⁸Arg^{26,34}Lys³⁶(N^{ε}-(choloyl))-GLP-1(7-39);
Lys²⁶(N^{ε}-(lithocholoyl))-GLP-1(7-39);
Lys³⁴(N^{ε}-(lithocholoyl))-GLP-1(7-39);
Lys^{26,34}-bis(N^{ε}-(lithocholoyl))-GLP-1(7-39);
Gly⁸Lys²⁶(N^{ε}-(lithocholoyl))-GLP-1(7-39);
Gly⁸Lys³⁴(N^{ε}-(lithocholoyl))-GLP-1(7-39);
Gly⁸Lys^{26,34}bis(N^{ε}-(lithocholoyl))-GLP-1(7-39);
Arg²⁶Lys³⁴(N^{ε}-(lithocholoyl))-GLP-1(7-39);
Gly⁸Arg²⁶Lys³⁴(N^{ε}-(choloyl))-GLP-1(7-40);
Lys²⁶(N^{ε}-(choloyl))Arg³⁴-GLP-1(7-40);
Gly⁸Lys²⁶(N^{ε}-(choloyl))Arg³⁴-GLP-1(7-40);
Arg^{26,34}Lys³⁶(N^{ε}-(choloyl))-GLP-1 (7-40);
Gly⁸Arg^{26,34}Lys³⁶(N^{ε}-(choloyl))-GLP-1 (7-40);
Lys²⁶(N^{ε}-(lithocholoyl))-GLP-1 (7-40);
Lys³⁴(N^{ε}-(lithocholoyl))-GLP-1 (7-40);
Lys^{26,34}-bis(N^{ε}-(lithocholoyl))-GLP-1 (7-40);
Gly⁸Lys²⁶(N^{ε}-(lithocholoyl))-GLP-1 (7-40);
Gly⁸Lys³⁴(N^{ε}-(lithocholoyl))-GLP-1 (7-40);
Gly⁸Lys^{26,34}-bis(N^{ε}-(lithocholoyl))-GLP-1 (7-40);
Arg²⁶Lys³⁴(N^{ε}-(lithocholoyl))-GLP-1 (7-37);
Lys²⁶(N^{ε}-(lithocholoyl))-GLP-1 (7-36);
Lys³⁴(N^{ε}-(lithocholoyl))-GLP-1(7-36);
Lys^{26,34}-bis(N^{ε}-(lithocholoyl))-GLP-1(7-36);
Gly⁸Lys²⁶(N^{ε}-(lithocholoyl))-GLP-1(7-36);
Gly⁸Lys³⁴(N^{ε}-(lithocholoyl) )-GLP-1 (7-36);
Gly⁸Lys^{26,34}-bis(N^{ε}-(lithocholoyl))-GLP-1 (7-36);
Arg²⁶Lys³⁴(N^{ε}-(lithocholoyl))-GLP-1 (7-36);
Lys²⁶(N^{ε}-(lithocholoyl))-GLP-1 (7-35);
Lys³⁴(N^{ε}-(lithocholoyl))-GLP-1 (7-35);
Lys^{26,34}-bis(N^{ε}-(lithocholoyl))-GLP-1 (7-35);
Gly⁸Lys²⁶(N^{ε}-(lithocholoyl))-GLP-1 (7-35);
Gly⁸Lys³⁴(N^{ε}-(lithocholoyl))-GLP-1 (7-35);
Gly⁸Lys^{26,34}-bis(N^{ε}-(lithocholoyl))-GLP-1 (7-35);
Arg²⁶Lys³⁴(N^{ε}-(lithocholoyl))-GLP-1 (7-35);
Lys²⁶(N^{ε}-(lithocholoyl))-GLP-1 (7-36)amide;
Lys³⁴(N^{ε}-(lithocholoyl))-GLP-1 (7-36)amide;
Lys^{26,34}-bis(N^{ε}-(lithocholoyl))-GLP-1 (7-36)amide;
Gly⁸Lys²⁶(N^{ε}-(lithocholoyl))-GLP-1 (7-36)amide;
Gly⁸Lys³⁴(N^{ε}-(lithocholoyl))-GLP-1 (7-36)amide;
Gly⁸Lys^{26,34}-bis(N^{ε}-(lithocholoyl))-GLP-1 (7-36)amide;
Arg²⁶Lys³⁴(N^{ε}-(lithocholoyl))-GLP-1 (7-36)amide;
Gly⁸Arg²⁶Lys³⁴(N^{ε}-(lithocholoyl))-GLP-1 (7-37);
Lys²⁶(N^{ε}-(lithocholoyl))Arg³⁴-GLP-1(7-37);
Gly⁸Lys²⁶(N^{ε}-(lithocholoyl))Arg³⁴-GLP-1 (7-37);
Arg^{26,34}Lys³⁶(N^{ε}-(lithocholoyl))-GLP-1(7-37);
Arg^{26,34}Lys³⁸(N^{ε}-(lithocholoyl))-GLP-1(7-37);
Gly⁸Arg^{26,34}Lys³⁶(N^{ε}-(lithocholoyl))-GLP-1(7-37);
Gly⁸Arg²⁶Lys³⁴(N^{ε}-(lithocholoyl))-GLP-1(7-38);
Lys²⁶(N^{ε}-(lithocholoyl))Arg³⁴-GLP-1(7-38);
Gly⁸Lys²⁶(N^{ε}-(lithocholoyl))Arg³⁴-GLP-1(7-38);
Arg^{26,34}Lys³⁶(N^{ε}-(lithocholoyl))-GLP-1(7-38);
Arg^{26,34}Lys³⁸(N^{ε}-(lithocholoyl))-GLP-1(7-38);
Gly⁸Arg^{26,34}Lys³⁶(N^{ε}-(lithocholoyl))-GLP-1(7-38);
Gly⁸Arg²⁶Lys³⁴(N^{ε}-(lithocholoyl))-GLP-1(7-39);
Lys²⁶(N^{ε}-(lithocholoyl))Arg³⁴-GLP-1(7-39);
Gly⁸Lys²⁶(N^{ε}-(lithocholoyl))Arg³⁴-GLP-1(7-39);
Arg^{26,34}Lys³⁶(N^{ε}-(lithocholoyl))-GLP-1(7-39);
Gly⁸Arg^{26,34}Lys³⁶(N'-(lithocholoyl))-GLP-1(7-39);
Gly⁸Arg²⁶Lys³⁴(N^{ε}-(lithocholoyl))-GLP-1(7-40);
Lys²⁶(N^{ε}-(lithocholoyl))Arg³⁴-GLP-1(7-40);
Gly⁸Lys²⁶(N^{ε}-(lithocholoyl))Arg³⁴-GLP-1(7-40);
Arg^{26,34}Lys³⁶(N^{ε}-(lithocholoyl))-GLP-1(7-40) and
Gly⁸Arg^{26.34}Lys³⁶(N^{ε}-(lithocholoyl))-GLP-1(7-40).

In a further preferred embodiment, the present invention relates to a pharmaceutical composition comprising a GLP-1 derivative and a pharmaceutically acceptable vehicle or carrier.

In a further preferred embodiment, the present invention relates to the use of a GLP-1 derivative according to the invention for the preparation of a medicament which has a protracted profile of action relative to GLP-1 (7-37).

In a further preferred embodiment, the present invention relates to the use of a GLP-1 derivative according to the invention for the preparation of a medicament with protracted effect for the treatment of non-insulin dependent diabetes mellitus.

In a further preferred embodiment, the present invention relates to the use of a GLP-1 derivative according to the invention for the preparation of a medicament with protracted effect for the treatment of insulin dependent diabetes mellitus.

In a further preferred embodiment, the present invention relates to the use of a GLP-1 derivative according to the invention for the preparation of a medicament with protracted effect for the treatment of obesity.

In a further preferred embodiment, the present invention relates to a method of treating insulin dependent or non-insulin dependent diabetes mellitus in a patient in need of such a treatment, comprising administering to the patient a therapeutically effective amount of a GLP-1 derivative according to claim 1 together with a pharmaceutically acceptable carrier.

### DETAILED DESCRIPTION OF THE INVENTION

To obtain a satisfactory protracted profile of action of the GLP-1 derivative, the lipophilic substituent attached to the GLP-1 moiety preferably comprises 4-40 carbon atoms, in particular 8-25 carbon atoms. The lipophilic substituent may be attached to an amino group of the GLP-1 moiety by means of a carboxyl group of the lipophilic substituent which forms an amide bond with an amino group of the amino acid residue to which it is attached. Alternatively, the lipophilic substituent may be attached to said amino acid residue in such a way that an amino group of the lipophilic substituent forms an amide bond with a carboxyl group of the amino acid residue. As a further option, the lipophilic substituent may be linked to the GLP-1 moiety via an ester bond. Formally, the ester can be formed either by reaction between a carboxyl group of the GLP-1 moiety and a hydroxyl group of the substituent-to-be or by reaction between a hydroxyl group of the GLP-1 moiety and a carboxyl group of the substituent-to-be. As a further alternative, the lipophilic substituent can be an alkyl group which is introduced into a primary amino group of the GLP-1 moiety.

In one preferred embodiment of the invention, the lipophilic substituent is attached to the GLP-1 moiety by means of a spacer in such a way that a carboxyl group of the spacer forms an amide bond with an amino group of the GLP-1 moiety. Examples of suitable spacers are succinic acid, Lys, Glu or Asp, or a dipeptide such as Gly-Lys. When the spacer is succinic acid, one carboxyl group thereof may form an amide bond with an amino group of the amino acid residue, and the other carboxyl group thereof may form an amide bond with an amino group of the lipophilic substituent. When the spacer is Lys, Glu or Asp, the carboxyl group thereof may form an amide bond with an amino group of the amino acid residue, and the amino group thereof may form an amide bond with a carboxyl group of the lipophilic substituent. When Lys is used as the spacer, a further spacer may in some instances be inserted between the ε-amino group of Lys and the lipophilic substituent. In one preferred embodiment, such a further spacer is succinic acid which forms an amide bond with the ε-amino group of Lys and with an amino group present in the lipophilic substituent. In another preferred embodiment such a further spacer is Glu or Asp which forms an amide bond with the ε-amino group of Lys and another amide bond with a carboxyl group present in the lipophilic substituent, that is, the lipophilic substituent is a N^{ε}-acylated lysine residue.

In another preferred embodiment of the present invention, the lipophilic substituent has a group which can be negatively charged. One preferred group which can be negatively charged is a carboxylic acid group.

The parent peptide can be produced by a method which comprises culturing a host cell containing a DNA sequence encoding the polypeptide and capable of expressing the polypeptide in a suitable nutrient medium under conditions permitting the expression of the peptide, after which the resulting peptide is recovered from the culture.

The medium used to culture the cells may be any conventional medium suitable for growing the host cells, such as minimal or complex media containing appropriate supplements. Suitable media are available from commercial suppliers or may be prepared according to published recipes (*e.g.* in catalogues of the American Type Culture Collection). The peptide produced by the cells may then be recovered from the culture medium by conventional procedures including separating the host cells from the medium by centrifugation or filtration, precipitating the proteinaceous components of the supernatant or filtrate by means of a salt, *e.g.* ammonium sulphate, purification by a variety of chromatographic procedures, e.g. ion exchange chromatography, gel filtration chromatography, affinity chromatography, or the like, dependent on the type of peptide in question.

The DNA sequence encoding the parent peptide may suitably be of genomic or cDNA origin, for instance obtained by preparing a genomic or cDNA library and screening for DNA sequences coding for all or part of the peptide by hybridisation using synthetic oligonucleotide probes in accordance with standard techniques (see, for example, Sambrook, J, Fritsch, EF and Maniatis, T, *Molecular Cloning: A Laboratory Manual,* Cold Spring Harbor Laboratory Press, New York, 1989). The DNA sequence encoding the peptide may also be prepared synthetically by established standard methods, e.g. the phosphoamidite method described by Beaucage and Caruthers, *Tetrahedron Letters* **22** (1981), 1859 - 1869, or the method described by Matthes *et al., EMBO Journal* **3** (1984), 801 - 805. The DNA sequence may also be prepared by polymerase chain reaction using specific primers, for instance as described in US 4,683,202 or Saiki *et al., Science* **239** (1988), 487 - 491.

The DNA sequence may be inserted into any vector which may conveniently be subjected to recombinant DNA procedures, and the choice of vector will often depend on the host cell into which it is to be introduced. Thus, the vector may be an autonomously replicating vector, *i.e.* a vector which exists as an extrachromosomal entity, the replication of which is independent of chromosomal replication, *e.g.* a plasmid. Alternatively, the vector may be one which, when introduced into a host cell, is integrated into the host cell genome and replicated together with the chromosome(s) into which it has been integrated.

The vector is preferably an expression vector in which the DNA sequence encoding the peptide is operably linked to additional segments required for transcription of the DNA, such as a promoter. The promoter may be any DNA sequence which shows transcriptional activity in the host cell of choice and may be derived from genes encoding proteins either homologous or heterologous to the host cell. Examples of suitable promoters for directing the transcription of the DNA encoding the peptide of the invention in a variety of host cells are well known in the art, cf. for instance Sambrook *et al., supra.*

The DNA sequence encoding the peptide may also, if necessary, be operably connected to a suitable terminator, polyadenylation signals, transcriptional enhancer sequences, and translational enhancer sequences. The recombinant vector of the invention may further comprise a DNA sequence enabling the vector to replicate in the host cell in question.

The vector may also comprise a selectable marker, *e.g.* a gene the product of which complements a defect in the host cell or one which confers resistance to a drug, *e.g.* ampicillin, kanamycin, tetracyclin, chloramphenicol, neomycin, hygromycin or methotrexate.

To direct a parent peptide of the present invention into the secretory pathway of the host cells, a secretory signal sequence (also known as a leader sequence, prepro sequence or pre sequence) may be provided in the recombinant vector. The secretory signal sequence is joined to the DNA sequence encoding the peptide in the correct reading frame. Secretory signal sequences are commonly positioned 5' to the DNA sequence encoding the peptide. The secretory signal sequence may be that normally associated with the peptide or may be from a gene encoding another secreted protein.

The procedures used to ligate the DNA sequences coding for the present peptide, the promoter and optionally the terminator and/or secretory signal sequence, respectively, and to insert them into suitable vectors containing the information necessary for replication, are well known to persons skilled in the art (cf., for instance, Sambrook *et al.., supra).*

The host cell into which the DNA sequence or the recombinant vector is introduced may be any cell which is capable of producing the present peptide and includes bacteria, yeast, fungi and higher eukaryotic cells. Examples of suitable host cells well known and used in the art are, without limitation, *E. coli, Saccharomyces cerevisiae,* or mammalian BHK or CHO cell lines.

Examples of compounds which can be useful as GLP-1 moieties according to the present invention are described in International Patent Application No. WO 87/06941 (The General Hospital Corporation) which relates to a peptide fragment which comprises GLP-1 (7-37) and functional derivatives thereof and to its use as an insulinotropic agent.

Further GLP-1 analogues are described in International Patent Application No. 90/11296 (The General Hospital Corporation) which relates to peptide fragments which comprise GLP-1 (7-36) and functional derivatives thereof and have an insulinotropic activity which exceeds the insulinotropic activity of GLP-1(1-36) or GLP-1(1-37) and to their use as insulinotropic agents.

International Patent Application No. 91/11457 (Buckley *et al..*) discloses analogues of the active GLP-1 peptides 7-34, 7-35, 7-36, and 7-37 which can also be useful as GLP-1 moieties according to the present invention.

### Pharmaceutical compositions

Pharmaceutical compositions containing a GLP-1 derivative according to the present invention may be administered parenterally to patients in need of such a treatment. Parenteral administration may be performed by subcutaneous, intramuscular or intravenous injection by means of a syringe, optionally a pen-like syringe. Alternatively, parenteral administration can be performed by means of an infusion pump. A further option is a composition which may be a powder or a liquid for the administration of the GLP-1 derivative in the form of a nasal or pulmonal spray. As a still further option, the GLP-1 derivatives of the invention can also be administered transdermally, *e.g.* from a patch, optionally a iontophoretic patch, or transmucosally, *e.g.* bucally.

Pharmaceutical compositions containing a GLP-1 derivative of the present invention may be prepared by conventional techniques, *e.g.* as described in Remington's *Pharmaceutical Sciences,* 1985 or in Remington: *The Science and Practice of Pharmacy,* 19^{th} edition, 1995.

Thus, the injectable compositions of the GLP-1 derivative of the invention can be prepared using the conventional techniques of the pharmaceutical industry which involves dissolving and mixing the ingredients as appropriate to give the desired end product.

According to one procedure, the GLP-1 derivative is dissolved in an amount of water which is somewhat less than the final volume of the composition to be prepared. An isotonic agent, a preservative and a buffer is added as required and the pH value of the solution is adjusted - if necessary - using an acid, *e.g.* hydrochloric acid, or a base, *e.g.* aqueous sodium hydroxide as needed. Finally, the volume of the solution is adjusted with water to give the desired concentration of the ingredients.

Examples of isotonic agents are sodium chloride, mannitol and glycerol.

Examples of preservatives are phenol, m-cresol, methyl p-hydroxybenzoate and benzyl alcohol.

Examples of suitable buffers are sodium acetate and sodium phosphate.

Further to the above-mentioned components, solutions containing a GLP-1 derivative according to the present invention may also contain a surfactant in order to improve the solubility and/or the stability of the GLP-1 derivative.

A composition for nasal administration of certain peptides may, for example, be prepared as described in European Patent No. 272097 (to Novo Nordisk A/S) or in WO 93/18785.

According to one preferred embodiment of the present invention, the GLP-1 derivative is provided in the form of a composition suitable for administration by injection. Such a composition can either be an injectable solution ready for use or it can be an amount of a solid composition, *e.g.* a lyophilised product, which has to be dissolved in a solvent before it can be injected. The injectable solution preferably contains not less than about 2 mg/ml, preferably not less than about 5 mg/ml, more preferred not less than about 10 mg/ml of the GLP-1 derivative and, preferably, not more than about 100 mg/ml of the GLP-1 derivative.

The GLP-1 derivatives of this invention can be used in the treatment of various diseases. The particular GLP-1 derivative to be used and the optimal dose level for any patient will depend on the disease to be treated and on a variety of factors including the efficacy of the specific peptide derivative employed, the age, body weight, physical activity, and diet of the patient, on a possible combination with other drugs, and on the severity of the case. It is recommended that the dosage of the GLP-1 derivative of this invention be determined for each individual patient by those skilled in the art.

In particular, it is envisaged that the GLP-1 derivative will be useful for the preparation of a medicament with a protracted profile of action for the treatment of non-insulin dependent diabetes mellitus and/or for the treatment of obesity.

The present invention is further illustrated by the following examples which, however, are not to be construed as limiting the scope of protection. The features disclosed in the foregoing description and in the following examples may, both separately and in any combination thereof, be material for realising the invention in diverse forms thereof.

### EXAMPLES

The following acronyms for commercially available chemicals are used:

| | |
|---|---|
| DMF : | N,N-Dimethylformamide. |
| NMP : | N-Methyl-2-pyrrolidone. |
| EDPA : | N-Ethyl-N,N-diisopropylamine. |
| EGTA : | Ethylene glycol-bis(β-aminoethyl ether)-N,N,N',N'-tetraacetic acid. |
| GTP | Guanosine 5'-triphosphate. |
| TFA : | Trifluoroacetic acid. |
| THF : | Tetrahydrofuran |
| Myr-ONSu: | Tetradecanoic acid 2,5-dioxopyrrolidin-1-yl ester. |
| Pal-ONSu: | Hexadecanoic acid 2,5-dioxopyrrolidin-1-yl ester. |
| Ste-ONSu | Octadecanoic acid 2,5-dioxopyrrolidin-1-yl ester. |
| HOOC-(CH₂)₆-COONSu: | ω-Carboxyheptanoic acid 2,5-dioxopyrrolidin-1-yl ester. |
| HOOC-(CH₂)₁₀-COONSu: | ω-Carboxyundecanoic acid 2,5-dioxopyrrolidin-1-yl ester. |
| HOOC-(CH₂)₁₂-COONSu: | ω-Carboxytridecanoic acid 2,5-dioxopyrrolidin-1-yl ester. |
| HOOC-(CH₂)₁₄-COONSu: | ω-Carboxypentadecanoic acid 2,5-dioxopyrrolidin-1-yl ester. |
| HOOC-(CH₂)₁₆-COONSu: | ω-Carboxyheptadecanoic acid 2,5-dioxopyrrolidin-1-yl ester. |
| HOOC-(CH₂)₁₈-COONSu: | ω-Carboxynonadecanoic acid 2,5-dioxopyrrolidin-1-yl ester. |

### Abbreviations:

PDMS: Plasma Desorption Mass Spectrometry
MALDI-MS: Matrix Assisted Laser Desorption/Ionisation Mass Spectrometry
HPLC: High Performance Liquid Chromatography
amu: atomic mass units

### Analytical

### Plasma Desorption Mass Spectrometry

### Sample preparation:

The sample is dissolved in 0.1 % TFA/EtOH (1:1) at a concentration of 1 µg/µl. The sample solution (5-10 µl) is placed on a nitrocellulose target (Bio-ion AB, Uppsala, Sweden) and allowed to adsorb to the target surface for 2 minutes. The target is subsequently rinsed with 2x25 µl 0.1 % TFA and spin-dried. Finally, the nitrocellulose target is placed in a target carrousel and introduced into the mass spectrometer.

### MS analysis:

PDMS analysis was carried out using a Bio-ion 20 time-of flight instrument (Bio-ion Nordic AB, Uppsala, Sweden). An acceleration voltage of 15 kV was applied and molecular ions formed by bombardment of the nitrocellulose surface with 252-Cf fission fragments were accelerated towards a stop detector. The resulting time-of-flight spectrum was calibrated into a true mass spectrum using the H⁺ and NO⁺ ions at m/z 1 and 30, respectively. Mass spectra were generally accumulated for 1.0x10⁶ fission events corresponding to 15-20 minutes. Resulting assigned masses all correspond to isotopically averaged molecular masses. The accuracy of mass assignment is generally better than 0.1 %.

### MALDI-MS

MALDI-TOF MS analysis was carried out using a Voyager RP instrument (PerSeptive Biosystems Inc., Framingham, MA) equipped with delayed extraction and operated in linear mode. Alpha-cyano-4-hydroxy-cinnamic acid was used as matrix, and mass assignments were based on external calibration.

### Example 1

### Synthesis of Lys²⁶(N^{ε}-tetradecanoyl)-GLP-1(7-37).

The title compound was synthesised from GLP-1 (7-37). A mixture of GLP-1 (7-37) (25 mg, 7.45 µm), EDPA (26.7 mg, 208 µm), NMP (520 µl) and water (260 µl) was gently shaken for 5 min. at room temperature. To the resulting mixture was added a solution of Myr-ONSu (2.5 mg, 7.67 µm) in NMP (62.5 µl), the reaction mixture was gently shaken for 5 min. at room temperature and then allowed to stand for 20 min. An additional amount of Myr-ONSu (2.5 mg, 7.67 µm) in NMP (62.5 µl) was added and the resulting mixture gently shaken for 5 min. After a total reaction time of 40 min. the reaction was quenched by the addition of a solution of glycine (12.5 mg, 166 µmol) in 50% aqueous ethanol (12.5 ml). The title compound was isolated from the reaction mixture by HPLC using a cyanopropyl column (Zorbax 300SB-CN) and a standard acetonitrile/TFA system, yield: 1.3 mg (corresponding to 4.9% of the theoretical yield). The column was heated to 65°C and the acetonitrile gradient was 0-100% in 60 minutes. The isolated product was analysed by PDMS and the m/z value for the protonated molecular ion was found to be 3567.9±3. The resulting molecular weight is thus 3566.9±3 amu (theoretical value: 3565.9 amu). The position of acylation (Lys26) was verified by enzymatic cleavage of the title compound with Staphylococcus aureus V8 protease and subsequent mass determination of the peptide fragments by PDMS.

In addition to the title compound two other GLP-1 derivatives were isolated from the reaction mixture by using the same chromatographic column and a more shallow gradient (35-38% acetonitrile in 60 minutes), see Examples 2 and 3.

### Example 2

### Synthesis of Lys³⁴(N^{ε}-tetradecanoyl)-GLP-1 (7-37).

The title compound was isolated by HPLC from the reaction mixture described in Example 1. PDMS analysis yielded a protonated molecular ion at m/z 3567.7±3. The molecular weight is thus found to be 3566.7±3 amu (theoretical value: 3565.9 amu): The acylation site was determined on the basis of the fragmentation pattern.

### Example 3

### Synthesis of Lys^{26,34}-bis(N^{ε}-tetradecanoyl)-GLP-1(7-37).

The title compound was isolated by HPLC from the reaction mixture described in Example 1. PDMS analysis yielded a protonated molecular ion at m/z 3778.4±3. The molecular weight is thus found to be 3777.4±3 amu (theoretical value: 3776.1 amu).

### Example 4

### Synthesis of Lys²⁶(N^{ε}-tetradecanoyl)Arg³⁴-GLP-1 (7-37).

The title compound was synthesised from Arg³⁴-GLP-1(7-37). A mixture of Arg³⁴-GLP-1(7-37) (5 mg, 1.47 µm), EDPA (5.3 mg, 41.1 µm), NMP (105 µl) and water (50 µl) was gently shaken for 5 min. at room temperature. To the resulting mixture was added a solution of Myr-ONSu (0.71 mg, 2.2 µm) in NMP (17.8 µl), the reaction mixture was gently shaken for 5 min. at room temperature and then allowed to stand for 20 min. After a total reaction time of 30 min. the reaction was quenched by the addition of a solution of glycine (25 mg, 33.3 µm) in 50% aqueous ethanol (2.5 ml). The reaction mixture was purified by HPLC as described in Example 1. PDMS analysis yielded a protonated molecular ion at m/z 3594.9±3. The molecular weight is thus found to be 3593.9±3 amu (theoretical value: 3593.9 amu).

### Example 5

### Synthesis of Gly⁸Arg^{26,34}Lys³⁶(N^{ε}-tetradecanoyl)-GLP-1 (7-37).

The title compound was synthesised from Gly⁸Arg^{26,34}Lys³⁶-GLP-1(7-37) which was purchased from QCB. A mixture of Gly⁸Arg^{26,34}Lys³⁶-GLP-1(7-37) (1.3 mg, 0.39 µm), EDPA (1.3 mg, 10 µm), NMP (125 µl) and water (30 µl) was gently shaken for 5 min. at room temperature. To the resulting mixture was added a solution of Myr-ONSu (0.14 mg, 0.44 µm) in NMP (3.6 ml), the reaction mixture was gently shaken for 15 min. at room temperature. The reaction was quenched by the addition of a solution of glycine (0.1 mg, 1.33 µm) in 50% aqueous ethanol (10 µl). The reaction mixture was purified by HPLC, and the title compound (60 µg, 4%) was isolated.

### Example 6

### Synthesis of Arg^{26,34}Lys³⁶ (N^{ε}-tetradecanoyl)-GLP-1 (7-37)-OH.

A mixture of Arg^{26,34}Lys³⁶-GLP-1(7-37)-OH (5.0 mg, 1.477 µmol), EDPA (5.4 mg, 41.78 µmol), NMP (105 µl) and water (50 µl) was gently shaken for 5 min. at room temperature.. To the resulting mixture was added a solution of Myr-ONSu (0.721 mg, 2.215 µmol) in NMP (18 µl). The reaction mixture was gently shaken for 5 min. at room temperature, and then allowed to stand for an additional 45 min. at room temperature. The reaction was quenched by the addition of a solution of glycine (2.5 mg, 33.3 µmol) in 50% aqueous ethanol (250 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound ( 1.49 mg, 28 %) was isolated, and the product was analysed by PDMS. The m/z value for the protonated molecular ion was found to be 3595 ± 3. The resulting molecular weight is thus 3594 ± 3 amu (theoretical value 3594 amu).

### Example 7

### Synthesis of Lys^{26,34}bis(N^{ε}-(ω-carboxynonadecanoyl))-GLP-1(7-37)-OH.

A mixture of GLP-1 (7-37)-OH (70 mg, 20.85 µmol), EDPA (75.71 mg, 585.8 µmol), NMP (1.47 ml) and water (700 µL) was gently shaken for 10 min. at room temperature. To the resulting mixture was added a solution of HOOC-(CH₂)₁₈-COONSu (27.44 mg, 62.42 µmol) in NMP (686 µl), the reaction mixture was gently shaken for 5 min. at room temperature, and then allowed to stand for an additional 50 min. at room temperature. The reaction was quenched by the addition of a solution of glycine (34.43 mg, 458.7 µmol) in 50% aqueous ethanol (3.44 ml). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (8.6 mg, 10 %) was isolated, and the product was analysed by PDMS. The m/z value for the protonated molecular ion was found to be 4006 ± 3. The resulting molecular weight is thus 4005 ± 3 amu (theoretical value 4005 amu).

### Example 8

### Synthesis of Arg^{26,34}Lys³⁶(N^{ε}-(ω-carboxynonadecanoyl))-GLP-1 (7-36)-OH.

A mixture of Arg^{26,34}Lys³⁶-GLP-1(7-36)-OH (5.06 mg, 1.52 µmol), EDPA (5.5 mg, 42.58 µmol), NMP (106 µl) and water (100 µl) was gently shaken for 5 min. at room temperature. To the resulting mixture was added a solution of HOOC-(CH₂)₁₈-COONSu (1.33 mg, 3.04 µmol) in NMP (33.2 µl), the reaction mixture was gently shaken for 5 min. at room temperature, and then allowed to stand for an additional 2.5 h at room temperature. The reaction was quenched by the addition of a solution of glycine (2.50 mg, 33.34 µmol) in 50% aqueous ethanol (250 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.46 mg, 8 %) was isolated, and the product was analysed by PDMS. The m/z value for the protonated molecular ion was found to be 3652 ± 3. The resulting molecular weight is thus 3651 ± 3 amu (theoretical value 3651 amu).

### Example 9

### Synthesis of Arg^{26,34}Lys³⁸(N^{ε}-(ω-carboxynonadecanoyl))-GLP-1 (7-38)-OH.

A mixture of Arg^{26,34}Lys³⁸-GLP-1(7-38)-OH (5.556 mg, 1.57µmol), EDPA (5.68 mg, 43.96 µmol), NMP (116.6 µl) and water (50 µl) was gently shaken for 10 min. at room temperature. To the resulting mixture was added a solution HOOC-(CH₂)₁₈-COONSu (1.38 mg, 3.14 µmol) in NMP (34.5 µl), the reaction mixture was gently shaken for 5 min. at room temperature, and then allowed to stand for an additional 2.5 h at room temperature. The reaction was quenched by the addition of a solution of glycine (2.5 mg, 33.3 µmol) in 50% aqueous ethanol (250 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.7 mg, 12 %) was isolated, and the product was analysed by PDMS. The m/z value for the protonated molecular ion was found to be 3866 ± 3. The resulting molecular weight is thus 3865 ± 3 amu (theoretical value 3865 amu).

### Example 10

### Synthesis of Arg³⁴Lys²⁶ (N^{ε}-(ω-carboxynonadecanoyl))-GLP-1(7-37)-OH.

A mixture of Arg³⁴-GLP-1(7-37)-OH (5.04 mg, 1.489 µmol), EDPA (5.39 mg, 41.70 µmol), NMP (105 µl) and water (50 µl) was gently shaken for 10 min. at room temperature. To the resulting mixture was added a solution HOOC-(CH₂)₁₈-COONSu (1.31 mg, 2.97 µmol) in NMP (32.8 µl), the reaction mixture was gently shaken for 5 min. at room temperature, and then allowed to stand for an additional 30 min. at room temperature. The reaction was quenched by the addition of a solution of glycine (2.46 mg, 32.75 µmol) in 50% aqueous ethanol (246 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (1.2 mg, 22 %) was isolated, and the product was analysed by PDMS. The m/z value for the protonated molecular ion was found to be 3709 ± 3. The resulting molecular weight is thus 3708 ± 3 amu (theoretical value 3708 amu).

### Example 11

### Synthesis of Arg³⁴Lys²⁶ (N^{ε}-(ω-carboxyheptadecanoyl))-GLP-1 (7-37)-OH.

A mixture of Arg-14-GLP-1(7-37)-OH (5.8 mg, 1.714 µmol), EDPA (6.20 mg, 47.99 µmol), NMP (121.8 µl) and water (58 µl) was gently shaken for 10 min. at room temperature. To the resulting mixture was added a solution HOOC-(CH₂)₁₆-COONSu (2.11 mg, 5.142 µmol) in NMP (52.8 µl), the reaction mixture was gently shaken for 5 min. at room temperature, and then allowed to stand for an additional 2 h at room temperature. The reaction was quenched by the addition of a solution of glycine (2.83 mg, 37.70 µmol) in 50% aqueous ethanol (283 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.81 mg, 13 %) was isolated, and the product was analysed by PDMS. The m/z value for the protonated molecular ion was found to be 3681 ± 3. The resulting molecular weight is thus 3680 ± 3 amu (theoretical value 3680 amu).

### Example 12

### Synthesis of Arg^{26,34}Lys³⁶ (N^{ε}-(ω-carboxyheptadecanoyl))-GLP-1 (7-37)-OH.

A mixture of Arg^{26,34}Lys³⁶-GLP-1(7-37)-OH (3.51 mg, 1.036 µmol), EDPA (3.75 mg, 29.03 µmol), NMP (73.8 µl) and water (35 µl) was gently shaken for 10 min. at room temperature. To the resulting mixture was added a solution HOOC-(CH₂)₁₆-COONSu (1.27 mg, 3.10 µmol) in NMP (31.8 µl), the reaction mixture was gently shaken for 5 min. at room temperature, and then allowed to stand for an additional 2 h and 10 min. at room temperature. The reaction was quenched by the addition of a solution of glycine (1.71 mg, 22.79 µmol) in 50% aqueous ethanol (171 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.8 mg, 21 %) was isolated, and the product was analysed by PDMS. The m/z value for the protonated molecular ion was found to be 3682 ± 3. The resulting molecular weight is thus 3681 ± 3 amu (theoretical value 3681 amu).

### Example 13

### Synthesis of Arg^{26,34}Lys³⁸(N^{ε}-(ω-carboxyheptadecanoyl))-GLP-1 (7-38)-OH.

A mixture of Arg^{26,34}Lys³⁸-GLP-1(7-38)-OH (5.168 mg, 1.459 µmol), EDPA (5.28 mg, 40.85 µmol), NMP (108.6 µl) and water (51.8 µl) was gently shaken for 10 min. at room temperature. To the resulting mixture was added a solution HOOC-(CH₂)₁₆-COONSu (1.80 mg, 4.37 µmol) in NMP (45 µl), the reaction mixture was gently shaken for 10 min. at room temperature, and then allowed to stand for an additional 2 h and 15 min. at room temperature. The reaction was quenched by the addition of a solution of glycine (2.41 mg, 32.09 µmol) in 50% aqueous ethanol (241 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.8 mg, 14 %) was isolated, and the product was analysed by PDMS. The m/z value for the protonated molecular ion was found to be 3838 ± 3. The resulting molecular weight is thus 3837 ± 3 amu (theoretical value 3837 amu).

### Example 14

### Synthesis of Arg^{26,34}Lys³⁶ (N^{ε}-(ω-carboxyheptadecanoyl))-GLP-1 (7-36)-OH.

A mixture of Arg^{26,34}Lys³⁸-GLP-1(7-36)-OH (24.44 mg, 7.34 µmol), EDPA (26.56 mg, 205.52 µmol), NMP (513 µl) and water (244.4 µl) was gently shaken for 5 min. at room temperature. To the resulting mixture was added a solution HOOC-(CH₂)₁₆-COONSu (9.06 mg, 22.02 µmol) in NMP (1.21 ml), the reaction mixture was gently shaken for 5 min. at room temperature, and then allowed to stand for an additional 30 min. at room temperature. The reaction was quenched by the addition of a solution of glycine (12.12 mg, 161.48 µmol) in 50% aqueous ethanol (1.21 ml). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (7.5 mg, 28 %) was isolated, and the product was analysed by PDMS. The m/z value for the protonated molecular ion was found to be 3625 ± 3. The resulting molecular weight is thus 3624 ± 3 amu (theoretical value 3624 amu).

### Example 15

### Synthesis of Arg^{26,34}Lys³⁶ (N^{ε}-(ω-carboxyundecanoyl))-GLP-1(7-37)-OH.

A mixture of Arg^{26,34}Lys³⁶-GLP-1(7-37)-OH (4.2 mg, 1.24 µmol), EDPA (4.49 mg, 34.72 µmol), NMP (88.2 µl) and water (42 µl) was gently shaken for 10 min. at room temperature. To the resulting mixture was added a solution HOOC-(CH₂)₁₀-COONSu (1.21 mg, 3.72 µmol) in NMP (30.25 µl), the reaction mixture was gently shaken for 5 min. at room temperature, and then allowed to stand for an additional 40 min. at room temperature. The reaction was quenched by the addition of a solution of glycine (2.04 mg, 27.28 µmol) in 50% aqueous ethanol (204 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.8 mg, 18 %) was isolated, and the product was analysed by PDMS. The m/z value for the protonated molecular ion was found to be 3598 ± 3. The resulting molecular weight is thus 3597 ± 3 amu (theoretical value 3597 amu).

### Example 16

### Synthesis of Arg^{26,34}Lys³⁸(N^{ε}-(ω-carboxyundecanoyl))-GLP-1(7-38)-OH.

A mixture of Arg^{26,34}Lys³⁸-GLP-1(7-38)-OH (5.168 mg, 1.46 µmol), EDPA (5.28 mg, 40.88 µmol), NMP (108.6 µl) and water (51.7 µl) was gently shaken for 10 min. at room temperature. To the resulting mixture was added a solution HOOC-(CH₂)₁₀-COONSu (1.43 mg, 4.38 µmol) in NMP (35.8 µl), the reaction mixture was gently shaken for 5 min. at room temperature, and then allowed to stand for an additional 50 min. at room temperature. The reaction was quenched by the addition of a solution of glycine (2.41 mg, 32.12 µmol) in 50% aqueous ethanol (241 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.85 mg, 16 %) was isolated, and the product was analysed by PDMS. The m/z value for the protonated molecular ion was found to be 3753 ± 3. The resulting molecular weight is thus 3752 ± 3 amu (theoretical value 3752 amu).

### Example 17

### Synthesis of Lys^{26,34}bis(N^{ε}-(ω-carboxyundecanoyl))-GLP-1(7-37)-OH.

A mixture of GLP-1(7-37)-OH (10.0 mg, 2.98 µmol), EDPA (10.8 mg, 83.43 µmol), NMP (210 µl) and water (100 µl) was gently shaken for 10 min. at room temperature. To the resulting mixture was added a solution HOOC-(CH₂)₁₀-COONSu (2.92 mg, 8.94 µmol) in NMP (73 µl), the reaction mixture was gently shaken for 5 min. at room temperature, and then allowed to stand for an additional 50 min. at room temperature. The reaction was quenched by the addition of a solution of glycine (4.92 mg, 65.56 µmol) in 50% aqueous ethanol (492 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (1.0 mg, 9 %) was isolated, and the product was analysed by PDMS. The m/z value for the protonated molecular ion was found to be 3781 ± 3. The resulting molecular weight is thus 3780 ± 3 amu (theoretical value 3780amu).

### Example 18

### Synthesis of Arg^{26,34}Lys³⁶ (N^{ε}-(ω-carboxyundecanoyl))-GLP-1(7-36)-OH.

A mixture of Arg^{26,34}Lys³⁶-GLP-1(7-36)-OH (15.04 mg, 4.52 µmol), EDPA (16.35 mg, 126.56 µmol), NMP (315.8 µl) and water (150.4 µl) was gently shaken for 10 min. at room temperature. To the resulting mixture was added a solution HOOC-(CH₂)₁₀-COONSu (4.44 mg, 13.56 µmol) in NMP (111 µl), the reaction mixture was gently shaken for 5 min. at room temperature, and then allowed to stand for an additional 40 min. at room temperature. The reaction was quenched by the addition of a solution of glycine (7.5 mg, 99.44 µmol) in 50% aqueous ethanol (750 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (3.45 mg, 22 %) was isolated, and the product was analysed by PDMS. The m/z value for the protonated molecular ion was found to be 3540 ± 3. The resulting molecular weight is thus 3539 ± 3 amu (theoretical value 3539 amu).

### Example 19

### Synthesis of Arg³⁴Lys²⁶ (N^{ε}-(ω-carboxyundecanoyl))-GLP-1 (7-37)-OH.

A mixture of Arg³⁴-GLP-1(7-37)-OH (5.87 mg, 1.73 µmol), EDPA (6.27 mg, 48.57 µmol), NMP (123.3 µl) and water (58.7 µl) was gently shaken for 10 min. at room temperature. To the resulting mixture was added a solution HOOC-(CH₂)₁₀-COONSu (1.70 mg, 5.20 µmol) in NMP (42.5 µl), the reaction mixture was gently shaken for 5 min. at room temperature, and then allowed to stand for an additional 40 min. at room temperature. The reaction was quenched by the addition of a solution of glycine (2.86 mg, 286 µmol) in 50% aqueous ethanol (286 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (1.27 mg, 20 %) was isolated, and the product was analysed by PDMS. The m/z value for the protonated molecular ion was found to be 3597 ± 3. The resulting molecular weight is thus 3596 ± 3 amu (theoretical value 3596 amu).

### Example 20

### Synthesis of Arg³⁴Lys²⁶ (N^{ε}-(ω-carboxyheptanoyl))-GLP-1(7-37)-OH.

A mixture of Arg³⁴-GLP-1(7-37)-OH (4.472 mg, 1.32 µmol), EDPA (4.78 mg, 36.96 µmol), NMP (94 µl) and water (44.8 µl) was gently shaken for 5 min. at room temperature. To the resulting mixture was added a solution HOOC-(CH₂)₆-COONSu (1.07 mg, 3.96 µmol) in NMP (26.8 µl), the reaction mixture was gently shaken for 5 min. at room temperature, and then allowed to stand for an additional 1 h and 50 min. at room temperature. The reaction was quenched by the addition of a solution of glycine (2.18 mg, 29.04 µmol) in 50% aqueous ethanol (218 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.5 mg, 11 %) was isolated, and the product was analysed by PDMS. The m/z value for the protonated molecular ion was found to be 3540 ± 3. The resulting molecular weight is thus 3539 ± 3 amu (theoretical value 3539 amu).

### Example 21

### Synthesis of Arg^{26,34}LyS³⁸(N^{ε}-(ω-carboxyheptanoyl))-GLP-1(7-38)-OH.

A mixture of Arg^{26,34}Lys³⁸-GLP-1(7-38)-OH (5.168 mg, 1.459 µmol), EDPA (5.28 mg, 40.85 µmol), NMP (108.6 µl) and water (51.6 µl) was gently shaken for 10 min. at room temperature. To the resulting mixture was added a solution HOOC-(CH₂)₆-COONSu (1.18 mg, 4.37 µmol) in NMP (29.5 µl), the reaction mixture was gently shaken for 5 min. at room temperature, and then allowed to stand for an additional 1 h and 50 min. at room temperature. The reaction was quenched by the addition of a solution of glycine (2.40 mg, 32.09 µmol) in 50% aqueous ethanol (240 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.5 mg, 9 %) was isolated, and the product was analysed by PDMS. The m/z value for the protonated molecular ion was found to be 3697 ± 3. The resulting molecular weight is thus 3695 ± 3 amu (theoretical value 3695 amu).

### Example 22

### Synthesis of Arg^{26,34}Lys³⁶ (N^{ε}-(ω-carboxyheptanoyl))-GLP-1 (7-37)-OH.

A mixture of Arg^{26,34}Lys³⁶-GLP-1(7-37)-OH (5.00 mg, 1.47 µmol), EDPA (5.32 mg, 41.16 µmol), NMP (105 µl) and water (50 µl) was gently shaken for 5 min. at room temperature. To the resulting mixture was added a solution HOOC-(CH₂)₆-COONSu (1.19 mg, 4.41 µmol) in NMP (29.8 µl), the reaction mixture was gently shaken for 5 min. at room temperature, and then allowed to stand for an additional 2 h at room temperature. The reaction was quenched by the addition of a solution of glycine (2.42 mg, 32.34 µmol) in 50% aqueous ethanol (242 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.78 mg, 15 %) was isolated, and the product was analysed by PDMS. The m/z value for the protonated molecular ion was found to be 3542 ± 3. The resulting molecular weight is thus 3541 ± 3 amu (theoretical value 3541 amu).

### Example 23

### Synthesis of Arg^{28,34}Lys³⁶ (N^{ε}-(ω-carboxyheptanoyl))-GLP-1 (7-36)-OH .

A mixture of Arg^{26,34}Lys³⁶-GLP-1(7-36)-OH (5.00 mg, 1.50 µmol), EDPA (5.44 mg, 42.08 µmol), NMP (210 µl) and water (50 µl) was gently shaken for 5 min. at room temperature. To the resulting mixture was added a solution HOOC-(CH₂)₆-COONSu (1.22 mg, 4.5 µmol) in NMP (30.5 µl), the reaction mixture was gently shaken for 5 min. at room temperature, and then allowed to stand for an additional 2 h at room temperature. The reaction was quenched by the addition of a solution of glycine (2.47 mg, 33.0 µmol) in 50% aqueous ethanol (247 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.71 mg, 14 %) was isolated, and the product was analysed by PDMS. The m/z value for the protonated molecular ion was found to be 3484 ± 3. The resulting molecular weight is thus 3483 ± 3 amu (theoretical value 3483 amu).

### Example 24

### Synthesis of Lys^{26,34}bis (N^{ε}-(ω-carboxyheptanoyl))-GLP-1(7-37)-OH.

A mixture of GLP-1(7-37)-OH (10 mg, 2.5 µmol), EDPA (10.8 mg, 83.56 µmol), NMP (210 µl) and water (100 µl) was gently shaken for 10 min. at room temperature. To the resulting mixture was added a solution HOOC-(CH₂)₆-COONSu (2.42 mg, 8.92 µmol) in NMP (60.5 µl), the reaction mixture was gently shaken for 5 min. at room temperature, and then allowed to stand for an additional 2 h and 35 min. at room temperature. The reaction was quenched by the addition of a solution of glycine (4.92 mg, 65.54 µmol) in 50% aqueous ethanol (492 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (2.16 mg, 24 %) was isolated, and the product was analysed by PDMS. The m/z value for the protonated molecular ion was found to be 3669 ± 3. The resulting molecular weight is thus 3668 ± 3 amu (theoretical value 3668 amu).

### Example 25

### Synthesis of Arg³⁴Lys²⁶ (N^{ε}-(ω-carboxypentadecanoyl))-GLP-1(7-37)-OH.

A mixture of Arg³⁴-GLP-1(7-37)-OH (4.472 mg, 1.321 µmol), EDPA (4.78 mg, 36.99 µmol), NMP (93.9 µl) and water (44.7 µl) was gently shaken for 10 min. at room temperature. To the resulting mixture was added a solution HOOC-(CH₂)₁₄-COONSu (1.519 mg, 3.963 µmol) in NMP (38 µl), the reaction mixture was gently shaken for 5 min. at room temperature, and then allowed to stand for an additional 1 h at room temperature. The reaction was quenched by the addition of a solution of glycine (2.18 mg, 29.06 µmol) in 50% aqueous ethanol (218 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.58 mg, 12 %) was isolated, and the product was analysed by PDMS. The m/z value for the protonated molecular ion was found to be 3654 ± 3. The resulting molecular weight is thus 3653 ± 3 amu (theoretical value 3653 amu).

### Example 26

### Synthesis of Arg^{26.34}Lys³⁶ (N^{ε}-(ω-carboxyheptanoyl))-GLP-1(7-36)-OH.

A mixture of Arg^{26.34}Lys³⁶-GLP-1(7-36)-OH (5.00 mg, 1.50 µmol), EDPA (5.44 mg, 42.08 µmol), NMP (210 µl) and water (50 µl) was gently shaken for 5 min. at room temperature. To the resulting mixture was added a solution HOOC-(CH₂)₁₄-COONSu (1.72 mg, 4.5 µmol) in NMP (43 µl), the reaction mixture was gently shaken for 5 min. at room temperature, and then allowed to stand for an additional 1 h at room temperature. The reaction was quenched by the addition of a solution of glycine (2.48 mg, 33 µmol) in 50% aqueous ethanol (248 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.58 mg, 11 %) was isolated, and the product was analysed by PDMS. The m/z value for the protonated molecular ion was found to be 3596 ± 3. The resulting molecular weight is thus 3595 ± 3 amu (theoretical value 3595 amu).

### Example 27

### Synthesis of lithocholic acid 2,5-dioxo-pyrrolidin-1-yl ester.

To a mixture of lithocholic acid (5.44 g, 14.34 mmol), N-hydroxysuccinimide (1.78 g, 15.0 mmol), anhydrous THF (120 ml) and anhydrous acetonitrile (30 ml), kept at to 10°C, was added a solution of N,N'-dicyclohexylcarbodiimide (3.44 g, 16.67 mmol) in anhydrous THF. The reaction mixture was stirred at ambient temperature for 16 h, filtered and concentrated *in vacuo.* The residue was dissolved in dichloromethane (450 ml), washed with a 10% aqueous Na₂CO₃ solution (2x150 ml) and water (2x150 ml), and dried (MgSO₄). Filtered and the filtrate concentrated *in vacuo* to give a crystalline residue. The residue was recrystallised from a mixture of dichloromethane (30 ml) and n-heptane (30 ml to give the title compound (3.46 g. 51 %) as a crystalline solid.

### Example 28

### Synthesis of Arg³⁴Lys²⁶(N^{ε}-lithocholyl)-GLP-1(7-37)-OH.

A mixture of Arg³⁴-GLP-1(7-37)-OH (4.472 mg, 1.32 µmol), EDPA (4.78 mg, 36.96 µmol), NMP (94 µl) and water (44.8 µl) was gently shaken for 10 min. at room temperature. To the resulting mixture was added a solution of lithocholic acid 2,5-dioxo-pyrrolidin-1-yl ester (1.87 mg, 3.96 µmol) in NMP (46.8 µl), the reaction mixture was gently shaken for 5 min. at room temperature, and then allowed to stand for an additional 1 h at room temperature. The reaction was quenched by the addition of a solution of glycine (2.18 mg, 29.04 µmol) in 50% aqueous ethanol (218 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (1.25 mg, 25 %) was isolated, and the product was analysed by PDMS. The m/z value for the protonated molecular ion was found to be 3744 +- 3. The resulting molecular weight is thus 3743 +- 3 amu (theoretical value 3743 amu).

### Example 29

### Synthesis of N^{α}-tetradecanoyl-Glu(ONSu)-OBu^{t}.

To a suspension of H-Glu(OH)-OBu^{t}(2.5 g, 12.3 mmol), DMF (283 ml) and EDPA (1.58 g, 12.3 mmol) was added drop by drop a solution of Myr-ONSu (4.0 g, 12.3 mmol) in DMF (59 ml). The reaction mixture was stirred for 16 h at room temperature and then concentrated *in vacuo* to a total volume of 20 ml. The residue was partitioned between 5% aqueous citric acid (250 ml) and ethyl acetate (150 ml), and the phases were separated. The organic phase was concentrated *in vacuo* and the residue dissolved in DMF (40 ml). The resulting solution was added drop by drop to a 10% aqueous solution of citric acid (300 ml) kept at 0 °C. The precipitated compound was collected and washed with iced water and dried in a vacuum drying oven. The dried compound was dissolved in DMF (23 ml) and HONSu (1.5 g, 13 mmol) was added. To the resulting mixture was added a solution of N,N'-dicyclohexylcarbodiimide (2.44 g, 11.9 mmol) in dichloromethane (47 ml). The reaction mixture was stirred for 16 h at room temperature, and the precipitated compound was filtered off. The precipitate was recrystallised from n-heptane/2-propanol to give the title compound (3.03 g, 50%).

### Example 30

### Synthesis of Glu^{22,23,30}Arg^{26,34}Lys³⁸(N^{ε}-(γ-glutamyl(N^{α}-tetradecanoyl)))-GLP-1(7-38)-OH.

A mixture of Glu^{22,23,30}Arg^{26,34}Lys³⁸-GLP-1(7-38)-OH (1.0 mg, 0.272 µmol), EDPA (0.98 mg, 7.62 µmol), NMP (70 µl) and water (70 µl) was gently shaken for 5 min. at room temperature. To the resulting mixture was added a solution of N^{α}-tetradecanoyl-Glu(ONSu)-OBu^{t}, prepared as described in Example 29, (0.41 mg, 0.816 µmol) in NMP (10.4 µl), the reaction mixture was gently shaken for 5 min. at room temperature, and then allowed to stand for an additional 45 min. at room temperature. The reaction was quenched by the addition of a solution of glycine (0.448 mg, 5.98 µmol) in 50% aqueous ethanol (45 µl). A 0.5 % aqueous solution of ammonium acetate (0.9 ml) was added, and the resulting mixture was immobilised on a Varian 500 mg C8 Mega Bond Elut® cartridge, the immobilised compound washed with 5% aqueous acetonitrile (10 ml), and finally liberated from the cartridge by elution with TFA (10 ml). The eluate was concentrated *in vacuo,* and the reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.35 mg, 32 %) was isolated, and the product was analysed by PDMS. The m/z value for the protonated molecular ion was found to be 4012 ± 3. The resulting molecular weight is thus 4011 ± 3 amu (theoretical value 4011 amu).

### Example 31

### Synthesis of Glu^{23,26}Arg³⁴Lys³⁸(N^{ε}-(γ-glutamyl(N^{α}-tetradecanoyl)))-GLP-1(7-38)-OH.

A mixture of Glu^{23,26}Arg³⁴Lys³⁸-GLP-1(7-38)-OH (6.07 mg, 1.727 µmol), EDPA (6.25 mg, 48.36 µmol), NMP (425 µl) and water (425 µl) was gently shaken for 5 min. at room temperature. To the resulting mixture was added a solution of N^{α}-tetradecanoyl-Glu(ONSu)-OBu^{t}, prepared as described in example 29, (2.65 mg, 5.18 µmol) in NMP (66.3 µl), the reaction mixture was gently shaken for 5 min. at room temperature, and then allowed to stand for an additional 45 min. at room temperature. The reaction was quenched by the addition of a solution of glycine (2.85 mg, 38.0 µmol) in 50% aqueous ethanol (285 µl). A 0.5 % aqueous solution of ammonium acetate (5.4 ml) was added, and the resulting mixture was immobilised on a Varian 500 mg C8 Mega Bond Elut® cartridge, the immobilised compound washed with 5% aqueous acetonitrile (10 ml), and finally liberated from the cartridge by elution with TFA (10 ml). The eluate was concentrated *in vacuo*, and the reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.78 mg, 12 %) was isolated, and the product was analysed by PDMS. The m/z value for the protonated molecular ion was found to be 3854 ± 3. The resulting molecular weight is thus 3853 ± 3 amu (theoretical value 3853 amu).

### Example 32

### Synthesis of Lys^{26,34}-bis(N^{ε}-(ω-carboxytridecanoyl))-GLP-1 (7-37)-OH.

A mixture of GLP-1(7-37)-OH (30 mg, 8.9 µmol), EDPA (32.3 mg, 250 µmol), NMP (2.1 ml) and water (2.1 ml) was gently shaken for 5 min. at room temperature. To the resulting mixture was added a solution HOOC-(CH₂)₁₂-COONSu (12.7 mg, 35.8 µmol) in NMP (318 µl), the reaction mixture was gently shaken for 1 h and 40 min. at room temperature. The reaction was quenched by the addition of a solution of glycine (3.4 mg, 44.7 µmol) in 50% aqueous ethanol (335 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (10 mg, 29 %) was isolated, and the product was analysed by PDMS. The m/z value for the protonated molecular ion was found to be 3840 ± 3. The resulting molecular weight is thus 3839 ± 3 amu (theoretical value 3839 amu).

### Example 33

### Synthesis of Lys^{28,34}-bis(N^{ε}-(γ-glutamyl(N^{α}-tetradecanoyl)))-GLP-1 (7-37)-OH. (NNC 90-1167).

A mixture of GLP-1 (7-37)-OH (300 mg, 79.8 µmol), EDPA (288.9 mg, 2.24 mmol), NMP (21 ml) and water (21 ml) was gently shaken for 5 min. at room temperature. To the resulting mixture was added a solution of N^{α}-tetradecanoyl-Glu(ONSu)-OBu^{t}, prepared as described in Example 29, (163 mg, 319.3 µmol) in NMP (4.08 ml), the reaction mixture was gently shaken for 5 min. at room temperature, and then allowed to stand for an additional 1 h at room temperature. The reaction was quenched by the addition of a solution of glycine (131.8 mg, 1.76 mmol) in 50% aqueous ethanol (13.2 ml). A 0.5 % aqueous solution of ammonium-acetate (250 ml) was added, and the resulting mixture was divided into four equal portions. Each portion was eluted onto a Varian 500 mg C8 Mega Bond Elut^{®}cartridge, the immobilised compound washed with 0.1% aqueous TFA (3.5 ml), and finally liberated from the cartridge by elution with 70% aqueous acetonitrile (4 ml). The combined eluates were diluted with 0.1% aqueous TFA (300 ml). The precipitated compound was collected by centrifugation, washed with 0.1 % aqueous TFA (50 ml), and finally isolated by centrifugation. To the precipitate was added TFA (60 ml), and the resulting reaction mixture was stirred for 1 h and 30 min. at room temperature. Excess TFA was removed *in vacuo,* and the residue was poured into water (50 ml). The precipitated compound was purified by column chromatography using a cyanopropyl column (Zorbax 300SB-CN) and a standard acetonitrile/TFA system. The column was heated to 65°C and the acetonitrile gradient was 0-100% in 60 minutes. The title compound (27.3 mg, 8 %) was isolated, and the product was analysed by PDMS. The m/z value for the protonated molecular ion was found to be 4036 ± 3. The resulting molecular weight is thus 4035 ± 3 amu (theoretical value 4035 amu).

### Example 34

### Synthesis of Arg^{26,34}Lys³⁸(N^{ε}-(ω-carboxypentadecanoyl))-GLP-1 (7-38)-OH.

A mixture of Arg^{26,34}Lys³⁸-GLP-1(7-38)-OH (30 mg, 8.9 µmol), EDPA (32.3 mg, 250 µmol), NMP (2.1 ml) and water (2.1 ml) was gently shaken for 5 min. at room temperature. To the resulting mixture was added a solution HOOC-(CH₂)₁₄ COONSu (13.7 mg, 35.8 µmol) in NMP (343 µl), the reaction mixture was gently shaken for 1 h at room temperature. The reaction was quenched by the addition of a solution of glycine (3.4 mg, 44.7 µmol) in 50% aqueous ethanol (335 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (4.8 mg, 14 %) was isolated, and the product was analysed by PDMS. The m/z value for the protonated molecular ion was found to be 3894 ± 3. The resulting molecular weight is thus 3893 ± 3 amu (theoretical value 3893 amu).

### Example 35

### Synthesis of N^{α}-hexadecanoyl-Glu(ONSu)-OBu^{t}.

To a suspension of H-Glu(OH)-OBu^{t} (4.2 g, 20.6 mmol), DMF (500 ml) and EDPA (2.65 g, 20.6 mmol) was added drop by drop a solution of Pal-ONSu (7.3 g, 20.6 mmol) in DMF (100 ml). The reaction mixture was stirred for 64 h at room temperature and then concentrated *in vacuo* to a total volume of 20 ml. The residue was partitioned between 10% aqueous citric acid (300 ml) and ethyl acetate (250 ml), and the phases were separated. The organic phase was concentrated *in vacuo* and the residue dissolved in DMF (50 ml). The resulting solution was added drop by drop to a 10% aqueous solution of citric acid (500 ml) kept at 0 °C. The precipitated compound was collected and washed with iced water and dried in a vacuum drying oven. The dried compound was dissolved in DMF (45 ml) and HONSu (2.15 g, 18.7 mmol) was added. To the resulting mixture was added a solution of N,N'-dicyclohexylcarbodiimide (3.5 g, 17 mmol) in dichloromethane (67 ml). The reaction mixture was stirred for 16 h at room temperature, and the precipitated compound was filtered off. The precipitate was recrystallised from n-heptane/2-propanol to give the title compound (6.6 g, 72%).

### Example 36

### Synthesis of Lys^{26,34}-bis(N^{ε}-(γ-glutamyl(N^{α}-hexadecanoyl)))-GLP-1(7-37)-OH.

A mixture of GLP-1 (7-37)-OH (10 mg, 2.9 µmol), EDPA (10.8 mg, 83.4 µmol), NMP (0.7 ml) and water (0.7 ml) was gently shaken for 5 min. at room temperature. To the resulting mixture was added a solution of N^{α}-hexadecanoyl-Glu(ONSu)-OBu^{t}, prepared as described in Example 33, (163 mg, 319.3 µmol) in NMP (4.08 ml), the reaction mixture was gently shaken 1 h and 20 min. at room temperature. The reaction was quenched by the addition of a solution of glycine (4.9 mg, 65.6 µmol) in 50% aqueous ethanol (492 µl). A 0.5 % aqueous solution of ammonium-acetate (9 ml) was added, and the resulting mixture eluted onto a Varian 1 g C8 Mega Bond Elut^{®} cartridge, the immobilised compound washed with 5% aqueous acetonitrile (10 ml), and finally liberated from the cartridge by elution with TFA (10 ml). The eluate was concentrated *in vacuo,* and the residue purified by column chromatography using a cyanopropyl column (Zorbax 300SB-CN) and a standard acetonitrile/TFA system. The column was heated to 65°C and the acetonitrile gradient was 0-100% in 60 minutes. The title compound (2.4 mg, 20 %) was isolated, and the product was analysed by PDMS. The m/z value for the protonated molecular ion was found to be 4092 ± 3. The resulting molecular weight is thus 4091 ± 3 amu (theoretical value 4091 amu).

### Example 37

### Synthesis of Arg³⁴Lys²⁶(N^{ε}-(γ-glutamyl(N^{α}-hexadecanoyl)))-GLP-1(7-37)-OH.

A mixture of Arg³⁴-GLP-1(7-37)-OH (3.7 mg, 1.1 µmol), EDPA (4.0 mg, 30.8 µmol), acetonitrile (260 µl) and water (260 µl) was gently shaken for 5 min. at room temperature. To the resulting mixture was added a solution of N^{α}-hexadecanoyl-Glu(ONSu)-OBu^{t} prepared as described in Example 35, (1.8 mg, 3.3 µmol) in acetonitrile (44.2 µl), and the reaction mixture was gently shaken for 1 h and 20 min. at room temperature. The reaction was quenched by the addition of a solution of glycine (1.8 mg, 24.2 µmol) in 50% aqueous ethanol (181 µl). A 0.5 % aqueous solution of ammonium-acetate (12 ml) and NMP (300 µl) were added, and the resulting mixture eluted onto a Varian 1g C8 Mega Bond Elut^{®} cartridge, the immobilised compound washed with 5% aqueous acetonitrile (10 ml), and finally liberated from the cartridge by elution with TFA (6 ml). The eluate was allowed to stand for 2 h at room temperature and then concentrated *in vacuo.* The residue was purified by column chromatography using a cyanopropyl column (Zorbax 300SB-CN) and a standard acetonitrile/TFA system. The column was heated to 65°C and the acetonitrile gradient was 0-100% in 60 minutes. The title compound (0.23 mg, 6 %) was isolated, and the product was analysed by PDMS. The m/z value for the protonated molecular ion was found to be 3752 ± 3. The resulting molecular weight is thus 3751 ± 3 amu (theoretical value 3751 amu).

### Example 38

### Synthesis of Arg^{26,34}Lys³⁸(N^{ε}-(γ-glutamyl(N^{α}-tetradecanoyl)))-GLP-1 (7-38)-OH.

A mixture of Arg^{26,34}Lys³⁸-GLP-1(7-38)-OH (14 mg, 4.0 µmol), EDPA (14.3 mg, 110.6 µmol), NMP (980 µl) and water (980 µl) was gently shaken for 5 min. at room temperature. To the resulting mixture was added a solution of N^{α}-tetradecanoyl-Glu(ONSu)-OBu^{t}, prepared as described in Example 29, (12.1 mg, 23.7 µmol) in NMP (303 µl), and the reaction mixture was gently shaken for 2 h at room temperature. The reaction was quenched by the addition of a solution of glycine (6.5 mg, 86.9 mmol) in 50% aqueous ethanol (652 µl). A 0.5 % aqueous solution of ammonium-acetate (50 ml) was added, and the resulting mixture eluted onto a Varian 1g C8 Mega Bond Elut^{®} cartridge, the immobilised compound washed with 5% aqueous acetonitrile (15 ml), and finally liberated from the cartridge by elution with TFA (6 ml). The eluate was allowed to stand for 1 h and 45 min. at room temperature and then concentrated *in vacuo.* The residue was purified by column chromatography using a cyanopropyl column (Zorbax 300SB-CN) and a standard acetonitrile/TFA system. The column was heated to 65°C and the acetonitrile gradient was 0-100% in 60 minutes. The title compound (3.9 mg, 26 %) was isolated, and the product was analysed by PDMS. The m/z value for the protonated molecular ion was found to be 3881 ± 3. The resulting molecular weight is thus 3880 ± 3 amu (theoretical value 3880 amu).

### Example 39

### Synthesis of Arg^{26,34}Lys³⁸(N^{ε}-(ω-carboxypentadecanoyl))-GLP-1(7-38)-OH.

A mixture of Arg^{26,34}Lys³⁸-GLP-1(7-38)-OH (14 mg, 4.0 µmol), EDPA (14.3 mg, 111 µmol), NMP (980 µl) and water (980 µl) was gently shaken for 5 min. at room temperature. To the resulting mixture was added a solution of HOOC-(CH₂)₁₄-COONSu (4.5 mg, 11.9 µmol) in NMP (114 µl), the reaction mixture was gently shaken for 1 h and 45 min. at room temperature. An additional solution of HOOC-(CH₂)₁₄-COONSu (4.0 mg, 10.4 µmol) in NMP (100 µl) was added, and the resulting mixture was gently shaken for an additional 1 h and 30 min. at room temperature. The reaction was quenched by the addition of a solution of glycine (1.5 mg, 19.8 µmol) in 50% aqueous ethanol (148 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (3.9 mg, 26 %) was isolated, and the product was analysed by PDMS. The m/z value for the protonated molecular ion was found to be 3809 ± 3. The resulting molecular weight is thus 3808 ± 3 amu (theoretical value 3808 amu).

### Example 40

### Synthesis of Arg ^{26.34} Lys³⁸(N^{ε}-(γ-glutamyl(N^{α}-hexadecanoyl)))-GLP-1 (7-38)-OH.

A mixture of Arg^{26,34}Lys³⁶-GLP-1(7-38)-OH (14 mg, 4.0 µmol), EDPA (14.3 mg, 110.6 µmol), NMP (980 µl) and water (980 µl) was gently shaken for 5 min. at room temperature. To the resulting mixture was added a solution of N^{α}-hexadecanoyl-Glu(ONSu)-OBu^{t} prepared as described in Example 35, (6.4 mg, 11.9 µmol) in NMP (160 µl), and the reaction mixture was gently shaken for 1 h and 20 min. at room temperature. The reaction was quenched by the addition of a solution of glycine (6.5 mg, 87 mmol) in 50% aqueous ethanol (653 µl). A 0.5 % aqueous solution of ammonium-acetate (50 ml) was added, and the resulting mixture eluted onto a Varian 1g C8 Mega Bond Elut^{®} cartridge, the immobilised compound washed with 5% aqueous acetonitrile (10 ml), and finally liberated from the cartridge by elution with TFA (6 ml). The eluate was allowed to stand for 1 h and 30 min. at room temperature and then concentrated *in vacuo.* The residue was purified by column chromatography using a cyanopropyl column (Zorbax 300SB-CN) and a standard acetonitrile/TFA system. The column was heated to 65°C and the acetonitrile gradient was 0-100% in 60 minutes. The title compound (7.2 mg, 47 %) was isolated, and the product was analysed by PDMS. The m/z value for the protonated molecular ion was found to be 3881 ± 3. The resulting molecular weight is thus 3880 ± 3 amu (theoretical value 3880 amu).

### Example 41

### Synthesis of Arg^{18.23.26.30.34}Lys³⁸(N^{ε}-hexadecanoyl)-GLP-1 (7-38)-OH.

A mixture of Arg^{18.23.26.30.34}Lys³⁸-GLP-1(7-38)-OH (1.0 mg, 0.27 µmol), EDPA (0.34 mg, 2.7 µmol) and DMSO (600 µl) was gently shaken for 5 min. at room temperature. To the resulting mixture was added a solution of Pal-ONSu (0.28 mg, 0.8 µmol) in NMP (7 µl). The reaction mixture was gently shaken for 5 min. at room temperature, and then allowed to stand for an additional 6 h at room temperature. The reaction was quenched by the addition of a solution of glycine (1.6 mg, 21.7 µmol) in 50% aqueous ethanol (163 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound ( 0.17 mg, 16 %) was isolated, and the product was analysed by MALDI-MS. The m/z value for the protonated molecular ion was found to be 3961 ± 3. The resulting molecular weight is thus 3960 ± 3 amu (theoretical value 3960 amu).

### Example 42

### Synthesis of Arg^{26,34}Lys³⁸(N^{ε}-(ω-carboxytridecanoyl))-GLP-1(7-38)-OH.

A mixture of Arg^{26,34}Lys³⁸-GLP-1(7-38)-OH (14 mg, 4.0 µmol), EDPA (14.3 mg, 111 µmol), NMP (980 µl) and water (980 µl) was gently shaken for 5 min. at room temperature. To the resulting mixture was added a solution of HOOC-(CH₂)₁₂-COONSu (4.2 mg, 11.9 µmol) in NMP (105 µl), the reaction mixture was gently shaken for 1 h and 50 min. at room temperature. The reaction was quenched by the addition of a solution of glycine (6.5 mg, 87 µmol) in 50% aqueous ethanol (652 µl). The reaction mixture was purified by column chromatography using a cyanopropyl column (Zorbax 300SB-CN) and a standard acetonitrile/TFA system. The column was heated to 65°C and the acetonitrile gradient was 0-100% in 60 minutes. The title compound (5.8 mg, 39 %) was isolated, and the product was analysed by MALDI-MS. The m/z value for the protonated molecular ion was found to be 3780 ± 3. The resulting molecular weight is thus 3779 ± 3 amu (theoretical value 3781 amu).

### Example 43

### Synthesis of Arg³⁴Lys²⁶(N^{ε}-(γ-glutamyl(N^{α}-tetradecanoyl)))-GLP-1(7-37)-OH.

A mixture of Arg³⁴-GLP-1(7-37)-OH (15 mg, 4.4 µmol), EDPA (16 mg, 124 µmol), NMP (2 ml) and water (4.8 ml) was gently shaken for 5 min. at room temperature. To the resulting mixture was added a solution of N^{α}-tetradecanoyl-Glu(ONSu)-OBu^{t}, prepared as described in Example 29, (12.1 mg, 23.7 µmol) in NMP (303 µl), and the reaction mixture was gently shaken for 2 h at room temperature. The reaction was quenched by the addition of a solution of glycine (6.5 mg, 86.9 µmol) in 50% aqueous ethanol (652 µl). A 0.5 % aqueous solution of ammonium-acetate (50 ml) was added, and the resulting mixture eluted onto a Varian 1g C8 Mega Bond Elut^{®} cartridge, the immobilised compound washed with 5% aqueous acetonitrile (15 ml), and finally liberated from the cartridge by elution with TFA (6 ml). The eluate was allowed to stand for 1 h and 45 min. at room temperature and then concentrated *in vacuo.* The residue was purified by column chromatography using a cyanopropyl column (Zorbax 300SB-CN) and a standard acetonitrite/TFA system. The column was heated to 65°C and the acetonitrile gradient was 0-100% in 60 minutes. The title compound (3.9 mg, 26 %) was isolated, and the product was analysed by MALDI-MS. The m/z value for the protonated molecular ion was found to be 3723 ± 3. The resulting molecular weight is thus 3722 ± 3 amu (theoretical value 3723 amu).

### Example 44

### Synthesis of N^{α}-octadecanoyl-Glu(ONSu)-OBu^{t}.

To a suspension of H-Glu(OH)-OBu^{t}(2.82 g, 13.9 mmol), DMF (370 ml) and EDPA (1.79 g, 13.9 mmol) was added drop by drop a solution of Ste-ONSu (5.3 g, 13.9 mmol) in DMF (60 ml). Dichloromethane (35 ml) was added, and the reaction mixture was stirred for 24 h at room temperature and then concentrated *in* vacuo. The residue was partitioned between 10% aqueous citric acid (330 ml) and ethyl acetate (200 ml), and the phases were separated. The organic phase was concentrated *in vacuo* and the residue dissolved in DMF (60 ml). The resulting solution was added drop by drop to a 10% aqueous solution of citric acid (400 ml) kept at 0 °C. The precipitated compound was collected and washed with iced water and dried in a vacuum drying oven. The dried compound was dissolved in DMF (40 ml) and HONSu (1.63 g, 14.2 mmol) was added. To the resulting mixture was added a solution of DCC (2.66 g, 12.9 mmol) in dichloromethane (51 ml). The reaction mixture was stirred for 64 h at room temperature, and the precipitated compound was filtered off. The precipitate was recrystallised from n-heptane/2-propanol to give the title compound (4.96 g, 68 %).

### Example 45

### Synthesis of Arg^{26,34}Lys³⁸(N^{ε}-(γ-glutamyl(N^{α}-octadecanoyl)))-GLP-1(7-38)-OH.

A mixture of Arg^{26,34}-GLP-1(7-38)-OH (28 mg, 7.9 µmol), EDPA (28.6 mg, 221.5 µmol), NMP (1.96 ml) and water (1.96 ml) was gently shaken for 5 min. at room temperature. To the resulting mixture was added a solution of N^{α}-octadecanoyl-Glu(ONSu)-OBu^{t}(17.93 g, 31.6 µmol), prepared as described in Example 44, in NMP (448 µl), and the reaction mixture was gently shaken for 2 h at room temperature. The reaction was quenched by the addition of a solution of glycine (13.1 mg, 174 µmol) in 50% aqueous ethanol (1.3 ml). A 0.5 % aqueous solution of ammonium-acetate (120 ml) was added, and the resulting mixture was divided into two equal portions. Each portion was eluted onto a Varian 5 g C8 Mega Bond Elut^{®} cartridge, the immobilised compound washed with 5% aqueous acetonitrile (25 ml), and finally liberated from the cartridge by elution TFA (25 ml). The combined eluates were allowed to stand for 1 h and 25 min. at room temperature and then concentrated *in vacuo.* The residue was purified by column chromatography using a cyanopropyl column (Zorbax 300SB-CN) and a standard acetonitrile/TFA system. The column was heated to 65°C and the acetonitrile gradient was 0-100% in 60 minutes. The title compound (3.6 mg, 11 %) was isolated, and the product was analysed by MALDI-MS. The m/z value for the protonated molecular ion was found to be 3940 ± 3. The resulting molecular weight is thus 3939 ± 3 amu (theoretical value 3937 amu).

### BIOLOGICAL FINDINGS

### Protraction of GLP-1 derivatives after s.c. administration

The protraction of a number GLP-1 derivatives of the invention was determined by monitoring the concentration thereof in plasma after sc administration to healthy pigs, using the method described below. For comparison also the concentration in plasma of GLP-1(7-37) after sc. administration was followed. The results are given in Table 1. The protraction of other GLP-1 derivatives of the invention can be determined in the same way.

Pigs (50% Duroc, 25% Yorkshire, 25% Danish Landrace, app 40 kg) were fasted from the beginning of the experiment. To each pig 0.5 nmol of test compound per kg body weight was administered in a 50 µM isotonic solution (5 mM phosphate, pH 7.4, 0.02% Tween^{®}-20 (Merck), 45 mg/ml mannitol (pyrogen free, Novo Nordisk). Blood samples were drawn from a catheter in vena jugularis at the hours indicated in Table 1. 5 ml of the blood samples were poured into chilled glasses containing 175 µl of the following solution: 0.18 M EDTA, 1500 KIE/ml aprotinin (Novo Nordisk) and 3% bacitracin (Sigma), pH 7.4. Within 30 min, the samples were centrifuged for 10 min at 5-6000*g. Temperature was kept at 4°C. The supernatant was pipetted into different glasses and kept at minus 20°C until use.

The plasma concentrations of the peptides were determined by RIA using a monoclonal antibody specific for the N-terminal region of GLP-1 (7-37). The cross reactivities were less than 1% with GLP-1(1-37) and GLP-1(8-36)amide and < 0.1% with GLP-1 (9-37), GLP-1(10-36)amide and GLP-1 (11-36)amide. The entire procedure was carried out at 4°C.

The assay was carried out as follows: 100 µl plasma was mixed with 271 µl 96% ethanol, mixed using a vortex mixer and centrifuged at 2600*g for 30 min. The supernatant was decanted into Minisorp tubes and evaporated completely (Savant Speedvac AS290). The evaporation residue was reconstituted in the assay buffer consisting of 80 mM NaH₂PO₄/Na₂HPO₄, 0.1 % HSA (Orpha 20/21, Behring), 10 mM EDTA, 0.6 mM thiomersal (Sigma), pH 7.5. Samples were reconstituted in volumes suitable for their expected concentrations, and were allowed to reconstitute for 30 min. To 300 µl sample, 100 µl antibody solution in dilution buffer containing 40 mM NaH₂PO₄/Na₂HPO₄, 0.1 % HSA, 0.6 mM thiomersal, pH 7.5, was added. A non-specific sample was prepared by mixing 300 µl buffer with 100 µl dilution buffer. Individual standards were prepared from freeze dried stocks, dissolved in 300 µl assay buffer. All samples were pre-incubated in Minisorp tubes with antibody as described above for 72 h. 200 µl tracer in dilution buffer containing 6-7000 CPM was added, samples were mixed and incubated for 48 h. 1.5 ml of a suspension of 200 ml per litre of heparin-stabilised bovine plasma and 18 g per litre of activated carbon (Merck) in 40 mM NaH₂PO₄/Na₂HPO₄, 0.6 mM thiomersal, pH 7.5, was added to each tube. Before use, the suspension was mixed and allowed to stand for 2 h at 4°C. All samples were incubated for 1 h at 4°C and then centrifuged at 3400*g for 25 min. Immediately after the centrifugation, the supernatant was decanted and counted in a γ-counter. The concentration in the samples was calculated from individual standard curves. The following plasma concentrations were found, calculated as % of the maximum concentration for the individual compounds (n=2):

**Table 1**

| Test compound^{*)} | Hours after sc. administration | | | | | | | | |
|---|---|---|---|---|---|---|---|---|---|
| | 0.75 | 1 | 2 | 4 | 6 | 8 | 10 | 12 | 24 |
| GLP-1(7-37) | | 100 | 9 | 1 | | | | | |
| Example 25 | 73 | 92 | 100 | 98 | 82 | 24 | 16 | 16 | 16 |
| Example 17 | 76 | 71 | 91 | 100 | 84 | 68 | 30 | | 9 |
| Example 43 | | 39 | 71 | 93 | 100 | 91 | 59 | 50 | 17 |
| Example 37 | | 26 | 38 | 97 | 100 | 71 | 81 | 80 | 45 |
| Example 11 | 24 | 47 | 59 | 71 | 100 | 94 | 100 | | 94 |
| Example 12 | 36 | 54 | 65 | 94 | 80 | 100 | 85 | | 93 |
| Example 32 | 55 | 53 | 90 | 83 | 88 | 70 | 98 | 100 | 100 |
| Example 14 | 18 | 25 | 32 | 47 | 98 | 83 | 97 | | 100 |
| Example 13 | 15 | 22 | 38 | 59 | 97 | 85 | 100 | | 76 |
| Example 38 | 60 | 53 | 100 | 66 | 48 | 39 | 25 | 29 | 0 |
| Example 39 | 38 | 100 | 70 | 47 | 33 | 33 | 18 | 27 | 14 |
| Example 40 | 47 | 19 | 50 | 100 | 51 | 56 | 34 | 14 | 0 |
| Example 34 | 19 | 32 | 44 | 84 | 59 | 66 | 83 | 84 | 100 |

| | | | | | | | | | |
|---|---|---|---|---|---|---|---|---|---|
| ^{*)} The test compounds are the title compounds of the examples with the numbers given | | | | | | | | | |

As it appears from Table 1, the GLP-1 derivatives of the invention have a protracted profile of action relative to GLP-1 (7-37) and are much more persistent in plasma than GLP-1 (7-37). It also appears from Table 1 that the time at which the peak concentration in plasma is achieved varies within wide limits, depending on the particular GLP-1 derivative selected.

### Stimulation of cAMP formation in a cell line expressing the cloned human GLP-1 receptor

In order to demonstrate efficacy of the GLP-1 derivatives, their ability to stimulate formation of cAMP in a cell line expressing the cloned human GLP-1 receptor was tested. An EC₅₀ was calculated from the dose-response curve.

Baby hamster kidney (BHK) cells expressing the human pancreatic GLP-1 receptor were used (Knudsen and Pridal, 1996, Eur. J. Pharm. 318, 429-435). Plasma membranes were prepared (Adelhorst *et al*, 1994, J. Biol. Chem. 269, 6275) by homogenisation in buffer (10 mmol/l Tris-HCl and 30 mmol/l NaCl pH 7.4, containing, in addition, 1 mmol/l dithiothreitol, 5 mg/l leupeptin (Sigma, St. Louis, MO, USA), 5 mg/l pepstatin (Sigma, St. Louis, MO, USA), 100 mg/l bacitracin (Sigma, St. Louis, MO, USA), and 16 mg/l aprotinin (Novo Nordisk A/S, Bagsvaerd, Denmark)). The homogenate was centrifuged on top of a layer of 41 w/v% sucrose. The white band between the two layers was diluted in buffer and centrifuged. Plasma membranes were stored at -80°C until used.

The assay was carried out in 96-well microtiter plates in a total volume of 140 µl. The buffer used was 50 mmol/l Tris-HCl, pH 7.4 with the addition of 1 mmol/l EGTA, 1.5 mmol/l MgSO₄, 1.7 mmol/l ATP, 20 mM GTP, 2 mmol/l 3-isobutyl-1-methylxanthine, 0.01 % Tween-20 and 0.1 % human serum albumin (Reinst, Behringwerke AG, Marburg, Germany). Compounds to be tested for agonist activity were dissolved and diluted in buffer, added to the membrane preparation and the mixture was incubated for 2 h at 37°C. The reaction was stopped by the addition of 25 µl of 0.05 mol/l HCl. Samples were diluted 10 fold before analysis for cAMP by a scintillation proximity assay (RPA 538, Amersham, UK). The following results were found:

| Test compound^{*)} | EC₅₀, pM | Test compound^{*)} | EC₅₀, pM |
|---|---|---|---|
| GLP-1 (7-37) | 61 | Example 31 | 96 |
| Example 45 | 120 | Example 30 | 41 |
| Example 43 | 24 | Example 26 | 8.8 |
| Example 40 | 55 | Example 25 | 99 |
| Example 39 | 5.1 | Example 19 | 79 |
| Example 38 | 54 | Example 16 | 3.5 |
| Example 37 | 60 | | |

| | | | |
|---|---|---|---|
| ^{*)} The test compounds are the title compounds of the examples with the numbers given. | | | |

## Claims

1. A derivative of GLP-1 (7-37) or an analogue of GLP-1 (7-37) which derivative is an agonist of the human GLP-1 receptor, **characterized in that** the derivative has only one lipophilic substituent which is attached to an amino acid residue which is not the N-terminal or C-terminal amino acid residue.

2. A derivative of GLP-1 (7-37) or an analogue of GLP-1 (7-37) which derivative is an agonist of the human GLP-1 receptor; **characterized in that** the derivative has only two lipophilic substituents, one of which is attached to the C-terminal amino acid residue while the other is attached to an amino acid residue which is not the N-terminal or C-terminal amino acid residue.

3. A derivative of GLP-1 (7-37) or an analogue of GLP-1 (7-37) which derivative is an agonist of the human GLP-1 receptor; **characterised in that** the derivative has only two lipophilic substituents which are attached, to amino acid residues which are not the N-terminal or C-terminal amino acid residue.

4. A derivative of an analogue of GLP-1 (7-37) which derivative is an agonist of the human GLP-1 receptor and wherein the analogue is GLP-1 (7-C) where C is from 38 to 45; **characterized in that** it has only one lipophilic substituent which is attached to the C-terminal amino acid residue.

5. A derivative of any of claims 1-4, wherein the analogue of GLP-1 (7-37) is selected from the group consisting of :
Arg²⁶-GLP-1(7-37); Arg³⁴-GLP-1(7-37); Lys³⁶-GLP-1(7-37); Arg^{26,34}Lys³⁶-GLP-1(7-37); Arg^{26,34}Lys³⁸GLP-1(7-38); Arg^{26,34}Lys³⁹-GLP-1(7-39); Arg^{26,34}Lys⁴⁰-GLP-1(7-40); Arg²⁶Lys³⁶-GLP-1(7-37); Arg³⁴Lys³⁶-GLP-1(7-37); Arg²⁶Lys³⁹-GLP-1(7-39); Arg³⁴Lys⁴⁰-GLP-1(7-40); Arg^{26,34}Lys^{36,39}-GLP-1(7-39); Arg^{26,34}Lys^{36,40}-GLP-1(7-40); Gly⁸Arg26-GLP-1(7-37); Gly⁸Arg³⁴-GLP-1(7-37); Gly⁸Lys³⁶-GLP-1(7-37); Gly⁸Arg^{26,34}Lys³⁶-GLP-1(7-37); Gly⁸Arg^{26,34}Lys³⁹-GLP-1(7-39); Gly⁸Arg^{26,34}Lys⁴⁰-GLP-1(7-40); Gly⁸Arg²⁶Lys³⁶-GLP-1(7-37); Gly⁸Arg³⁴Lys³⁶-GLP-1(7-37); Gly⁸Arg²⁶Lys³⁹-GLP-1(7-39); Gly⁸Arg³⁴Lys⁴⁰-GLP-1(7-40); Gly⁸Arg^{26,34}Lys^{36,39}-GLP-1(7-39) and Gly⁸Arg^{26,34}Lys^{36,40}-GLP-1(7-40).

6. A derivative of any of claims 1-4, wherein the analogue of GLP-1 (7-37) is selected from the group consisting of:
Arg^{26,34}Lys³⁸GLP-1(7-38); Arg^{26,36}Lys³⁹GLP-1(7-39) ; Arg^{26,34}Lys⁴⁰GLP-1(7-40); Arg^{26,34}Lys⁴¹GLP-1(7-41); Arg^{26,34}Lys⁴²GLP-1(7-42); Arg^{26,34}Lys⁴³GLP-1(7-43); Arg^{26,34}Lys⁴⁴GLP-1(7-44); Arg^{26,34}Lys⁴⁵GLP-1(7-45); Arg^{26,34}Lys³⁸GLP-1(1-38); Arg^{26,34}Lys³⁹GLP-1(1-39); Arg^{26,34}Lys⁴⁰GLP-1(1-40); Arg^{26,34}Lys⁴¹GLP-1(1-41); Arg^{26,34}Lys⁴²GLP-1(1-42); Arg^{26,34}Lys⁴³GLP-1(1-43); Arg^{26,34}Lys⁴⁴GLP-1(1-44); Arg^{26,34}Lys⁴⁵GLP-1(1-45) ; Arg^{26,34}Lys³⁸GLP-1(2-38); Arg^{26,34}Lys³⁹GLP-1(2-39); Arg^{26,34}Lys⁴⁰GLP-1(2-40); Arg^{26,34}Lys⁴¹GLP-1(2-41); Arg^{26,34}Lys⁴²GLP-1(2-42); Arg^{26,34}Lys⁴³GLP-1(2-43); Arg^{26,34}Lys⁴⁴GLP-1(2-44); Arg^{26,34}Lys⁴⁵GLP-1(2-45); Arg^{26,34}Lys³⁸GLP-1(3-38); Arg^{26,34}Lys³⁹GLP-1(3-39); Arg^{26,34}Lys⁴⁰GLP-1(3-40); Arg^{26,34}Lys⁴¹GLP-1(3-41); Arg^{26,34}Lys⁴²GLP-1(3-42); Arg^{26,34}Lys⁴³GLP-1(3-43); Arg^{26,34}Lys⁴⁴GLP-1(3-44); Arg^{26,34}Lys⁴⁵GLP-1(3-45); Arg^{26,34}Lys³⁸GLP-1(4-38); Arg^{26,34}Lys³⁹GLP-1(4-39); Arg^{26,34}Lys⁴⁰GLP-1(4-40); Arg^{26,34}Lys⁴¹GLP-1(4-41); Arg^{26,34}Lys⁴²GLP-1(4-42); Arg^{26,34}Lys⁴³GLP-1(4-43); Arg^{26,34}Lys⁴⁴GLP-1(4-44); Arg^{26,34}Lys⁴⁵GLP-1(4-45); Arg^{26,34}Lys³⁸GLP-1(5-38); Arg^{26,34}Lys³⁹GLP-1(5-39); Arg^{26,34}Lys⁴⁰GLP-1(5-40); Arg^{26,34}Lys⁴¹GLP-1(5-41); Arg^{26,34}Lys⁴²GLP-1(5-42); Arg^{26,34}Lys⁴³GLP-1(5-43); Arg^{26,34}Lys⁴⁴GLP-1(5-44); Arg^{26,34}Lys⁴⁵GLP-1(5-45); Arg^{26,34}Lys³⁸GLP-1(6-38); Arg^{26,34}Lys³⁹GLP-1(6-39); Arg^{26,34}Lys⁴⁰GLP-1(6-40); Arg^{26,34}Lys⁴¹GLP-1(6-41); Arg^{26,34}Lys⁴²GLP-1(6-42); Arg^{26,34}Lys⁴³GLP-1(6-43); Arg^{26,34}Lys⁴⁴GLP-1(6-44); Arg^{26,34}Lys⁴⁵GLP-1(6-45); Arg²⁶Lys³⁶GLP-1(1-38); Arg³⁴Lys³⁶GLP-1(1-38); Arg^{26,34}Lys^{36,38}GLP-1(1-38); Arg²⁶Lys³⁸GLP-1(7-38); Arg³⁴Lys³⁸GLP-1(7-38); Arg^{26,34}Lys^{36,38}GLP-1(7-38); Arg^{26,34}Lys³⁸GLP-1(7-38); Arg²⁶Lys³⁹GLP-1(1-39); Arg³⁴Lys³⁹GLP-1(1-39); Arg^{26,34}Lys^{36,39}GLP-1(1-39); Arg²⁶Lys³⁹GLP-1(7.39); Arg³⁴Lys³⁹GLP-1(7-39) and Arg^{26,34}Lys^{36,39}GLP-1(7-39).

7. A derivative of an analogue of GLP-1 (7-37) which derivative is an agonist of the human GLP-1 receptor, wherein at least one amino acid residue has a lipophilic substituent attached and the analogue is selected from the group consisting of:
Arg^{26,34}Lys³⁶GLP-1(7-38); Arg^{26,34}Lys³⁹GLP-1(7-39); Arg^{26,34}Lys⁴⁰GLP-1(7-40); Arg^{26,34}Lys⁴¹GLP-1(7-41); Arg^{26,34}Lys⁴²GLP-1(7-42); Arg^{26,34}Lys⁴³GLP-1(7-43); Arg^{26,34}Lys⁴⁴GLP-1(7-44); Arg^{26,34}Lys⁴⁵GLP-1(7-45); Arg^{26,34}Lys³⁸GLP-1(1-38); Arg^{26,34}Lys³⁹GLP-1(1-39); Arg^{26,34}Lys⁴⁰GLP-1(1-40); Arg^{26,34}Lys⁴¹GLP-1(1-41); Arg^{26,34}Lys⁴²GLP-1(1-42); Arg^{26,34}Lys⁴³GLP-1(1-43); Arg^{26,34}Lys⁴⁴GLP-1(1-44); Arg^{26,34}Lys⁴⁵GLP-1(1-45); Arg^{26,34}Lys³⁸GLP-1(2-38); Arg^{26,34}Lys³⁹GLP-1(2-39); Arg^{26,34}Lys⁴⁰GLP-1(2-40); Arg^{26,34}Lys⁴¹GLP-1(2-41); Arg^{26,34}Lys⁴²GLP-1(2-42); Arg^{26,34}Lys⁴³GLP-1(2-43); Arg^{26,34}Lys⁴⁴GLP-1(2-44); Arg^{26,34}Lys⁴⁵GLP-1(2-45); Arg^{26,34}Lys³⁶GLP-1(3-38); Arg^{26,34}Lys³⁹GLP-1(3-39); Arg^{26,34}Lys⁴⁰GLP-1(3-40); Arg^{26,34}Lys⁴¹GLP-1(3-41); Arg^{26,34}Lys⁴²GLP-1(3-42); Arg^{26,34}Lys⁴³GLP-1(3-43); Arg^{26,34}Lys⁴⁴GLP-1(3-44); Arg^{26,34}Lys⁴⁵GLP-1(3-45); Arg^{26,34}Lys³⁸GLP-1(4-38); Arg^{26,34}Lys³⁹GLP-1(4-39); Arg^{26,34}Lys⁴⁰GLP-1(4-40); Arg^{26,34}Lys⁴¹GLP-1(4-41); Arg^{26,34}Lys⁴²GLP-1(4-42); Arg^{26,34}Lys⁴³GLP-1(4-43); Arg^{26,34}Lys⁴⁴GLP-1(4-44); Arg^{26,34}Lys⁴⁵GLP-1(4-45); Arg^{26,34}Lys³⁸GLP-1(5-38); Arg^{26,34}Lys³⁹GLP-1(5-39); Arg^{26,34}Lys⁴⁰GLP-1(5-40);Arg^{26,34}Lys⁴¹GLP-1(5-41); Arg^{26,34}Lys⁴²GLP-1-(5-42); Arg^{26,34}Lys⁴³GLP-1(5-43);Arg^{26,34}Lys⁴⁴GLP-1(5-44);Arg^{26,34}Lys⁴⁵GLP-1(5-45); Arg^{26.34}Lys³⁸GLP-1(6-38); Arg^{26,34}Lys³⁹GLP-1(6-39);Arg^{26,34}Lys⁴⁰GLP-1(6-40); Arg^{26,34}Lys⁴¹GLP-1(6-41); Arg^{26,34}Lys⁴²GLP-1(6-42); Arg^{26,34}Lys⁴³GLP-1(6-43); Arg^{26,34}Lys⁴⁴GLP-1(6-44); Arg^{26,34}Lys⁴⁵GLP-1(6-45); Arg²⁶Lys³⁸GLP-1(1-38); Arg³⁴Lys³⁸GLP-1(1-38); Arg^{26,34}Lys^{36,38}GLP-1(1-38); Arg²⁶Lys³⁸GLP-1(7-38); Arg³⁴Lys³⁸GLP-1(7-38); Arg^{26,34}Lys^{36,38}GLP-1(7-38); Arg^{26,34}Lys³⁸GLP-1(7-38); Arg²⁶Lys³⁹GPL-1(1-39); Arg³⁴Lys³⁹GLP-1(1-39); Arg^{26,34}Lys^{36,39}GLP-1(1-39); Arg²⁶Lys³⁹GLP-1(7-39); Arg³⁴Lys³⁹GLP-1(7-39) and Arg^{26,34}Lys^{36,39}GLP-1(7-39),
with the proviso that if only one lipophilic substituent is present and this substituent is attached to the N-terminal or to the C-terminal amino acid residue, then this substituent is an alkyl group or a group which has an ω-carboxylic group.

8. A derivative of any of the preceding claims, wherein the lipophilic substituent comprises from 4 to 40 carbon atoms, more preferred from 8 to 25 carbon atoms.

9. A derivative of any of the preceding claims, wherein a lipophilic substituent is attached to an amino acid residue in such a way that a carboxyl group of the lipophilic substituent forms an amide bond with an amino group of the amino acid residue.

10. A derivative of any of the claims 1-8, wherein a lipophilic substituent is attached to an amino acid residue in such a way that an amino group of the lipophilic substituent forms an amide bond with a carboxyl group of the amino acid residue.

11. A derivative of any of the preceding claims, wherein the lipophilic substituent is attached to the amino acid residue by means of a spacer.

12. A derivative of claim 11, wherein the spacer is an unbranched alkane α,ω-dicarboxylic acid group having from 1 to 7 methylene groups, preferably two methylene groups, which form a bridge between an amino group of the peptide and an amino group of the lipophilic substituent

13. A derivative of claim 11, wherein the spacer is an amino acid residue except Cys, or a dipeptide such as Gly-Lys.

14. A derivative of claim 13, wherein a carboxyl group of the parent peptide forms an amide bond with an amino group of Lys or a dipeptide containing a Lys residue, and the other amino group of the Lys spacer or a dipeptide spacer containing a Lys .residue forms an amide bond with a carboxyl group of the lipophilic substituent.

15. A derivative of claim 13, wherein an amino group of the peptide forms an amide bond with a carboxylic group of the amino acid residue or dipeptide spacer, and an amino group of the amino acid residue or dipeptide spacer forms an amide bond with a carboxyl group of the lipophilic substituent.

16. A derivative of claim 13, wherein a carboxyl group of the peptide forms an amide bond with an amino group of the amino acid residue spacer or dipeptide spacer, and a carboxyl group of the amino acid residue spacer or dipeptide spacer forms an amide bond with an amino group of the lipophilic substituent.

17. A derivative of claim 13, wherein a carboxyl group of the peptide forms an amide bond with an amino group of a spacer which is Asp or Glu, or a dipeptide spacer containing an Asp or Glu residue, and a carboxyl group of the spacer forms an amide bond with an amino group of the lipophilic substituent.

18. A derivative of any of the preceding claims, wherein the lipophilic substituent comprises a partially or completely hydrogenated cyclopentanophenathrene skeleton.

19. A derivative of any of the claims 1-17, wherein the lipophilic substituent is an straight-chain or branched alkyl group.

20. A derivative of any of the claims 1-17, wherein the lipophilic substituent is an acyl group of a straight-chain or branched fatty acid.

21. A derivative of claim 20, wherein the acyl group is selected form the group comprising CH₃(CH₂)ₙCO-, wherein n is 4 to 38, preferably CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-.

22. A derivative of any of the claims 1-17, wherein the lipophilic substituent is an acyl group of a straight-chain or branched alkane α,ω-dicarboxylic acid.

23. A derivative of claim 22, wherein the acyl group is selected from the group comprising HOOC(CH₂)ₘCO-, wherein m is from 4 to 38, preferably from 4 to 24, more preferred selected from the group comprising HOOC(CH₂)₁₄CO-, HOOC(CH₂)₁₆CO-, HOOC(CH₂)₁₈CO-, HOOC(CH₂)₂₀CO- and HOOC(CH₂)₂₂CO-.

24. A derivative of any of the claims 1-17, wherein the lipophilic substituent with the spacer is a group of the formula CH₃(CH₂)ₚ((CH₂)_{q}COOH)CHNH-CO(CH₂)₂CO-, wherein p and q are integers and p+q is an integer of from 8 to 33, preferably from 12 to 28.

25. A derivative of any of the claims 1-17, wherein the lipophilic substituent with the spacer is a group of the formula CH₃(CH₂)ᵣCO-NHCH(COOH)(CH₂)₂CO-, wherein r is an integer of from 10 to 24.

26. A derivative of any of the claims 1-17 wherein the lipophilic substituent with the spacer is a group of the formula CH₃(CH₂)₅CO-NHCH((CH₂)₂COOH)CO-, wherein s is an integer of from 8 to 24.

27. A derivative of any of the claims 1-17, wherein the lipophilic substituent with the spacer is a group of the formula -NHCH(COOH)(CH₂)₄NH-CO(CH₂)₁₁CH₃, wherein u is an integer of from 8 to 18.

28. A derivative of any of the claims 1-17 wherein the lipophilic substituent with the spacer is a group of the formula -NHCH(COOH)(CH₂)₄NH-COCH((CH₂)₂COOH)NH-CO(CH₂)_{w}CH₃, wherein w is an integer of from 10 to 16.

29. A derivative of any of the claims 1-17 wherein the lipophilic substituent with the spacer is a group of the formula -NHCH(COOH)(CH₂)₄NH-CO(CH₂)₂CH(COOH)NH-CO(CH₂)ₓCH₃, wherein x is an integer of from 10 to 16.

30. A derivative of any of the claims 1-17 wherein the lipophilic substituent with the spacer is a group of the formula-NHCH(COOH)(CH₂)₄NH-CO(CH₂)₂CH(COOH)NH-CO(CH₂)₂CH₃, wherein y is zero or an integer of from 1 to 22.

31. A derivative of any of the preceding claims, wherein a total of up to six amino acid residues of GLP-1 (7-37) have been exchanged with any α-amino acid residue which can be coded for by the genetic code.

32. A derivative of claim 1 which is Lys²⁶(N^{ε}-tetradecanoyl)-GLP-1(7-37).

33. A derivative of claim 1 which is Lys³⁴(N^{ε}-tetradecanoyl)-GLP-1(7-37).

34. A derivative of claim 2 which is Lys^{26,34}-bis(N^{ε}-tetradecanoyl)-GLP-1(7-37).

35. A derivative of claim 1 which is Lys²⁶(N^{ε}-tetradecanoyl)Arg³⁴-GLP-1 (7-37).

36. A derivative of claim 1 which is Gly⁸Arg^{26,34}Lys³⁵(N^{ε}-tetradecanoyl)-GLP-1(7-37).

37. A derivative of claim 1 which is Arg^{26,34}Lys³⁶(N^{ε}-tetradecanoyl)-GLP-1(7-37).

38. A derivative of claim 3 which is Lys^{26,34}bis(N^{ε}-(ω-carboxynonadecanoyl))-GLP-1(7-37).

39. A derivative of claim 1 which is Arg³⁴Lys²⁶(N^{ε}-(ω-carboxynonadecanoyl))-GLP-1 (7-37).

40. A derivative of claim 1 which is Arg³⁴Lys²⁶(N^{ε}-(ω-carboxyheptadecanoyl))-GLP-1(7-37).

41. A derivative of claim 1 which is Arg^{26,34}Lys³⁶(N^{ε}-(ω-carboxyheptadecanoyl))-GLP-1(7-37).

42. A derivative of claim 1 which is Arg^{26,34}Lys³⁶(N^{ε}-(ω-carboxyundecanoyl))-GLP-1(7-37).

43. A derivative of claim 3 which is Lys^{26,34}bis(N^{ε}-(ω-carborytmderanoyl))-GLP-1 (7-37).

44. A derivative of claim 1 which is Arg³⁴Lys²⁶(N^{ε}-(ω-carboxyundecanoyl))-GLP-1(7-37).

45. A derivative of claim 1 which is Arg³⁴Lys²⁶(N^{ε}-(ω-carboxyheptanoyl))-GLP-1(7-37).

46. A derivative of claim 1 which is Arg^{26,34}Lys³⁶(N^{ε}(ω-carboxyheptanoyl))-GLP-1 (7-37).

47. A derivative of claim 3 which is Lys^{26,34}bis(N^{ε}-(ω-carboxyheptanoyl))-GLP-1(7-37).

48. A derivative of claim 1 which is Arg³⁴Lys²⁶(N^{ε}-(ω-carboxypentadecanoyl))-GLP-1 (7-37).

49. A derivative of claim 1 which is Arg³⁴Lys²⁶(N^{ε}-lithocholyl)-GLP-1 (7-37).

50. A derivative of claim 3 which is Lys^{26,34}-bis(N^{ε}-(ω-carboxytridecanoyl))-GLP-1(7-37).

51. A derivative of claim 3 which is Ly^{26,34}-bis(N^{ε}-(γ-glutamyl(N^{α}-tetradecanoyl)))-GLP-1(7-37).

52. A derivative of claim 3 which is Lys^{26,34}-bis(N^{ε}-(γ-glutamyl(N^{α}-hexadecanoyl)))-GLP-1(7-37).

53. A derivative of claim 1 which is Arg³⁴Lys²⁶(N^{ε}-(γ-glutamyl(N^{α}-hexadecanoyl)))-GLP-1(7-37).

54. A derivative of claim 1 which is Arg³⁴Lys²⁶(N^{ε}-(γ-glutamyl(N^{α}-tetradecanoyl)))-GLP-1(7-37).

55. A pharmaceutical composition comprising a derivative of any of claims 1-54 and a pharmaceutically acceptable vehicle or carrier.

56. Use of a derivative of any of claims 1-54 for the preparation of a medicament which has a protracted profile of action relative to GLP-1 (7-37).

57. Use of a derivative of any of claims 1-54 for the preparation of a medicament for the treatment of non-insulin dependent diabetes mellitus.

58. Use of a derivative of any of claims 1-54, for the preparation of a medicament for the treatment of insulin dependent diabetes mellitus.

59. Use of a derivative of any of claims 1-54 for the preparation of a medicament for the treatment of obesity.

60. Use of a derivative of any of Claims 1-54 for the preparation of a medicament for the treatment of one or more conditions selected from non-insulin dependent diabetes mellitus, insulin dependent diabetes mellitus and obesity.

## Patentansprüche

1. Derivat von GLP-1(7-37) oder Analogon von GLP-1(7-37), wobei das Derivat ein Agonist des menschlichen GLP-1-Rezeptors ist, **dadurch gekennzeichnet, dass** das Derivat nur einen lipophilen Substituenten aufweist, der an den N-terminalen oder den C-terminalen Aminosäurerest angelagert ist.

2. Derivat von GLP-1(7-37) oder Analogon von GLP-1(7-37), wobei das Derivat ein Agonist des menschlichen GLP-1-Rezeptors ist, **dadurch gekennzeichnet, dass** das Derivat nur zwei lipophile Substituenten aufweist, wobei einer davon an den C-terminalen Aminosäurerest angelagert ist, während der andere an einen Aminosäurerest angelagert ist, bei welchem es sich nicht um den N-terminalen oder C-terminalen Aminosäurerest handelt.

3. Derivat von GLP-1(7-37) oder Analogon von GLP-1(7-37), wobei das Derivat ein Agonist des menschlichen GLP-1-Rezeptors ist, **dadurch gekennzeichnet, dass** das Derivat nur zwei lipophile Substituenten aufweist, die an Aminosäurereste angelagert sind, bei welchen es sich nicht um den N-terminalen oder C-terminalen Aminosäurerest handelt.

4. Derivat eines Analogons von GLP-1(7-37), wobei das Derivat ein Agonist des menschlichen GLP-1-Rezeptors ist und wobei das Analogon GLP-1(7-C) ist, wobei C 38 bis 45 beträgt, **dadurch gekennzeichnet, dass** das Derivat nur einen lipophilen Substituenten aufweist, der an den C-terminalen Aminosäurerest angelagert ist.

5. Derivat nach einem der Ansprüche 1 bis 4, wobei das Analogon von GLP-1(7-37) ausgewählt ist aus der Gruppe, bestehend aus Arg²⁶-GLP-1(7-37); Arg³⁴-GLP-1(7-37); Lys³⁶-GLP-1(7-37); Arg^{26,34}Lys³⁶-GLP-1(7-37); Arg^{26,34}Lys³⁸GLP-1(7-38); Arg^{26,34}Lys³⁹-GLP-1(7-39); Arg^{26,34}Lys⁴⁰-GLP-1(7-40); Arg²⁶Lys³⁶-GLP-1(7-37); Arg³⁴Lys³⁶-GLP-1(7-37); Arg²⁶Lys³⁹-GLP-1(7-39); Arg³⁴Lys⁴⁰-GLP-1(7-40); Arg^{26,34}Lys^{36,39}-GLP-1(7-39); Arg^{26,34}Lys^{36,40}-GLP-1(7-40); Gly⁸Arg26-GLP-1(7-37); Gly⁸Arg³⁴-GLP-1(7-37); Gly⁸Lys³⁶-GLP-1(7-37); Gly⁸Arg^{26,34}Lys³⁶-GLP-1(7-37); Gly⁸Arg^{26,34}Lys³⁹-GLP-1(7-39); Gly⁸Arg^{26,34}Lys⁴⁰-GLP-1(7-40); Gly⁸Arg²⁶Lys³⁶-GLP-1(7-37); Gly⁸Arg³⁴Lys³⁶-GLP-1(7-37); Gly⁸Arg²⁶Lys³⁹-GLP-1(7-39); Gly⁸Arg³⁴Lys⁴⁰-GLP-1(7-40); Gly⁸Arg^{26,34}Lys^{36,39}-GLP-1(7-39) und Gly⁸Arg^{26,34}Lys^{36,40}-GLP-1(7-40).

6. Derivat nach einem der Ansprüche 1 bis 4, wobei das Analogon von GLP-1(7-37) ausgewählt ist aus der Gruppe, bestehend aus
Arg^{26,34}Lys³⁸GLP-1(7-38); Arg^{26,34}Lys³⁹GLP-1(7-39); Arg^{26,34}Lys⁴⁰GLP-1 (7-40); Arg^{26,34}Lys⁴¹GLP-1(7-41); Arg^{26,34}Lys⁴²GLP-1(7-42); Arg^{26,34}Lys⁴³GLP-1(7-43); Arg^{26,34}Lys⁴⁴GLP-1(7-44); Arg^{26,34}Lys⁴⁵GLP-1(7-45); Arg^{26,34}Lys³⁸GLP-1(1-38); Arg^{26,34}Lys³⁹GLP-1(1-39); Arg^{26,34}Lys⁴⁰GLP-1(1-40); Arg^{26,34}Lys⁴¹GLP-1(1-41); Arg^{26,34}Lys⁴²GLP-1(1-42); Arg^{26,34}Lys⁴³GLP-1(1-43); Arg^{26,34}Lys⁴⁴GLP-1(1-44); Arg^{26,34}Lys⁴⁵GLP-1(1-45); Arg^{26,34}Lys³⁸GLP-1(2-38); Arg^{26,34}Lys³⁹GLP-1(2-39); Arg^{26,34}Lys⁴⁰GLP-1(2-40); Arg^{26,34}Lys⁴¹GLP-1(2-41); Arg^{26,34}Lys⁴²GLP-1(2-42); Arg^{26,34}Lys⁴³GLP-1(2-43); Arg^{26,34}Lys⁴⁴GLP-1(2-44); Arg^{26,34}Lys⁴⁵GLP-1 (2-45); Arg^{26,34}Lys³⁸GLP-1(3-38); Arg^{26,34}Lys³⁹GLP-1(3-39); Arg^{26,34}Lys⁴⁰GLP-1(3-40); Arg^{26,34}Lys⁴¹GLP-1(3-41); Arg^{26,34}Lys⁴²GLP-1(3-42); Arg^{26,34}Lys⁴³GLP-1(3-43); Arg^{26,34}Lys⁴⁴GLP-1(3-44); Arg^{26,34}Lys⁴⁵GLP-1(3-45); Arg^{26,34}Lys³⁸GLP-1(4-38); Arg^{26,34}Lys³⁹GLP-1(4-39), Arg^{26,34}Lys⁴⁰GLP-1(4-40); Arg^{26,34}Lys⁴¹GLP-1(4-41); Arg^{26,34}Lys⁴²GLP-1(4-42); Arg^{26,34}Lys⁴³GLP-1(4-43); Arg^{26,34}Lys⁴⁴GLP-1(4-44); Arg^{26,34}Lys⁴⁵GLP-1(4-45); Arg^{26,34}Lys³⁸GLP-1(5-38); Arg^{26,34}Lys³⁹GLP-1(5-39); Arg^{26,34}Lys⁴⁰GLP-1(5-40); Arg^{26,34}Lys⁴¹GLP-1(5-41); Arg^{26,34}Lys⁴²GLP-1(5-42); Arg^{26,34}Lys⁴³GLP-1(5-43); Arg^{26,34}Lys⁴⁴GLP-1(5-44); Arg^{26,34}Lys⁴⁵GLP-1(5-45); Arg^{26,34}Lys³⁸GLP-1(6-38); Arg^{26,34}Lys³⁹GLP-1(6-39); Arg^{26,34}Lys⁴⁰GLP-1(6-40); Arg^{26,34}Lys⁴¹GLP-1(6-41); Arg^{26,34}Lys⁴²GLP-1(6-42); Arg^{26,34}Lys⁴³GLP-1(6-43); Arg^{26,34}Lys⁴⁴GLP-1(6-44); Arg^{26,34}Lys⁴⁵GLP-1(6-45); Arg²⁶Lys³⁸GLP-1(1-38); Arg³⁴Lys³⁸GLP-1(1-38); Arg^{26,34}Lys^{36,38}GLP-1(1-38); Arg²⁶Lys³⁸GLP-1(7-38); Arg³⁴Lys³⁸GLP-1(7-38); Arg^{26,34}Lys^{36,38}GLP-1(7-38); Arg^{26,34}Lys³⁸GLP-1 (7-3 8); Arg²⁶Lys³⁹GLP-1(1-39); Arg³⁴Lys³⁹GLP-1(1-39); Arg^{26,34}Lys^{36,39}GLP-1(1-39); Arg²⁶Lys³⁹GLP-1(7-39); Arg³⁴Lys³⁹GLP-1(7-39) und Arg^{26,34}Lys^{36,39}GLP-1(7-39).

7. Derivat oder Analogon von GLP-1(7-37), wobei das Derivat ein Agonist des menschlichen GLP-1-Rezeptors ist, wobei mindestens ein Aminosäurerest einen angelagerten lipophilen Substituenten aufweist und das Analogon ausgewählt ist aus der Gruppe, bestehend aus
Arg^{26,34}Lys³⁸GLP-1(7-38); Arg^{26,34}Lys³⁹GLP-1(7-39); Arg^{26,34}Lys⁴⁰GLP-1(7-40); Arg^{26,34}Lys⁴¹GLP-1(7-41); Arg^{26,34}Lys⁴²GLP-1(7-42); Arg^{26,34}Lys⁴³GLP-1(7-43); Arg^{26,34}Lys⁴⁴GLP-1(7-44); Arg^{26,34}Lys⁴⁵GLP-1(7-45); Arg^{26,34}Lys³⁸GLP-1(1-38); Arg^{26,34}Lys³⁹GLP-1(1-39); Arg^{26,34}Lys⁴⁰GLP-1(1-40); Arg^{26,34}Lys⁴¹GLP-1(1-41); Arg^{26,34}Lys⁴²GLP-1(1-42); Arg^{26,34}Lys⁴³GLP-1(1-43); Arg^{26,34}Lys⁴⁴GLP-1(1-44); Arg^{26,34}Lys⁴⁵GLP-1(1-45); Arg^{26,34}Lys³⁸GLP-1(2-38); Arg^{26,34}Lys³⁹GLP-1(2-39); Arg^{26,34}LyS⁴⁰GLP-1(2-40), Arg^{26,34}Lys⁴¹GLP-1(2-41); Arg^{26,34}Lys⁴²GLP-1(2-42); Arg^{26,34}Lys⁴³GLP-1(2-43); Arg^{26,34}Lys⁴⁴GLP-1(2-44); Arg^{26,34}Lys⁴⁵GLP-1 (2-45); Arg^{26,34}Lys³⁸GLP-1(3-38); Arg^{26,34}Lys³⁹GLP-1(3-39); Arg^{26,34}Lys⁴⁰GLP-1(3-40); Arg^{26,34}LyS⁴¹GLP-1(3-41); Arg^{26,34}Lys⁴²GLP-1(3-42); Arg^{26,34}Lys⁴³GLP-1(3-43); Arg^{26,34}Lys⁴⁴GLP-1(3-44); Arg^{26,34}Lys⁴⁵GLP-1(3-45); Arg^{26,34}Lys³⁸GLP-1(4-38); Arg^{26,34}Lys³⁹GLP-1(4-39); Arg^{26,34}Lys⁴⁰GLP-1(4-40); Arg^{26,34}Lys⁴¹GLP-1(4-41); Arg^{26,34}Lys⁴²GLP-1(4-42); Arg^{26,34}Lys⁴³GLP-1(4-43); Arg^{26,34}Lys⁴⁴GLP-1(4-44); Arg^{26,34}Lys⁴⁵GLP-1(4-45); Arg^{26,34}Lys³⁸GLP-1(5-38); Arg^{26,34}Lys³⁹GLP-1(5-39); Arg^{26,34}Lys⁴⁰GLP-1(5-40); Arg^{26,34}Lys⁴¹GLP-1(5-41); Arg^{26,34}Lys⁴²GLP-1(5-42); Arg^{26,34}Lys⁴³GLP-1(5-43); Arg^{26,34}Lys⁴⁴GLP-1(5-44); Arg^{26,34}Lys⁴⁵GLP-1(5-45); Arg^{26,34}Lys³⁸GLP-1(6-38); Arg^{26,34}Lys³⁹GLP-1(6-39); Arg^{26,34}Lys⁴⁰GLP-1(6-40); Arg^{26,34}Lys⁴¹GLP-1(6-41); Arg^{26,34}Lys⁴²GLP-1(6-42); Arg^{26,34}Lys⁴³GLP-1(6-43); Arg^{26,34}Lys⁴⁴GLP-1(6-44); Arg^{26,34}Lys⁴⁵GLP-1(6-45); Arg²⁶Lys³⁸GLP-1(1-38); Arg³⁴Lys³⁸GLP-1(1-38); Arg^{26,34}Lys^{36,38}GLP-1(1-38); Arg²⁶Lys³⁸GLP-1(7-38); Arg³⁴Lys³⁸GLP-1(7-38); Arg^{26,34}Lys^{36,38}GLP-1(7-38); Arg^{26,34}Lys³⁸GLP-1 (7-38); Arg²⁶Lys³⁹GLP-1(1-39); Arg³⁴Lys³⁹GLP-1(1-39); Arg^{26,34}Lys^{36,39}GLP-1(1-39); Arg²⁶Lys³⁹GLP-1(7-39); Arg³⁴Lys³⁹GLP-1(7-39) und Arg^{26,34}Lys^{36,39}GLP-1(7-39),
mit der Maßgabe, dass, falls nur ein lipophiler Substituent vorliegt und dieser Substituent an den N-terminalen oder an den C-terminalen Aminosäurerest angelagert ist, dieser Substituent dann an einer Alkylgruppe oder einer Gruppe, die eine (ω-Carboxygruppe aufweist, angelagert ist.

8. Derivat nach einem der vorangehenden Ansprüche, wobei der lipophile Substituent 4 bis 40 Kohlenstoffatome, stärker bevorzugt 8 bis 25 Kohlenstoffatome umfasst.

9. Derivat nach einem der vorangehenden Ansprüche, wobei ein lipophiler Substituent an einen Aminosäurerest in einer derartigen Weise angelagert ist, dass eine Carboxygruppe des lipophilen Substituenten mit einer Aminogruppe des Aminosäurerests eine Amidbindung bildet.

10. Derivat nach einem der Ansprüche 1 - 8, wobei ein lipophiler Substituent an einen Aminosäurerest in einer derartigen Weise angelagert ist, dass eine Aminogruppe des lipophilen Substituenten mit einer Carboxygruppe des Aminosäurerests eine Amidbindung bildet.

11. Derivat nach einem der vorangehenden Ansprüche, wobei der lipophile Substituent durch einen Abstandhalter an den Aminosäurerest angelagert ist.

12. Derivat nach Anspruch 11, wobei der Abstandhalter eine unverzweigte Alkan-α,ω-dicarbonsäuregruppe mit 1 bis 7 Methylengruppen, vorzugsweise zwei Methylengruppen ist, die zwischen einer Aminogruppe des Peptids und einer Aminogruppe des lipophilen Substituenten eine Brücke bilden.

13. Derivat nach Anspruch 11, wobei der Abstandhalter ein Aminosäurerest außer Cys oder ein Dipeptid wie Gly-Lys ist.

14. Derivat nach Anspruch 13, wobei eine Carboxygruppe des Ursprungspeptids mit einer Aminogruppe von Lys oder eines einen Lys-Rest enthaltenden Dipeptids eine Amidbindung bildet und die andere Aminogruppe des Lys-Abstandhalters oder eines einen Lys-Rest enthaltenden Dipeptid-Abstandhalters mit einer Carboxygruppe des lipophilen Substituenten eine Amidbindung bildet.

15. Derivat nach Anspruch 13, wobei eine Aminogruppe des Peptids mit einer Carboxygruppe des Aminosäurerest- oder Dipeptid-Abstandhalters eine Amidbindung bildet und eine Aminogruppe des Aminosäurerest- oder Dipeptid-Abstandhalters mit einer Carboxygruppe des lipophilen Substituenten eine Amidbindung bildet.

16. Derivat nach Anspruch 13, wobei eine Carboxygruppe des Peptids mit einer Aminogruppe des Aminosäurerestabstandhalters oder Dipeptid-Abstandhalters eine Amidbindung bildet und eine Carboxygruppe des Aminosäurerestabstandhalters oder Dipeptid-Abstandhalters mit einer Aminogruppe des lipophilen Substituenten eine Amidbindung bildet.

17. Derivat nach Anspruch 13, wobei eine Carboxygruppe des Ursprungspeptids mit einer Aminogruppe eines Abstandhalters, bei welchem es sich um Asp oder Glu handelt, oder eines einen Asp- oder Glu-Rest enthaltenden Dipeptid-Abstandhalters eine Amidbindung bildet und eine Carboxygruppe des Abstandhalters mit einer Aminogruppe des lipophilen Substituenten eine Amidbindung bildet.

18. Derivat nach einem der vorangehenden Ansprüche 1 - 17, wobei der lipophile Substituent ein teilweise oder vollständig hydriertes Cyclopentanophenathrengerüst umfasst.

19. Derivat nach einem der Ansprüche 1 - 17, wobei der lipophile Substituent eine geradkettige oder verzweigte Alkylgruppe ist.

20. Derivat nach einem der Ansprüche 1 - 17, wobei der lipophile Substituent eine Acylgruppe einer geradkettigen oder verzweigten Fettsäure ist.

21. Derivat nach Anspruch 20, wobei die Acylgruppe ausgewählt ist aus der Gruppe, umfassend CH₃(CH₂)ₙCO-, wobei n 4 bis 38 ist, vorzugsweise CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- und CH₃(CH₂)₂₂CO-.

22. Derivat nach einem der Ansprüche 1-17, wobei der lipophile Substituent eine Acylgruppe einer geradkettigen oder verzweigten Alkan-α,ω-dicarbonsäure ist.

23. Derivat nach Anspruch 22, wobei die Acylgruppe ausgewählt ist aus der Gruppe, umfassend HOOC(CH₂)ₘCO-, wobei m 4 bis 38, vorzugsweise 4 bis 24 ist, stärker bevorzugt ausgewählt aus der Gruppe, umfassend HOOC(CH₂)₁₄CO-, HOOC(CH₂)₁₆CO-, HOOC(CH₂)₁₈CO-, HOOC(CH₂)₂₀CO-und HOOC(CH₂)₂₂CO-.

24. Derivat nach einem der Ansprüche 1 - 17, wobei der lipophile Substituent mit dem Abstandhalter eine Gruppe der Formel CH₃(CH₂)ₚ((CH₂)_{q}COOH)CHNH-CO(CH₂)₂CO- ist, wobei p und q ganze Zahlen sind und p+q eine ganze Zahl von 8 bis 33, vorzugsweise von 12 bis 28 ist.

25. Derivat nach einem der Ansprüche 1 - 17, wobei der lipophile Substituent mit dem Abstandhalter eine Gruppe der Formel CH₃(CH₂)ᵣCO-NHCH(COOH)(CH₂)₂CO- ist, wobei r eine ganze Zahl von 10 bis 24 ist

26. Derivat nach einem der Ansprüche 1 - 17, wobei der lipophile Substituent mit dem Abstandhalter eine Gruppe der Formel CH₃(CH₂)ₛCO-NHCH((CH₂)₂COOH)CO- ist, wobei s eine ganze Zahl von 8 bis 24 ist.

27. Derivat nach einem der Ansprüche 1 - 17, wobei der lipophile Substituent mit dem Abstandhalter eine Gruppe der Formel -NHCH(COOH)(CH₂)₄NH-CO(CH₂)ᵤCH₃ ist, wobei u eine ganze Zahl von 8 bis 18 ist.

28. Derivat nach einem der Ansprüche 1 - 17, wobei der lipophile Substituent mit dem Abstandhalter eine Gruppe der Formel -NHCH(COOH)(CH₂)₄NH-COCH((CH₂)₂COOH)NH-CO(CH₂)_{w}CH₃ ist, wobei w eine ganze Zahl von 10 bis 16 ist.

29. Derivat nach einem der Ansprüche 1-17, wobei der lipophile Substituent mit dem Abstandhalter eine Gruppe der Formel -NHCH(COOH)(CH₂)₄NH-CO(CH₂)₂CH(COOH)NH-CO(CH₂)ₓCH₃ ist, wobei x eine ganze Zahl von 10 bis 16 ist.

30. Derivat nach einem der Ansprüche 1-17, wobei der lipophile Substituent mit dem Abstandhalter eine Gruppe der Formel -NHCH(COOH)(CH₂)₄NH-CO(CH₂)₂CH(COOH)NH-CO(CH₂)_{y}CH₃ ist, wobei y null oder eine ganze Zahl von 1 bis 22 ist.

31. Derivat nach einem der vorangehenden Ansprüche, wobei insgesamt bis zu sechs Aminosäurereste von GLP-1(7-37) mit einem beliebigen α-Aminosäurerest, der durch den genetischen Kode kodiert werden kann, ausgetauscht worden sind.

32. Derivat nach Anspruch 1, bei welchem es sich um Lys²⁶(N^{ε}-tetradecanoyl)-GLP-1(7-37) handelt.

33. Derivat nach Anspruch 1, bei welchem es sich um Lys³⁴(N^{ε}-tetradecanoyl)-GLP-1(7-37) handelt.

34. Derivat nach Anspruch 1, bei welchem es sich um Lys^{26,34}-bis(N^{ε}-tetradecanoyl)-GLP-1(7-37) handelt.

35. Derivat nach Anspruch 1, bei welchem es sich um Lys²⁶(N^{ε}-tetradecanoyl)Arg³⁴-GLP-1(7-37) handelt.

36. Derivat nach Anspruch 1, bei welchem es sich um Gly⁸Arg^{26,34}Lys³⁵(N^{ε}-tetradecanoyl)-GLP-1(7-37) handelt.

37. Derivat nach Anspruch 1, bei welchem es sich um Arg^{26,34}Lys³⁶(N^{ε}-tetradecanoyl)-GLP-1(7-37) handelt.

38. Derivat nach Anspruch 3, bei welchem es sich um Lys^{26,34}-bis(N^{ε}-(ω-carboxynonadecanoyl)-GLP-1(7-37) handelt.

39. Derivat nach Anspruch 1, bei welchem es sich um Arg³⁴Lys²⁶(N^{ε}-(ω-carboxynonadecanoyl)-GLP-1(7-37) handelt.

40. Derivat nach Anspruch 1, bei welchem es sich um Arg³⁴Lys²⁶(N^{ε}-(ω-carboxyheptadecanoyl)-GLP-1(7-37) handelt.

41. Derivat nach Anspruch 1, bei welchem es sich um Arg^{26,34}Lys³⁶(N^{ε}-(ω-carboxyheptadecanoyl)-GLP-1(7-37) handelt.

42. Derivat nach Anspruch 1, bei welchem es sich um Arg^{26,34}Lys³⁶(N^{ε}-(ω-carboxyundecadecanoyl)-GLP-1(7-37) handelt.

43. Derivat nach Anspruch 3, bei welchem es sich um Lys^{26,34}-bis(N^{ε}-(ω-carboxyundecadecanoyl)-GLP-1(7-37) handelt.

44. Derivat nach Anspruch 1, bei welchem es sich um Arg³⁴Lys²⁶(N^{ε}-(ω-carboxyundecadecanoyl)-GLP- 1(7-37) handelt.

45. Derivat nach Anspruch 1, bei welchem es sich um Arg³⁴Lys²⁶(N^{ε}-(ω-carboxyheptadecanoyl)-GLP-1(7-37) handelt.

46. Derivat nach Anspruch 1, bei welchem es sich um Arg^{26,4}Lys³⁶(N^{ε}-(ω-carboxyheptadecanoyl)-GLP-1(7-37) handelt.

47. Derivat nach Anspruch 3, bei welchem es sich um Lys^{26,34}-bis(N^{ε}-(ω-carboxyheptadecanoyl)-GLP-1(7-37) handelt.

48. Derivat nach Anspruch 1, bei welchem es sich um Arg³⁴Lys²⁶(N^{ε}-(ω-carboxypentadecanoyl)-GLP-1(7-37) handelt.

49. Derivat nach Anspruch 1, bei welchem es sich um Arg³⁴Lys²⁶(N^{ε}-(lithocholyl)-GLP-1(7-37) handelt.

50. Derivat nach Anspruch 3, bei welchem es sich um Lys^{26,34}-bis(N^{ε}-(carboxytridecanoyl)-GLP-1(7-37) handelt.

51. Derivat nach Anspruch 1, bei welchem es sich um Lys^{26,34}-bis(N^{ε}-(γ-glutamyl(N^{α}-tetradecanoyl)))-GLP-1(7-37) handelt.

52. Derivat nach Anspruch 3, bei welchem es sich um Lys^{26,34}-bis(N^{ε}-(γ-glutamyl(N^{α}-hexadecanoyl)))-GLP-1(7-37) handelt.

53. Derivat nach Anspruch 1, bei welchem es sich um Arg³⁴Lys²⁶(N^{ε}-(γ-glutamyl(N^{α}-hexadecanoyl)))-GLP-1(7-37) handelt.

54. Derivat nach Anspruch 1, bei welchem es sich um Arg³⁴Lys²⁶(N^{ε}-(γ-glutamyl(N^{α}-tetradecanoyl)))-GLP-1(7-3 7) handelt.

55. Arzneimittel, umfassend ein Derivat nach einem der Ansprüche 1-54 und einen pharmazeutisch verträglichen Träger.

56. Verwendung eines Derivats nach einem der Ansprüche 1 - 54 zur Herstellung eines Medikaments, das ein lang anhaltendes Wirkungsprofil in Bezug auf GLP-1 (7-37) aufweist.

57. Verwendung eines Derivats nach einem der Ansprüche 1 - 54 zur Herstellung eines Medikaments zur Behandlung von nicht-insulinabhängiger Diabetes mellitus.

58. Verwendung eines Derivats nach einem der Ansprüche 1 - 54 zur Herstellung eines Medikaments zur Behandlung von insulinabhängiger Diabetes mellitus.

59. Verwendung eines Derivats nach einem der Ansprüche 1-54 zur Herstellung eines Medikaments zur Behandlung von Fettsucht.

60. Verwendung eines Derivats nach einem der Ansprüche 1-54 zur Herstellung eines Medikaments zur Behandlung von einem oder mehreren Zuständen, ausgewählt aus nicht-insulinabhängiger Diabetes mellitus, insulinabhängiger Diabetes mellitus und Fettsucht.

## Revendications

1. Dérivé du GLP-1 (7-37) ou analogue du GLP-1 (7-37), lequel dérivé est un agoniste du récepteur du GLP-1 humain, **caractérisé en ce que** le dérivé n'a qu'un substituant lipophile qui est attaché à un résidu d'acide aminé qui n'est pas le résidu d'acide aminé N-terminal ou C-terminal.

2. Dérivé du GLP-1 (7-37) ou analogue du GLP-1 (7-37), lequel dérivé est un agoniste du récepteur du GLP-1 humain, **caractérisé en ce que** le dérivé n'a que deux substituants lipophiles, dont un est attaché au résidu d'acide aminé C-terminal alors que l'autre est attaché à un résidu d'acide aminé qui n'est pas le résidu d'acide aminé N-terminal ou C-terminal.

3. Dérivé du GLP-1 (7-37) ou analogue du GLP-1 (7-37), lequel dérivé est un agoniste du récepteur du GLP-1 humain, **caractérisé en ce que** le dérivé n'a que deux substituants lipophiles qui sont attachés à des résidus d'acides aminés qui ne sont pas le résidu d'acide aminé N-terminal ou C-terminal.

4. Dérivé d'un analogue du GLP-1 (7-37), lequel dérivé est un agoniste du récepteur du GLP-1 humain et où l'analogue est le GLP-1 (7-C) où C est de 38 à 45 ; **caractérisé en ce que** il n'a qu'un substituant lipophile qui est attaché au résidu d'acide aminé C-terminal.

5. Dérivé selon l'une quelconque des revendications 1 à 4, où l'analogue du GLP-1 (7-37) est choisi dans le groupe constitué par : Arg²⁶-GLP-1(7-37); Arg³⁴-GLP-1(7-37); Lys³⁶-GLP-1(7-37); Arg^{26,34}Lys³⁶-GLP-1(7-37); Arg^{26,34}Lys³⁸GLP-1(7-38); Arg^{26,34}Lys³⁹-GLP-1(7-39); Arg^{26,34}Lys⁴⁰-GLP-1(7-40); Arg²⁶Lys³⁶-GLP-1(7-37); Arg³⁴Lys³⁶-GLP-1(7-37); Arg²⁶Lys³⁹-GLP-1(7-39); Arg³⁴Lys⁴⁰-GLP-1(7-40); Arg^{26,34}Lys^{36,39}-GLP-1(7-39); Arg^{26,34}Lys^{36,40}-GLP-1(7-40); Gly⁸Arg26-GLP-1(7-37); Gly⁸Arg³⁴-GLP-1(7-37); Gly⁸Lys³⁶-GLP-1(7-37); Gly⁸Arg^{26,34}Lys³⁶-GLP-1(7-37); Gly⁸Arg^{26,34}Lys³⁹-GLP-1(7-39); Gly⁸Arg^{26,34}Lys⁴⁰-GLP-1(7-40); Gly⁸Arg²⁶Lys³⁶-GLP-1(7-37); Gly⁸Arg³⁴Lys³⁶-GLP-1(7-37); Gly⁸Arg²⁶Lys³⁹-GLP-1(7-39); Gly⁸Arg³⁴Lys⁴⁰-GLP-1(7-40); Gly⁸Arg^{26,34}Lys^{36,39}-GLP-1(7-39) und Gly⁸Arg^{26,34}Lys^{36,40}-GLP-1(7-40).

6. Dérivé selon l'une quelconque des revendications 1 à 4, où l'analogue du GLP-1 (7-37) est choisi dans le groupe constitué par : Arg^{26,34}Lys³⁸GLP-1(7-38); Arg^{26,34}Lys³⁹GLP-1(7-39); Arg^{26,34}Lys⁴⁰GLP-1 (7-40); Arg^{26,34}Lys⁴¹GLP-1(7-41); Arg^{26,34}Lys⁴²GLP-1(7-42); Arg^{26,34}Lys⁴³GLP-1(7-43); Arg^{26,34}Lys⁴⁴GLP-1(7-44); Arg^{26,34}Lys⁴⁵GLP-1(7-45); Arg^{26,34}Lys³⁸GLP-1(1-38); Arg^{26,34}Lys³⁹GLP-1(1-39); Arg^{26,34}Lys⁴⁰GLP-1(1-40); Arg^{26,34}Lys⁴¹GLP-1(1-41); Arg^{26,34}Lys⁴²GLP-1(1-42); Arg^{26,34}Lys⁴³GLP-1(1-43); Arg^{26,34}Lys⁴⁴GLP-1(1-44); Arg^{26,34}Lys⁴⁵GLP-1(1-45); Arg^{26,34}Lys³⁸GLP-1(2-38); Arg^{26,34}Lys³⁹GLP-1(2-39); Arg^{26,34}Lys⁴⁰GLP-1(2-40); Arg^{26,34}Lys⁴¹GLP-1(2-41); Arg^{26,34}Lys⁴²GLP-1(2-42); Arg^{26,34}Lys⁴³GLP-1(2-43); Arg^{26,34}Lys⁴⁴GLP-1(2-44); Arg^{26,34}Lys⁴⁵GLP-1 (2-45); Arg^{26,34}Lys³⁸GLP-1(3-38); Arg^{26,34}Lys³⁹GLP-1(3-39); Arg^{26,34}Lys⁴⁰GLP-1(3-40); Arg^{26,34}Lys⁴¹GLP-1(3-41); Arg^{26,34}Lys⁴²GLP-1(3-42); Arg^{26,34}Lys⁴³GLP-1(3-43); Arg^{26,34}Lys⁴⁴GLP-1(3-44); Arg^{26,34}Lys⁴⁵GLP-1(3-45); Arg^{26,34}Lys³⁸GLP-1(4-38); Arg^{26,34}Lys³⁹GLP-1(4-39), Arg^{26,34}Lys⁴⁰GLP-1(4-40); Arg^{26,34}Lys⁴¹GLP-1(4-41); Arg^{26,34}Lys⁴²GLP-1(4-42); Arg^{26,34}Lys⁴³GLP-1(4-43); Arg^{26,34}Lys⁴⁴GLP-1(4-44); Arg^{26,34}Lys⁴⁵GLP-1(4-45); Arg^{26,34}Lys³⁸GLP-1(5-38); Arg^{26,34}Lys³⁹GLP-1(5-39); Arg^{26,34}Lys⁴⁰GLP-1(5-40); Arg^{26,34}Lys⁴¹GLP-1(5-41); Arg^{26,34}Lys⁴²GLP-1(5-42); Arg^{26,34}Lys⁴³GLP-1(5-43); Arg^{26,34}Lys⁴⁴GLP-1(5-44); Arg^{26,34}Lys⁴⁵GLP-1(5-45); Arg^{26,34}Lys³⁸GLP-1(6-38); Arg^{26,34}Lys³⁹GLP-1(6-39); Arg^{26,34}Lys⁴⁰GLP-1(6-40); Arg^{26,34}Lys⁴¹GLP-1(6-41); Arg^{26,34}Lys⁴²GLP-1(6-42); Arg^{26,34}Lys⁴³GLP-1(6-43); Arg^{26,34}Lys⁴⁴GLP-1(6-44); Arg^{26,34}Lys⁴⁵GLP-1(6-45); Arg²⁶Lys³⁸GLP-1(1-38); Arg³⁴Lys³⁸GLP-1(1-38); Arg^{26,34}Lys^{36,38}GLP-1(1-38); Arg²⁶Lys³⁸GLP-1(7-38); Arg³⁴Lys³⁸GLP-1(7-38); Arg^{26,34}Lys^{36,38}GLP-1(7-38); Arg^{26,34}Lys³⁸GLP-1 (7-3 8); Arg²⁶Lys³⁹GLP-1(1-39); Arg³⁴Lys³⁹GLP-1(1-39); Arg^{26,34}Lys^{36,39}GLP-1(1-39); Arg²⁶Lys³⁹GLP-1(7-39); Arg³⁴Lys³⁹GLP-1(7-39) und Arg^{26,34}Lys^{36,39}GLP-1(7-39).

7. Dérivé d'un analogue du GLP-1 (7-27), lequel dérivé est un agoniste du récepteur du GLP-1 humain, où au moins un résidu d'acide aminé a un substituant lipophile attaché et l'analogue est choisi dans le groupe constitué par : Arg^{26,34}Lys³⁸GLP-1(7-38); Arg^{26,34}Lys³⁹GLP-1(7-39); Arg^{26,34}Lys⁴⁰GLP-1(7-40); Arg^{26,34}Lys⁴¹GLP-1(7-41); Arg^{26,34}Lys⁴²GLP-1(7-42); Arg^{26,34}Lys⁴³GLP-1(7-43); Arg^{26,34}Lys⁴⁴GLP-1(7-44); Arg^{26,34}Lys⁴⁵GLP-1(7-45); Arg^{26,34}Lys³⁸GLP-1(1-38); Arg^{26,34}Lys³⁹GLP-1(1-39); Arg^{26,34}Lys⁴⁰GLP-1(1-40); Arg^{26,34}Lys⁴¹GLP-1(1-41); Arg^{26,34}Lys⁴²GLP-1(1-42); Arg^{26,34}Lys⁴³GLP-1(1-43); Arg^{26,34}Lys⁴⁴GLP-1(1-44); Arg^{26,34}Lys⁴⁵GLP-1(1-45); Arg^{26,34}Lys³⁸GLP-1(2-38); Arg^{26,34}Lys³⁹GLP-1(2-39); Arg^{26,34}LyS⁴⁰GLP-1(2-40), Arg^{26,34}Lys⁴¹GLP-1(2-41); Arg^{26,34}Lys⁴²GLP-1(2-42); Arg^{26,34}Lys⁴³GLP-1(2-43); Arg^{26,34}Lys⁴⁴GLP-1(2-44); Arg^{26,34}Lys⁴⁵GLP-1 (2-45); Arg^{26,34}Lys³⁸GLP-1(3-38); Arg^{26,34}Lys³⁹GLP-1(3-39); Arg^{26,34}Lys⁴⁰GLP-1(3-40); Arg^{26,34}LyS⁴¹GLP-1(3-41); Arg^{26,34}Lys⁴²GLP-1(3-42); Arg^{26,34}Lys⁴³GLP-1(3-43); Arg^{26,34}Lys⁴⁴GLP-1(3-44); Arg^{26,34}Lys⁴⁵GLP-1(3-45); Arg^{26,34}Lys³⁸GLP-1(4-38); Arg^{26,34}Lys³⁹GLP-1(4-39); Arg^{26,34}Lys⁴⁰GLP-1(4-40); Arg^{26,34}Lys⁴¹GLP-1(4-41); Arg^{26,34}Lys⁴²GLP-1(4-42); Arg^{26,34}Lys⁴³GLP-1(4-43); Arg^{26,34}Lys⁴⁴GLP-1(4-44); Arg^{26,34}Lys⁴⁵GLP-1(4-45); Arg^{26,34}Lys³⁸GLP-1(5-38); Arg^{26,34}Lys³⁹GLP-1(5-39); Arg^{26,34}Lys⁴⁰GLP-1(5-40); Arg^{26,34}Lys⁴¹GLP-1(5-41); Arg^{26,34}Lys⁴²GLP-1(5-42); Arg^{26,34}Lys⁴³GLP-1(5-43); Arg^{26,34}Lys⁴⁴GLP-1(5-44); Arg^{26,34}Lys⁴⁵GLP-1(5-45); Arg^{26,34}Lys³⁸GLP-1(6-38); Arg^{26,34}Lys³⁹GLP-1(6-39); Arg^{26,34}Lys⁴⁰GLP-1(6-40); Arg^{26,34}Lys⁴¹GLP-1(6-41); Arg^{26,34}Lys⁴²GLP-1(6-42); Arg^{26,34}Lys⁴³GLP-1(6-43); Arg^{26,34}Lys⁴⁴GLP-1(6-44); Arg^{26,34}Lys⁴⁵GLP-1(6-45); Arg²⁶Lys³⁸GLP-1(1-38); Arg³⁴Lys³⁸GLP-1(1-38); Arg^{26,34}Lys^{36,38}GLP-1(1-38); Arg²⁶Lys³⁸GLP-1(7-38); Arg³⁴Lys³⁸GLP-1(7-38); Arg^{26,34}Lys^{36,38}GLP-1(7-38); Arg^{26,34}Lys³⁸GLP-1 (7-38); Arg²⁶Lys³⁹GLP-1(1-39); Arg³⁴Lys³⁹GLP-1(1-39); Arg^{26,34}Lys^{36,39}GLP-1(1-39); Arg²⁶Lys³⁹GLP-1(7-39); Arg³⁴Lys³⁹GLP-1(7-39) und Arg^{26,34}Lys^{36,39}GLP-1(7-39),
à la condition que si seulement un substituant lipophile est présent et que ce substituant est attaché au résidu d'acide aminé N-terminal ou C-terminal, alors ce substituant est un groupe alkyle ou un groupe qui possède un groupe ω-carboxylique.

8. Dérivé selon l'une quelconque des revendications précédentes, où le substituant lipophile comprend de 4 à 40 atomes de carbone, très préférentiellement de 8 à 25 atomes de carbone.

9. Dérivé selon l'une quelconque des revendications précédentes, où un substituant lipophile est attaché à un résidu d'acide aminé de telle manière qu'un groupe carboxyle du substituant lipophile forme une liaison amide avec un groupe amino du résidu d'acide aminé.

10. Dérivé selon l'une quelconque des revendications 1 à 8, où un substituant lipophile est attaché à un résidu d'acide aminé de telle manière qu'un groupe amino du substituant lipophile forme une liaison amide avec un groupe caboxyle du résidu d'acide aminé.

11. Dérivé selon l'une quelconque des revendications précédentes, où le substituant lipophile est attaché au résidu d'acide aminé au moyen d'un espaceur.

12. Dérivé selon la revendication 11, où l'espaceur est un groupe acide α,ω-dicarboxylique à alcane non ramifié ayant de 1 à 7 groupes méthylène, de préférence deux groupes méthylène, qui forment un pont entre un groupe amino du peptide et un groupe amino du substituant lipophile.

13. Dérivé selon la revendication 11, où l'espaceur est un résidu d'acide aminé à l'exception de Cys, ou un dipeptide tel que Gly-Cys.

14. Dérivé selon la revendication 13, où un groupe carboxyle du peptide parent forme une liaison amide ave un groupe amino de Lys ou un dipeptide contenant un résidu Lys, et l'autre groupe amino de l'espaceur Lys ou d'un dipeptide espaceur contenant un résidu Lys forme une liaison amide avec un groupe carboxyle du substituant lipophile.

15. Dérivé selon la revendication 13, où un groupe amino du peptide forme une liaison amide avec un groupe carboxyle du résidu d'acide aminé ou du dipeptide espaceur, et un groupe amino du résidu d'acide aminé ou du dipeptide espaceur forme une liaison amide avec un groupe carboxyle du substituant lipophile.

16. Dérivé selon la revendication 13, où un groupe carboxyle du peptide forme une liaison amide avec un groupe amino du résidu d'acide aminé espaceur ou du dipeptide espaceur, et un groupe carboxyle du résidu d'acide aminé espaceur ou du dipeptide espaceur forme une liaison amide avec un groupe amino du substituant lipophile.

17. Dérivé selon la revendication 13, où un groupe carboxyle du peptide forme une liaison amide avec un groupe amino d'un espaceur qui est Asp ou Glu, ou d'un dipeptide espaceur contenant un résidu Asp ou Glu, et un groupe carboxyle de l'espaceur forme une liaison amide avec un groupe amino du substituant lipophile.

18. Dérivé selon l'une quelconque des revendications précédentes, où le substituant lipophile comprend un squelette de cyclopentanophénathrène complètement hydrogéné.

19. Dérivé selon l'une quelconque des revendications 1 à 17, où le substituant lipophile est un groupe alkyle à chaîne linéaire ou ramifié.

20. Dérivé selon l'une quelconque des revendications 1 à 17, où le substituant lipophile est un groupe acyle d'un acide gras à chaîne linéaire ou ramifié.

21. Dérivé selon la revendication 20, dans lequel le groupe acyle est choisi dans le groupe comprenant CH₃(CH₂)ₙCO-, où n vaut 4 à 38, de préférence CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- et CH₃(CH₂)₂₂CO-.

22. Dérivé selon l'une quelconque des revendications 1 à 17, où le substituant lipophile est un groupe acyle d'un acide α,ω-dicarboxylique alcane à chaîne linéaire ou ramifié.

23. Dérivé selon la revendication 22, où le groupe acyle est choisi dans le groupe comprenant COOH(CH₂)ₘCO-,où m vaut de 4 à 38, de préférence de 4 à 24, très préférentiellement choisi dans le groupe comprenant COOH(CH₂)₁₄CO-, COOH(CH₂)₁₆CO-, COOH(CH₂)₁₈CO-, COOH(CH₂)₂₀CO- et COOH(CH₂)₂₂CO-.

24. Dérivé selon l'une quelconque des revendications 1 à 17, où le substituant lipophile avec l'espaceur est un groupe de formule CH₃(CH₂)ₚ₍(CH₂)_{q}COOH)CHNH-CO(CH₂)₂CO, où p et q sont des nombres entiers et p + q est un nombre entier de 8 à 33, de préférence de 12 à 28.

25. Dérivé selon l'une quelconque des revendications 1 à 17, où le substituant lipophile avec l'espaceur est un groupe de formule CH₃(CH₂)ᵣCO-NHCH(COOH)(CH₂)₂CO- , où r est un nombre entier de 10 à 24.

26. Dérivé selon l'une quelconque des revendications 1 à 17, où le substituant lipophile avec l'espaceur est un groupe de formule CH₃(CH₂)_{S}CO-NHCH((CH₂)₂COOH)CO- , où s est un nombre entier de 8 à 24.

27. Dérivé selon l'une quelconque des revendications 1 à 17, où le substituant lipophile avec l'espaceur est un groupe de formule NHCH(COOH)(CH₂)₄NH-CO(CH₂)ᵤCH₃, où u est un nombre entier de 8 à 18.

28. Dérivé selon l'une quelconque des revendications 1 à 17, où le substituant lipophile avec l'espaceur est un groupe de formule NHCH(COOH)(CH₂)₄NH-COCH(CH₂)₂COOH)NH-CO(CH₂)_{w}CH₃, où w est un nombre entier de 10 à 16.

29. Dérivé selon l'une quelconque des revendications 1 à 17, où le substituant lipophile avec l'espaceur est un groupe de formule NHCH(COOH)(CH₂)₄NH-CO(CH₂)₂CH(COOH)NH-CO(CH₂)ₓCH₃, où x est un nombre entier de 10 à 16.

30. Dérivé selon l'une quelconque des revendications 1 à 17, où le substituant lipophile avec l'espaceur est un groupe de formule NHCH(COOH)(CH₂)₄NH-CO(CH₂)₂CH(COOH)NH-CO(CH₂)_{y}CH₃, où y est zéro ou un nombre entier de 1 à 22.

31. Dérivé selon l'une quelconque des revendications précédentes, où un total de jusqu'à six résidus d'acides aminés du GLP-1 (7-37) ont été échangés avec un quelconque résidu d'acide α-aminé qui peut être codé par le code génétique.

32. Dérivé selon la revendication 1, qui est le Lys²⁶(N⁶-tétradécanoyl)-GLP-1 (7-37).

33. Dérivé selon la revendication 1, qui est le Lys³⁴(N⁶-tétradécanoyl)-GLP-1 (7-37).

34. Dérivé selon la revendication 1, qui est le Lys^{26,34}-bis(N⁶-tétradécanoyl)-GLP-1 (7-37).

35. Dérivé selon la revendication 1, qui est le Lys²⁵(N⁶-tétradécanoyl)Arg³⁴-GLP-1 (7-37).

36. Dérivé selon la revendication 1, qui est le Gly⁸Arg^{26,34}Lys³⁶(N⁶-tétradécanoyl)-GLP-1 (7-37).

37. Dérivé selon la revendication 1, qui est l'Arg^{26,34}Lys³⁶(N⁶-tétradécanoyl)-GLP-1 (7-37).

38. Dérivé selon la revendication 3, qui est le Lys^{26,34}-bis(N⁶-(ω-carboxynonadécanoyl))-GLP-1 (7-37).

39. Dérivé selon la revendication 1, qui est l'Arg³⁴Lys²⁶(N⁶-(ω-carboxynonadécanoyl))-GLP-1 (7-37).

40. Dérivé selon la revendication 1, qui est l'Arg³⁴Lys²⁶(N⁶-(ω-carboxyheptadécanoyl))-GLP-1 (7-37).

41. Dérivé selon la revendication 1, qui est l'Arg^{26,34}Lys²⁶(N⁶-(ω-carboxyheptadécanoyl))-GLP-1 (7-37).

42. Dérivé selon la revendication 1, qui est l'Arg^{26,34}Lys²⁶(N⁶-(ω-carboxyundécanoyl))-GLP-1 (7-37).

43. Dérivé selon la revendication 3, qui est le Lys^{26,34}bis(N⁶-(ω-carboxyundécanoyl))-GLP-1 (7-37).

44. Dérivé selon la revendication 1, qui est l'Arg³⁴Lys²⁶(N⁶-(ω-carboxyundécanoyl))-GLP-1 (7-37).

45. Dérivé selon la revendication 1, qui est l'Arg³⁴Lys²⁶(N⁶-(ω-carboxyheptanoyl))-GLP-1 (7-37).

46. Dérivé selon la revendication 1, qui est l'Arg^{26,34}Lys³⁶(N⁶-(ω-carboxyheptanoyl))-GLP-1 (7-37).

47. Dérivé selon la revendication 3, qui est le Lys^{26,34}bis(N⁶-(ω-carboxyheptanoyl))-GLP-1 (7-37).

48. Dérivé selon la revendication 1, qui est l'Arg³⁴Lys²⁶(N⁶-(ω-carboxypentadécanoyl))-GLP-1 (7-37).

49. Dérivé selon la revendication 1, qui est l'Arg³⁴Lys²⁵(N⁶-(N⁶-lithocholyl)-GLP-1 (7-37).

50. Dérivé selon la revendication 3, qui est le Lys^{26,34}bis(N⁶-(ω-carboxytridécanoyl))-GLP-1 (7-37).

51. Dérivé selon la revendication 3, qui est le Lys^{26,34}bis(N⁶-(γ-glutamyl(N^{α}-tétradécanoyl))-GLP-1 (7-37).

52. Dérivé selon la revendication 3, qui est le Lys^{26,34}bis(N⁶-(γ-glutamyl(N^{α}-hexadécanoyl))-GLP-1 (7-37).

53. Dérivé selon la revendication 1, qui est l'Arg³⁴Lys²⁶(N⁶-(γ-glutamyl(N^{α}-hexadécanoyl))-GLP-1 (7-37).

54. Dérivé selon la revendication 1, qui est l'Arg³⁴Lys²⁶(N⁶-(γ-glutamyl(N^{α}-tétradécanoyl))-GLP-1 (7-37).

55. Composition pharmaceutique comprenant un dérivé de l'une quelconque des revendications 1 à 54 et un véhicule ou transporteur pharmaceutiquement acceptable.

56. Utilisation d'un dérivé selon l'une quelconque des revendications 1 à 54 pour la préparation d'un médicament qui a un profil d'action prolongé par rapport au GLP-1 (7-37).

57. Utilisation d'un dérivé selon l'une quelconque des revendications 1 à 54 pour la préparation d'un médicament pour le traitement du diabetes mellitus non insulino-dépendant.

58. Utilisation d'un dérivé selon l'une quelconque des revendications 1 à 54 pour la préparation d'un médicament pour le traitement du diabetes mellitus insulino-dépendant.

59. Utilisation d'un dérivé selon l'une quelconque des revendications 1 à 54 pour la préparation d'un médicament pour le traitement de l'obésité.

60. Utilisation d'un dérivé selon l'une quelconque des revendications 1 à 54 pour la préparation d'un médicament pour le traitement d'un ou plusieurs états choisis parmi le diabetes mellitus non insulino-dépendant, le diabetes mellitus insulino-dépendant et l'obésité.
